(19) Europäisches Patentamt
European Patent Office
Office européen des brevets

(11) EP 4 387 968 B1

(12) # EUROPEAN PATENT SPECIFICATION

(45) Date of publication and mention of the grant of the patent:
**12.11.2025 Bulletin 2025/46**

(21) Application number: **22769445.2**

(22) Date of filing: **19.08.2022**

(51) International Patent Classification (IPC):
***C07D 403/12*** *(2006.01)* ***A61K 31/501*** *(2006.01)*
***A61P 29/00*** *(2006.01)* ***A61P 37/00*** *(2006.01)*

(52) Cooperative Patent Classification (CPC):
**C07D 403/12**

(86) International application number:
**PCT/US2022/040884**

(87) International publication number:
**WO 2023/023322 (23.02.2023 Gazette 2023/08)**

(54) **CRYSTAL FORMS OF 6-(CYCLOPROPANECARBOXAMIDO)-4-((2-METHOXY-3-(1-METHYL-1H-1,2,4-TRIAZOL-3-YL) PHENYL)AMINO)-N-(METHYL-D3)PYRIDAZINE-3-CARBOXAMIDE**

KRISTALLINE FORMEN VON 6-(CYCLOPROPANCARBOXAMIDO)-4-((2-METHOXY-3-(1-METHYL-1H-1,2,4-TRIAZOL-3-YL) PHENYL)AMINO)-N-(METHYL-D3)PYRIDAZIN-3-CARBOXAMID

FORME CRISTALLINE DE 6-(CYCLOPROPANECARBOXAMIDO)-4-((2-MÉTHOXY-3-(1-MÉTHYL-1H-1,2,4-TRIAZOL-3-YL) PHÉNYL)AMINO)-N-(MÉTHYL-D3)PYRIDAZINE-3-CARBOXAMIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2021 US 202163235635 P**

(43) Date of publication of application:
**26.06.2024 Bulletin 2024/26**

(73) Proprietor: **Bristol-Myers Squibb Company**
**Princeton, NJ 08543 (US)**

(72) Inventors:
- **WEI, Chenkou**
  **Princeton Junction, New Jersey 08550 (US)**
- **SHATTOCK-GORDON, Tanise**
  **West Lafayette, Indiana 47906-1076 (US)**
- **OHLAND, Joshua**
  **West Lafayette, Indiana 47906-1076 (US)**
- **WILLIFORD, Tabitha**
  **West Lafayette, Indiana 47906-1076 (US)**

(74) Representative: **Mewburn Ellis LLP**
**Aurora Building**
**Counterslip**
**Bristol BS1 6BX (GB)**

(56) References cited:
**WO-A1-2014/074661 WO-A1-2018/183656**
**WO-A1-2019/232138 WO-A1-2020/251911**
**WO-A1-2022/165141 WO-A1-2022/212181**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

## FIELD OF THE INVENTION

The claimed invention generally relates to crystalline Form R of 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide. The claimed invention also generally relates to pharmaceutical compositions comprising the crystalline form, as well as to methods for obtaining the crystalline form.

## BACKGROUND OF THE INVENTION

The compound, 6-(cyclopropanecarboxamido)-4-((2-methoxy-3-(1-methyl-1H-1,2,4-triazol-3-yl)phenyl)amino)-N-(methyl-$d_3$)pyridazine-3-carboxamide, has the structure of Formula (I):

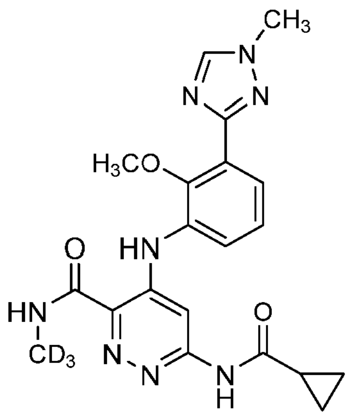

(I)

and is referred to herein as "Compound (I)". Compound (I) is disclosed in WO 2014/074661. WO 2014/074661 also discloses methods of treatment employing Compound (I). Compound (I) is also known as Deucravacitinib. Some crystalline forms are known from WO2018/183656, WO2019/232138 and WO2020/251911.

Compound (I) is a Tyk2 inhibitor currently in clinical trials for the treatment of autoimmune and auto-inflammatory diseases such as psoriasis, psoriatic arthritis, lupus, lupus nephritis, Sjögren's syndrome, inflammatory bowel disease, Crohn's disease, and ankylosing spondylitis.

In the synthesis of a chemical compound intended for pharmaceutical use, it is necessary to isolate and purify the compound at the completion of the synthesis process and prior to further processing to provide the compound in a pharmaceutical formulation. The isolation and the purification steps, which can be combined or separate consecutive steps, provide the compound as a purified solid, ideally with minimal loss of yield during isolation of the compound from other components of the reaction mixture and/or during purification to remove impurities from the isolated compound sample.

It is desirable to provide a solid form of such a compound that can be reproducibly produced from the isolation and/or purification steps.

It is also desirable to isolate the compound in a solid form that is physically and chemically stable upon storage, including at different conditions of temperature and humidity. Furthermore, it is desirable to provide a compound in a solid form that is amenable to additional processing, for example a crystalline form that can be converted to other solid forms, such as an amorphous form or other crystalline forms.

In addition, different crystalline forms can be used to modify the physiochemical properties of a compound. In some instances, the physiochemical properties of a compound can be modified through the formation of cocrystals. Cocrystallization also can be used to isolate or purify a compound during manufacturing.

As described herein, crystalline forms of Compound (I) are surprisingly amenable to additional processing and can be converted to other solid forms.

## SUMMARY OF THE INVENTION

The present disclosure provides crystalline forms Compound (I), namely Form L, Form M, Form N, Form O, Form P, Form Q, Form R, Form S, Form T, Form U, Form V, Form W, Form X, Form Y, Form Z, Form AA, Form AB, Form AC, Form AD, Form AE, Form AF, and Form AG. The claimed subject matter relates only to crystalline Form R of Compound (I). The

name used herein to characterize a specific form, e.g. "Form L", "Form M", etc. should not be considered limiting with respect to any other substance possessing similar or identical physical and chemical characteristics, but rather it should be understood that this designation is a mere identifier that should be interpreted according to the characterization information also presented herein.

## BRIEF DESCRIPTION OF THE DRAWINGS

FIG. 1 shows an observed powdered X-ray diffraction pattem (CuKα, measured at room temperature) of crystalline Form L of Compound (I).
FIG. 2 shows a thermogravimetric analysis (TGA) thermogram of crystalline Form L of Compound (I) (top graph) and a differential scanning calorimetry (DSC) thermogram of crystalline Form L of Compound (I) (bottom graph).
FIG. 3 shows an observed powdered X-ray diffraction pattern (CuKα, measured at room temperature) of crystalline Form M of Compound (I).
FIG. 4 shows a thermogravimetric analysis (TGA) thermogram of crystalline Form M of Compound (I) (top graph) and a differential scanning calorimetry (DSC) thermogram of crystalline Form M of Compound (I) (bottom graph).
FIG. 5 shows an observed powdered X-ray diffraction pattern (CuKα, measured at room temperature) of crystalline Form N of Compound (I).
FIG. 6 shows a thermogravimetric analysis (TGA) thermogram of crystalline Form N of Compound (I) (top graph) and a differential scanning calorimetry (DSC) thermogram of crystalline Form N of Compound (I) (bottom graph).
FIG. 7 shows an observed powdered X-ray diffraction pattern (CuKα, measured at room temperature) of crystalline Form O of Compound (I).
FIG. 8 shows an observed powdered X-ray diffraction pattern (CuKα, measured at room temperature) of crystalline Form P of Compound (I).
FIG. 9 shows an observed powdered X-ray diffraction pattern (CuKα, measured at room temperature) of crystalline Form Q of Compound (I).
FIG. 10 shows an observed powdered X-ray diffraction pattern (CuKα, measured at room temperature) of crystalline Form R of Compound (I).
FIG. 11 shows a thermogravimetric analysis (TGA) thermogram of crystalline Form R of Compound (I) (top graph) and a differential scanning calorimetry (DSC) thermogram of crystalline Form R of Compound (I) (bottom graph).
FIG. 12 shows an observed powdered X-ray diffraction pattern (CuKα, measured at room temperature) of crystalline Form S of Compound (I).
FIG. 13 shows a thermogravimetric analysis (TGA) thermogram of crystalline Form S of Compound (I) (top graph) and a differential scanning calorimetry (DSC) thermogram of crystalline Form S of Compound (I) (bottom graph).
FIG. 14 shows an observed powdered X-ray diffraction pattern (CuKα, measured at room temperature) of crystalline Form T of Compound (I).
FIG. 15 shows an observed powdered X-ray diffraction pattern (CuKα, measured at room temperature) of crystalline Form U of Compound (I).
FIG. 16 shows an observed powdered X-ray diffraction pattern (CuKα, measured at room temperature) of crystalline Form V of Compound (I).
FIG. 17 shows an observed powdered X-ray diffraction pattern (CuKα, measured at room temperature) of crystalline Form W of Compound (I).
FIG. 18 shows a thermogravimetric analysis (TGA) thermogram of crystalline Form W of Compound (I) (top graph) and a differential scanning calorimetry (DSC) thermogram of crystalline Form W of Compound (I) (bottom graph).
FIG. 19 shows an observed powdered X-ray diffraction pattern (CuKα, measured at room temperature) of crystalline Form X of Compound (I).
FIG. 20 shows an observed powdered X-ray diffraction pattern (CuKα, measured at room temperature) of crystalline Form Y of Compound (I).
FIG. 21 shows an observed powdered X-ray diffraction pattern (CuKα, measured at room temperature) of crystalline Form Z of Compound (I).
FIG. 22 shows an observed powdered X-ray diffraction pattern (CuKα, measured at room temperature) of crystalline Form AA of Compound (I).
FIG. 23 shows a differential scanning calorimetry (DSC) thermogram of crystalline Form AA of Compound (I).
FIG. 24 shows an observed powdered X-ray diffraction pattern (CuKα, measured at room temperature) of crystalline Form AB of Compound (I).
FIG. 25 shows a differential scanning calorimetry (DSC) thermogram of crystalline Form AB of Compound (I).
FIG. 26 shows a thermogravimetric analysis (TGA) thermogram of crystalline Form AB of Compound (I).

FIG. 27 shows an observed powdered X-ray diffraction pattern (CuKα, measured at room temperature) of crystalline Form AC of Compound (I).

FIG. 28 shows an observed powdered X-ray diffraction pattern (CuKα, measured at room temperature) of crystalline Form AD of Compound (I).

FIG. 29 shows an observed powdered X-ray diffraction pattern (CuKα, measured at room temperature) of crystalline Form AE of Compound (I).

FIG. 30 shows a differential scanning calorimetry (DSC) thermogram of crystalline Form AE of Compound (I).

FIG. 31 shows an observed powdered X-ray diffraction pattern (CuKα, measured at room temperature) of crystalline Form AF of Compound (I).

FIG. 32 shows a differential scanning calorimetry (DSC) thermogram of crystalline Form AF of Compound (I).

FIG. 33 shows a thermogravimetric analysis (TGA) thermogram of crystalline Form AF of Compound (I).

FIG. 34 shows an observed powdered X-ray diffraction pattern (CuKα, measured at room temperature) of crystalline Form AG of Compound (I).

FIG. 35 shows a differential scanning calorimetry (DSC) thermogram of crystalline Form AG of Compound (I).

FIG. 36 shows a thermogravimetric analysis (TGA) thermogram of crystalline Form AG of Compound (I).

## DETAILED DESCRIPTION OF THE INVENTION

The features and advantages of the invention may be more readily understood by those of ordinary skill in the art upon reading the following detailed description. It is to be appreciated that certain features of the invention that are, for clarity reasons, described above and below in the context of separate embodiments, may also be combined to form a single embodiment. Conversely, various features of the invention that are, for brevity reasons, described in the context of a single embodiment, may also be combined so as to form sub-combinations thereof.

The names used herein to characterize a specific form, e.g., "Form L" etc., are merely identifiers that are to be interpreted in accordance with the characterization information presented herein (or in references cited herein) and are not to be limited so as to exclude any other substance possessing similar or identical physical and chemical characteristics.

The definitions set forth herein take precedence over definitions set forth in any patent, patent application, and/or patent application publication referenced herein.

All numbers expressing quantities of ingredients, weight percentages, temperatures, and so forth that are preceded by the word "about" are to be understood as only approximations so that slight variations above and below the stated number may be used to achieve substantially the same results as the stated number. Accordingly, unless indicated to the contrary, numerical parameters preceded by the word "about" are approximations that may vary depending upon the desired properties sought to be obtained. At the very least, each numerical parameter should at least be construed in light of the number of reported significant digits and by applying ordinary rounding techniques.

All measurements are subject to experimental error.

As used herein, "polymorphs" refer to crystalline forms having the same chemical structure but different spatial arrangements of the molecules and/or ions forming the crystals.

A crystalline form may be a single-component crystalline form or a multi-component crystalline form. Multi-component crystalline forms comprise more than one type of molecule and include salts, solvates (such as hydrates), and cocrystals. Multi-component crystalline forms may have some variability in the exact molar ratio of their components depending on a variety of conditions. For example, a molar ratio of components within a solvate or cocrystal provides information as to the general relative quantities of each component of the solvate or cocrystal. In some cases, the molar ratio may vary by $\pm$ 0.2% from a stated value or a stated range. For example, a molar ratio of 1:0.5 should be understood to include the ratios 1:0.4 and 1:0.6, as well as all of the individual ratios in between; similarly, a molar ratio of 1:1 should be understood to include the ratios 1:0.8 and 1:1.2, as well as all of the individual ratios in between.

As used herein, "hydrate" refers to having a stoichiometric or non-stoichiometric amount of water molecules incorporated into the crystalline lattice structure. In some embodiments, a stoichiometric amount may be specified (e.g., monohydrate).

As used herein, "amorphous" refers to a solid form of a molecule and/or ion that is not crystalline. An amorphous solid does not display a definitive X-ray diffraction pattern with sharp maxima.

As used herein, "substantially pure," when used in reference to a crystalline form of a compound, means having the crystalline form at a purity greater than 90 weight %, including greater than 90, 91, 92, 93, 94, 95, 96, 97, 98, and 99 weight %, and also including equal to about 100 weight %, based on the weight of the sample or specimen. The remaining material comprises other form(s) of the compound, and/or reaction impurities and/or processing impurities arising from its preparation. For example, a crystalline form of Compound (I) may be deemed substantially pure in that it has a purity greater than 90 weight %, as measured by means that are at this time known and generally accepted in the art, where the remaining less than 10 weight % of material comprises amorphous and/or other form(s) of Compound (I) and/or reaction impurities and/or processing impurities.

As used herein, a powder X-ray diffraction (PXRD) pattern "comprising" a number of peaks selected from a specified group of peaks, is intended to include PXRD patterns having additional peaks that are not included in the specified group of peaks. For example, a PXRD pattern comprising four or more (or five or more, etc.) peaks or 2θ values selected from: a, b, c, d, e, f, g, and h, is intended to include a PXRD pattern having: (a) four or more (or five or more, etc.) 2θ values selected from: a, b, c, d, e, f, g, and h; and (b) zero, one, or more peaks that are not one of peaks a, b, c, d, e, f, g, and h.

The presence of reaction impurities and/or processing impurities may be determined by analytical techniques known in the art, such as, for example, chromatography, nuclear magnetic resonance spectroscopy, mass spectrometry, and/or infrared spectroscopy.

As used herein, the unit cell parameter "molecules per unit cell" refers to the number of molecules of Compound (I) in the unit cell.

Forms of Compound (I) have been described in WO 2018/183656 (Form A), WO 2019/232138 (Form B), WO 2020/251911 (Forms C and D), WO 2022/165141 (Form E), and WO 2022/212181 (Forms F, G, H, I, J, and K). The present invention generally relates to Form L, Form M, Form N, Form O, Form P, Form Q, Form R, Form S, Form T, Form U, Form V, Form W, Form X, Form Y, Form Z, Form AA, Form AB, Form AC, Form AD, Form AE, Form AF, and Form AG of Compound (I).

**Form L of Compound (I)**

In some embodiments, Compound (I) is provided as a crystalline material comprising Form L. The crystalline Form L of Compound (I) is a crystalline form comprising Compound (I) and gentisic acid. The molar ratio of Compound (I) to gentisic acid in Form L is approximately 1:1. Crystalline Form L may be non-solvated.

**Table 1: Form L of Compound (I)**

| PXRD 2θ values (CuKα) | | | | | | |
|---|---|---|---|---|---|---|
| 5.6±0.2° | 6.8±0.2° | 7.5±0.2° | 9.4±0.2° | 11.3±0.2° | 11.9±0.2° | 12.6±0.2° |
| 13.1±0.2° | 13.6±0.2° | 14.0±0.2° | 15.1±0.2° | 16.0±0.2° | 16.7±0.2° | 17.1±0.2° |
| 17.6±0.2° | 18.3±0.2° | 18.7±0.2° | 19.1±0.2° | 19.4±0.2° | 19.6±0.2° | 20.2±0.2° |
| 21.9±0.2° | 22.3±0.2° | 23.4±0.2° | 23.9±0.2° | 24.1±0.2° | 24.7±0.2° | 25.0±0.2° |
| 25.5±0.2° | 26.0±0.2° | 26.4±0.2° | 27.2±0.2° | 27.9±0.2° | 28.5±0.2° | 29.5±0.2° |

In some embodiments, crystalline Form L of Compound (I) is characterized by a powder X-ray diffraction (PXRD) pattern comprising two or more 2θ values in degrees (CuKα) selected from: 7.5±0.2, 13.1±0.2, 14.0±0.2, 15.1±0.2, 22.3 ±0.2, and 23.9±0.2, wherein the PXRD pattern of Form L is measured at room temperature.

In some embodiments, crystalline Form L of Compound (I) is characterized by a powder X-ray diffraction (PXRD) pattern comprising three or more 2θ values in degrees (CuKα) selected from: 7.5±0.2, 13.1±0.2, 14.0±0.2, 15.1±0.2, 22.3±0.2, and 23.9±0.2, wherein the PXRD pattern of Form L is measured at room temperature.

In some embodiments, crystalline Form L of Compound (I) is characterized by a powder X-ray diffraction (PXRD) pattern comprising four or more 2θ values in degrees (CuKα) selected from: 7.5±0.2, 13.1±0.2, 14.0±0.2, 15.1±0.2, 22.3 ±0.2, and 23.9±0.2, wherein the PXRD pattern of Form L is measured at room temperature.

In some embodiments, crystalline Form L of Compound (I) is characterized by a powder X-ray diffraction (PXRD) pattern comprising five or more 2θ values in degrees (CuKα) selected from: 7.5±0.2, 13.1±0.2, 14.0±0.2, 15.1±0.2, 22.3 ±0.2, and 23.9±0.2, wherein the PXRD pattern of Form L is measured at room temperature.

In certain embodiments, crystalline Form L of Compound (I) is characterized by a powder X-ray diffraction (PXRD) pattern comprising 2θ values in degrees (CuKα) at 7.5±0.2 and 15.1±0.2, wherein the PXRD pattern of Form L is measured at room temperature. In further embodiments, the PXRD pattern further comprises one or more 2θ values in degrees selected from: 13.1±0.2, 14.0±0.2, 22.3±0.2, and 23.9±0.2. For example, in some embodiments, crystalline Form L of Compound (I) is characterized by a powder X-ray diffraction (PXRD) pattern comprising 2θ values in degrees (CuKα) at 7.5±0.2, 13.1±0.2, and 15.1±0.2, wherein the PXRD pattern of Form L is measured at room temperature.

In certain embodiments, crystalline Form L of Compound (I) is characterized by a powder X-ray diffraction (PXRD) pattern comprising 2θ values in degrees (CuKα) at 7.5±0.2, 15.1±0.2, and 23.9±0.2, wherein the PXRD pattern of Form L is measured at room temperature.

In certain embodiments, crystalline Form L of Compound (I) is characterized by a powder X-ray diffraction (PXRD) pattern comprising 2θ values in degrees (CuKα) at 7.5±0.2, 13.1±0.2, 14.0±0.2, and 15.1±0.2, wherein the PXRD pattern of Form L is measured at room temperature.

In further embodiments, crystalline Form L of Compound (I) is characterized by a powder X-ray diffraction (PXRD) pattern comprising 2θ values in degrees (CuKα) at 7.5±0.2, 13.1±0.2, 14.0±0.2, 15.1±0.2, and 22.3±0.2, wherein the PXRD pattern of Form L is measured at room temperature.

In further embodiments, crystalline Form L of Compound (I) is characterized by a powder X-ray diffraction (PXRD) pattern comprising 2θ values in degrees (CuKα) at 7.5±0.2, 13.1±0.2, 14.0±0.2, 15.1±0.2, 22.3±0.2, and 23.9 ±0.2, wherein the PXRD pattern of Form L is measured at room temperature.

In certain embodiments, crystalline Form L of Compound (I) is characterized by an observed powder X-ray diffraction pattern (CuKα, measured at room temperature) substantially as shown in FIG. 1.

In some embodiments, crystalline Form L of Compound (I) is characterized by an endotherm with a peak having an onset temperature in the approximate range of from 210 °C to 220 °C. In further embodiments, the endotherm has a peak with an onset temperature that is about 215 °C. For example, in some embodiments, crystalline Form L of Compound (I) is characterized by a differential scanning calorimetry (DSC) thermogram comprising an endotherm with a peak having an onset temperature in the range of from about 210 °C to about 220 °C; in certain embodiments, the differential scanning calorimetry (DSC) thermogram comprises an endotherm with a peak having an onset temperature that is about 215 °C. It should be understood that, in some cases, the endothermic event may not be detected.

In certain embodiments, crystalline Form L of Compound (I) is characterized by (i) a powder X-ray diffraction (PXRD) pattern comprising 2θ values in degrees (CuKα) at 7.5±0.2 and 15.1±0.2, wherein the PXRD pattern of Form L is measured at room temperature; and (ii) an endotherm with a peak having an onset temperature in the approximate range of from 210 °C to 220 °C. In further embodiments, the endotherm peak has an onset temperature that is about 215 °C.

In certain embodiments, crystalline Form L of Compound (I) is characterized by (i) a powder X-ray diffraction (PXRD) pattern comprising 2θ values in degrees (CuKα) at 7.5±0.2, 13.1±0.2, and 15.1±0.2, wherein the PXRD pattern of Form L is measured at room temperature; and (ii) an endotherm with a peak having an onset temperature in the approximate range of from 210 °C to 220 °C. In further embodiments, the endotherm peak has an onset temperature that is about 215 °C.

In certain embodiments, crystalline Form L of Compound (I) is characterized by (i) a powder X-ray diffraction (PXRD) pattern comprising 2θ values in degrees (CuKα) at 7.5±0.2, 15.1±0.2, and 23.9±0.2, wherein the PXRD pattern of Form L is measured at room temperature; and (ii) an endotherm with a peak having an onset temperature in the approximate range of from 210 °C to 220 °C. In further embodiments, the endotherm peak has an onset temperature that is about 215 °C.

In some embodiments, crystalline Form L of Compound (I) is characterized by a differential scanning calorimetry (DSC) thermogram substantially in accordance with the thermogram shown in FIG. 2 (bottom graph).

In certain embodiments, crystalline Form L of Compound (I) is characterized by (i) a powder X-ray diffraction (PXRD) pattern comprising two or more 2θ values in degrees (CuKα) selected from: 7.5±0.2, 13.1±0.2, 14.0±0.2, 15.1 ±0.2, 22.3±0.2, and 23.9±0.2, wherein the PXRD pattern of Form L is measured at room temperature; and (ii) a differential scanning calorimetry (DSC) thermogram substantially in accordance with the thermogram shown in FIG. 2 (bottom graph).

In some embodiments, crystalline Form L of Compound (I) is characterized by a thermogravimetric analysis (TGA) thermogram having weight loss of less than 0.1% upon being heated (e.g., from room temperature) to a temperature of about 150 °C. In certain embodiments, crystalline Form L of Compound (I) is characterized by a thermogravimetric analysis (TGA) thermogram having weight loss of less than 0.1% upon being heated to a temperature of about 175 °C. In further embodiments, crystalline Form L of Compound (I) is characterized by a thermogravimetric analysis (TGA) thermogram showing less than about 1.0% weight loss, less than about 0.5% weight loss, or in certain embodiments less than about 0.1% weight loss, up to a temperature of about 200 °C.

In certain embodiments, crystalline Form L of Compound (I) exhibits a thermogravimetric analysis (TGA) thermogram substantially as shown in FIG. 2 (top graph).

In some embodiments, the Form L of Compound (I) is substantially pure. For example, Form L of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form L. For such embodiments, crystalline Compound (I) may comprise at least about 90 weight %, preferably at least about 95 weight %, and more preferably at least about 99 weight %, of crystalline Form L.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form L.

In further embodiments, a pharmaceutical composition is provided comprising Form L of Compound (I), and at

least one pharmaceutically-acceptable carrier and/or diluent.

In certain embodiments, a pharmaceutical composition comprises substantially pure Form L of Compound (I), and at least one pharmaceutically-acceptable carrier and/or diluent.

In certain embodiments, Form L of Compound (I) is combined with at least one pharmaceutically acceptable carrier and/or diluent to provide at least one pharmaceutical composition.

In some embodiments, a pharmaceutical composition comprises Form L of Compound (I) and other solid forms of Compound (I). Such other solid forms can be, for example, other crystalline forms and/or amorphous Compound (I).

**Form M of Compound (I)**

In some embodiments, Compound (I) is provided as a crystalline material comprising Form M. The crystalline Form M of Compound (I) is a hydrobromic acid (HBr) salt crystalline form. The molar ratio of Compound (I) to bromide (Br) in Form M is approximately 1:2. Crystalline Form M may be non-solvated.

**Table 2: Form M of Compound (I)**

| PXRD 2θ values (CuKα) | | | | | | |
|---|---|---|---|---|---|---|
| 7.9±0.2° | 8.9±0.2° | 9.3±0.2° | 9.8±0.2° | 10.7±0.2° | 12.3+0.2° | 12.6±0.2° |
| 13.0±0.2° | 13.7±0.2° | 15.1±0.2° | 15.6±0.2° | 15.9±0.2° | 16.5±0.2° | 17.8±0.2° |
| 18.1±0.2° | 18.5±0.2° | 19.1±0.2° | 19.7±0.2° | 21.0±0.2° | 21.5±0.2° | 21.9±0.2° |
| 22.5±0.2° | 23.2±0.2° | 23.7±0.2° | 24.1±0.2° | 24.8±0.2° | 25.1±0.2° | 25.4±0.2° |
| 25.8±0.2° | 26.0±0.2° | 27.0±0.2° | 27.7±0.2° | 29.1±0.2° | 29.6±0.2° | - |

In some embodiments, crystalline Form M of Compound (I) is characterized by a powder X-ray diffraction (PXRD) pattern comprising two or more 2θ values in degrees (CuKα) selected from: 9.8±0.2, 12.3±0.2, 16.5±0.2, 21.0±0.2, and 25.1±0.2, wherein the PXRD pattern of Form M is measured at room temperature.

In some embodiments, crystalline Form M of Compound (I) is characterized by a powder X-ray diffraction (PXRD) pattern comprising three or more 2θ values in degrees (CuKα) selected from: 9.8±0.2, 12.3±0.2, 16.5±0.2, 21.0±0.2, and 25.1±0.2, wherein the PXRD pattern of Form M is measured at room temperature.

In some embodiments, crystalline Form M of Compound (I) is characterized by a powder X-ray diffraction (PXRD) pattern comprising four or more 2θ values in degrees (CuKα) selected from: 9.8±0.2, 12.3±0.2, 16.5±0.2, 21.0±0.2, and 25.1±0.2, wherein the PXRD pattern of Form M is measured at room temperature.

In certain embodiments, crystalline Form M of Compound (I) is characterized by a powder X-ray diffraction (PXRD) pattern comprising 2θ values in degrees (CuKα) at 9.8±0.2 and 12.3±0.2, wherein the PXRD pattern of Form M is measured at room temperature. In further embodiments, the PXRD pattern further comprises one or more 2θ values in degrees selected from: 16.5±0.2, 21.0±0.2, and 25.1±0.2.

In certain embodiments, crystalline Form M of Compound (I) is characterized by a powder X-ray diffraction (PXRD) pattern comprising 2θ values in degrees (CuKα) at 12.3±0.2 and 16.5±0.2, wherein the PXRD pattern of Form M is measured at room temperature.

In further embodiments, crystalline Form M of Compound (I) is characterized by a powder X-ray diffraction (PXRD) pattern comprising 2θ values in degrees (CuKα) at 9.8±0.2, 12.3±0.2, and 16.5±0.2, wherein the PXRD pattern of Form M is measured at room temperature.

In further embodiments, crystalline Form M of Compound (I) is characterized by a powder X-ray diffraction (PXRD) pattern comprising 2θ values in degrees (CuKα) at 9.8±0.2, 12.3±0.2, 16.5±0.2, and 21.0±0.2, wherein the PXRD pattern of Form M is measured at room temperature.

In further embodiments, crystalline Form M of Compound (I) is characterized by a powder X-ray diffraction (PXRD) pattern comprising 2θ values in degrees (CuKα) at 9.8±0.2, 12.3±0.2, 16.5±0.2, 21.0±0.2, and 25.1±0.2, wherein the PXRD pattern of Form M is measured at room temperature.

In certain embodiments, crystalline Form M of Compound (I) is characterized by an observed powder X-ray diffraction pattern (CuKα, measured at room temperature) substantially as shown in FIG. 3.

In some embodiments, crystalline Form M of Compound (I) is characterized by an endotherm with a peak maximum in the approximate range of from 171 °C to 181 °C. For example, in some embodiments, crystalline Form M of Compound (I) is characterized by a differential scanning calorimetry (DSC) thermogram comprising an endotherm with a maximum in the range of from about 171 °C to about 181 °C. It should be understood that, in some cases, the endothermic event may not be detected.

In certain embodiments, crystalline Form M of Compound (I) is characterized by (i) a powder X-ray diffraction

(PXRD) pattern comprising 2θ values in degrees (CuKα) at 9.8±0.2 and 12.3±0.2, wherein the PXRD pattern of Form M is measured at room temperature; and (ii) an endotherm with a peak maximum in the approximate range of from 171 °C to 181 °C.

In certain embodiments, crystalline Form M of Compound (I) is characterized by (i) a powder X-ray diffraction (PXRD) pattern comprising 2θ values in degrees (CuKα) at 9.8±0.2, 12.3±0.2, and 16.5±0.2, wherein the PXRD pattern of Form M is measured at room temperature; and (ii) an endotherm with a peak maximum in the approximate range of from 171 °C to 181 °C.

In some embodiments, crystalline Form M of Compound (I) is characterized by a differential scanning calorimetry (DSC) thermogram substantially in accordance with the thermogram shown in FIG. 4 (bottom graph).

In certain embodiments, crystalline Form M of Compound (I) is characterized by (i) a powder X-ray diffraction (PXRD) pattern comprising two or more 2θ values in degrees (CuKα) selected from: 9.8±0.2, 12.3±0.2, 16.5±0.2, 21.0 ±0.2, and 25.1±0.2, wherein the PXRD pattern of Form M is measured at room temperature; and (ii) a differential scanning calorimetry (DSC) thermogram substantially in accordance with the thermogram shown in FIG. 4 (bottom graph).

In some embodiments, crystalline Form M of Compound (I) is characterized by a thermogravimetric analysis (TGA) thermogram having weight loss of less than 0.5% upon being heated (e.g., from room temperature) to a temperature of about 100 °C. In certain embodiments, crystalline Form M of Compound (I) is characterized by a thermogravimetric analysis (TGA) thermogram having weight loss of less than 0.5% upon being heated to a temperature of about 120 °C. In certain embodiments, crystalline Form M of Compound (I) is characterized by a thermogravimetric analysis (TGA) thermogram showing less than about 1.0% weight loss, less than about 0.5% weight loss, or in further embodiments less than about 0.1% weight loss, up to a temperature of about 120 °C.

In certain embodiments, crystalline Form M of Compound (I) exhibits a thermogravimetric analysis (TGA) thermogram substantially as shown in FIG. 4 (top graph).

In some embodiments, the Form M of Compound (I) is substantially pure. For example, Form M of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form M. For such embodiments, crystalline Compound (I) may comprise at least about 90 weight %, preferably at least about 95 weight %, and more preferably at least about 99 weight %, of crystalline Form M.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form M.

In further embodiments, a pharmaceutical composition is provided comprising Form M of Compound (I), and at least one pharmaceutically-acceptable carrier and/or diluent.

In certain embodiments, a pharmaceutical composition comprises substantially pure Form M of Compound (I), and at least one pharmaceutically-acceptable carrier and/or diluent.

In certain embodiments, Form M of Compound (I) is combined with at least one pharmaceutically acceptable carrier and/or diluent to provide at least one pharmaceutical composition.

In some embodiments, a pharmaceutical composition comprises Form M of Compound (I) and other solid forms of Compound (I). Such other solid forms can be, for example, other crystalline forms and/or amorphous Compound (I).

**Form N of Compound (I)**

In some embodiments, Compound (I) is provided as a crystalline material comprising Form N. The crystalline Form N of Compound (I) is a nitrate salt crystalline form. The molar ratio of Compound (I) to $NO_3^-$ in Form N is approximately 1:2. Crystalline Form N may be non-solvated.

**Table 3: Form N of Compound (I)**

| PXRD 2θ values (CuKα) | | | | | | |
|---|---|---|---|---|---|---|
| 6.4±0.2° | 7.2±0.2° | 7.9±0.2° | 8.1±0.2° | 9.0±0.2° | 9.4±0.2° | 10.2±0.2° |
| 10.9±0.2° | 11.2±0.2° | 11.5±0.2° | 12.3±0.2° | 12.8±0.2° | 13.2±0.2° | 14.0±0.2° |
| 14.5±0.2° | 15.2±0.2° | 15.8±0.2° | 16.1±0.2° | 16.6±0.2° | 17.3±0.2° | 17.6±0.2° |
| 18.0±0.2° | 18.4±0.2° | 18.9±0.2° | 19.2±0.2° | 19.9±0.2° | 20.5±0.2° | 20.8±0.2° |

(continued)

| PXRD 2θ values (CuKα) | | | | | | |
|---|---|---|---|---|---|---|
| 21.1±0.2° | 21.5±0.2° | 21.9±0.2° | 22.3±0.2° | 22.6±0.° | 23.0+0.2° | 23.5±0.2° |
| 23.8±0.2° | 24.7±0.2° | 25.0±0.2° | 25.8±0.2° | 26.4±0.2° | 26.8±0.2° | 28.8±0.2° |

In some embodiments, crystalline Form N of Compound (I) is characterized by a powder X-ray diffraction (PXRD) pattern comprising two or more 2θ values in degrees (CuKα) selected from: 8.1±0.2, 9.0±0.2, 15.8±0.2, 22.6±0.2, and 25.0±0.2, wherein the PXRD pattern of Form N is measured at room temperature.

In some embodiments, crystalline Form N of Compound (I) is characterized by a powder X-ray diffraction (PXRD) pattern comprising three or more 2θ values in degrees (CuKα) selected from: 8.1±0.2, 9.0±0.2, 15.8±0.2, 22.6±0.2, and 25.0±0.2, wherein the PXRD pattem of Form N is measured at room temperature.

In some embodiments, crystalline Form N of Compound (I) is characterized by a powder X-ray diffraction (PXRD) pattern comprising four or more 2θ values in degrees (CuKα) selected from: 8.1±0.2, 9.0±0.2, 15.8±0.2, 22.6±0.2, and 25.0±0.2, wherein the PXRD pattem of Form N is measured at room temperature.

In certain embodiments, crystalline Form N of Compound (I) is characterized by a powder X-ray diffraction (PXRD) pattern comprising 2θ values in degrees (CuKα) at 8.1±0.2 and 9.0±0.2, wherein the PXRD pattern of Form N is measured at room temperature. In further embodiments, the PXRD pattern further comprises one or more 2θ values in degrees selected from: 15.8±0.2, 22.6±0.2, and 25.0±0.2.

In certain embodiments, crystalline Form N of Compound (I) is characterized by a powder X-ray diffraction (PXRD) pattern comprising 2θ values in degrees (CuKα) at 9.0±0.2 and 22.6±0.2, wherein the PXRD pattern of Form N is measured at room temperature. In further embodiments, the PXRD pattern further comprises one or more 2θ values in degrees selected from: 8.1±0.2, 15.8±0.2, and 25.0±0.2.

In further embodiments, crystalline Form N of Compound (I) is characterized by a powder X-ray diffraction (PXRD) pattern comprising 2θ values in degrees (CuKα) at 8.1±0.2, 9.0±0.2, and 22.6±0.2, wherein the PXRD pattern of Form N is measured at room temperature.

In certain embodiments, crystalline Form N of Compound (I) is characterized by a powder X-ray diffraction (PXRD) pattern comprising 2θ values in degrees (CuKα) at 8.1±0.2, 9.0±0.2, and 15.8±0.2, wherein the PXRD pattern of Form N is measured at room temperature.

In further embodiments, crystalline Form N of Compound (I) is characterized by a powder X-ray diffraction (PXRD) pattern comprising 2θ values in degrees (CuKα) at 8.1±0.2, 9.0±0.2, 15.8±0.2, and 22.6±0.2, wherein the PXRD pattern of Form N is measured at room temperature.

In further embodiments, crystalline Form N of Compound (I) is characterized by a powder X-ray diffraction (PXRD) pattern comprising 2θ values in degrees (CuKα) at 8.1±0.2, 9.0±0.2, 15.8±0.2, 22.6±0.2, and 25.0±0.2, wherein the PXRD pattern of Form N is measured at room temperature.

In certain embodiments, crystalline Form N of Compound (I) is characterized by an observed powder X-ray diffraction pattern (CuKα, measured at room temperature) substantially as shown in FIG. 5.

In some embodiments, crystalline Form N of Compound (I) is characterized by an endotherm with a peak maximum in the approximate range of from 166 °C to 176 °C. For example, in some embodiments, crystalline Form N of Compound (I) is characterized by a differential scanning calorimetry (DSC) thermogram comprising an endotherm with a maximum in the range of from about 166 °C to about 176 °C. It should be understood that, in some cases, the endothermic event may not be detected.

In certain embodiments, crystalline Form N of Compound (I) is characterized by (i) a powder X-ray diffraction (PXRD) pattern comprising 2θ values in degrees (CuKα) at 8.1±0.2 and 9.0±0.2, wherein the PXRD pattern of Form N is measured at room temperature; and (ii) an endotherm with a peak maximum in the approximate range of from 166 °C to 176 °C.

In certain embodiments, crystalline Form N of Compound (I) is characterized by (i) a powder X-ray diffraction (PXRD) pattern comprising 2θ values in degrees (CuKα) at 8.1±0.2, 9.0±0.2, and 15.8±0.2, wherein the PXRD pattern of Form N is measured at room temperature; and (ii) an endotherm with a peak maximum in the approximate range of from 166 °C to 176 °C.

In some embodiments, crystalline Form N of Compound (I) is characterized by a differential scanning calorimetry (DSC) thermogram substantially in accordance with the thermogram shown in FIG. 6 (bottom graph).

In certain embodiments, crystalline Form N of Compound (I) is characterized by (i) a powder X-ray diffraction (PXRD) pattern comprising two or more 2θ values in degrees (CuKα) selected from: 8.1±0.2, 9.0±0.2, 15.8±0.2, 22.6 ±0.2, and 25.0±0.2, wherein the PXRD pattern of Form N is measured at room temperature; and (ii) a differential scanning calorimetry (DSC) thermogram substantially in accordance with the thermogram shown in FIG. 6 (bottom graph).

In some embodiments, crystalline Form N of Compound (I) is characterized by a thermogravimetric analysis

(TGA) thermogram having weight loss of less than 0.5% upon being heated (e.g., from room temperature) to a temperature of about 100 °C. In certain embodiments, crystalline Form N of Compound (I) is characterized by a thermogravimetric analysis (TGA) thermogram having weight loss of less than 0.5% upon being heated to a temperature of about 120 °C. In certain embodiments, crystalline Form N of Compound (I) is characterized by a thermogravimetric analysis (TGA) thermogram showing less than about 1.0% weight loss, less than about 0.5% weight loss, or in further embodiments less than about 0.1% weight loss, up to a temperature of about 120 °C.

In certain embodiments, crystalline Form N of Compound (I) exhibits a thermogravimetric analysis (TGA) thermogram substantially as shown in FIG. 6 (top graph).

In some embodiments, the Form N of Compound (I) is substantially pure. For example, Form N of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form N. For such embodiments, crystalline Compound (I) may comprise at least about 90 weight %, preferably at least about 95 weight %, and more preferably at least about 99 weight %, of crystalline Form N.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form N.

In further embodiments, a pharmaceutical composition is provided comprising Form N of Compound (I), and at least one pharmaceutically-acceptable carrier and/or diluent.

In certain embodiments, a pharmaceutical composition comprises substantially pure Form N of Compound (I), and at least one pharmaceutically-acceptable carrier and/or diluent.

In certain embodiments, Form N of Compound (I) is combined with at least one pharmaceutically acceptable carrier and/or diluent to provide at least one pharmaceutical composition.

In some embodiments, a pharmaceutical composition comprises Form N of Compound (I) and other solid forms of Compound (I). Such other solid forms can be, for example, other crystalline forms and/or amorphous Compound (I).

## Form O of Compound (I)

In some embodiments, Compound (I) is provided as a crystalline material comprising Form O. The crystalline Form O of Compound (I) is a p-toluenesulfonic acid (TSA) salt crystalline form. The molar ratio of Compound (I) to p-toluenesulfonic acid in Form O is approximately 1:1.

**Table 4: Form O of Compound (I)**

| PXRD 2θ values (CuKα) | | | | | | |
|---|---|---|---|---|---|---|
| 7.3±0.2° | 9.3±0.2° | 10.1±0.2° | 10.5±0.2° | 11.1±0.2° | 11.4±0.2° | 12.5±0.2° |
| 12.8±0.2° | 14.5±0.2° | 15.1±0.2° | 16.2±0.2° | 17.2±0.2° | 17.5±0.2° | 18.0±0.2° |
| 18.6±0.2° | 19.2±0.2° | 20.0±0.2° | 21.2±0.2° | 21.6±0.2° | 22.1±0.2° | 22.4±0.2° |
| 22.9±0.2° | 23.2±0.2° | 23.6±0.2° | 25.2±0.2° | 25.8±0.2° | 26.3±0.2° | 26.7±0.2° |
| 27.1±0.2° | 27.6±0.2° | 28.0±0.2° | 28.6±0.2° | - | - | - |

In some embodiments, crystalline Form O of Compound (I) is characterized by a powder X-ray diffraction (PXRD) pattern comprising two or more 2θ values in degrees (CuKα) selected from: 7.3±0.2, 10.1±0.2, 15.1±0.2, 17.2±0.2, and 25.2±0.2, wherein the PXRD pattem of Form O is measured at room temperature.

In some embodiments, crystalline Form O of Compound (I) is characterized by a powder X-ray diffraction (PXRD) pattern comprising three or more 2θ values in degrees (CuKα) selected from: 7.3±0.2, 10.1±0.2, 15.1±0.2, 17.2±0.2, and 25.2±0.2, wherein the PXRD pattern of Form O is measured at room temperature.

In some embodiments, crystalline Form O of Compound (I) is characterized by a powder X-ray diffraction (PXRD) pattern comprising four or more 2θ values in degrees (CuKα) selected from: 7.3±0.2, 10.1±0.2, 15.1±0.2, 17.2±0.2, and 25.2±0.2, wherein the PXRD pattern of Form O is measured at room temperature.

In certain embodiments, crystalline Form O of Compound (I) is characterized by a powder X-ray diffraction (PXRD) pattern comprising 2θ values in degrees (CuKα) at 7.3±0.2 and 10.1±0.2, wherein the PXRD pattern of Form O is measured at room temperature. In further embodiments, the PXRD pattern further comprises one or more 2θ values in

degrees selected from: 15.1±0.2, 17.2±0.2, and 25.2±0.2.

In certain embodiments, crystalline Form O of Compound (I) is characterized by a powder X-ray diffraction (PXRD) pattern comprising 2θ values in degrees (CuKα) at 7.3±0.2 and 15.1±0.2, wherein the PXRD pattern of Form O is measured at room temperature. In further embodiments, the PXRD pattern further comprises one or more 2θ values in degrees selected from: 10.1±0.2, 17.2±0.2, and 25.2±0.2.

In certain embodiments, crystalline Form O of Compound (I) is characterized by a powder X-ray diffraction (PXRD) pattern comprising 2θ values in degrees (CuKα) at 7.3±0.2, 10.1±0.2, and 15.1±0.2, wherein the PXRD pattern of Form O is measured at room temperature.

In further embodiments, crystalline Form O of Compound (I) is characterized by a powder X-ray diffraction (PXRD) pattern comprising 2θ values in degrees (CuKα) at 7.3±0.2, 10.1±0.2, 15.1±0.2, and 17.2±0.2, wherein the PXRD pattern of Form O is measured at room temperature.

In further embodiments, crystalline Form O of Compound (I) is characterized by a powder X-ray diffraction (PXRD) pattern comprising 2θ values in degrees (CuKα) at 7.3±0.2, 10.1±0.2, 15.1±0.2, 17.2±0.2, and 25.2±0.2, wherein the PXRD pattern of Form O is measured at room temperature.

In certain embodiments, crystalline Form O of Compound (I) is characterized by an observed powder X-ray diffraction pattern (CuKα, measured at room temperature) substantially as shown in FIG. 7.

In some embodiments, the Form O of Compound (I) is substantially pure. For example, Form O of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form O. For such embodiments, crystalline Compound (I) may comprise at least about 90 weight %, preferably at least about 95 weight %, and more preferably at least about 99 weight %, of crystalline Form O.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form O.

In further embodiments, a pharmaceutical composition is provided comprising Form O of Compound (I), and at least one pharmaceutically-acceptable carrier and/or diluent.

In certain embodiments, a pharmaceutical composition comprises substantially pure Form O of Compound (I), and at least one pharmaceutically-acceptable carrier and/or diluent.

In certain embodiments, Form O of Compound (I) is combined with at least one pharmaceutically acceptable carrier and/or diluent to provide at least one pharmaceutical composition.

In some embodiments, a pharmaceutical composition comprises Form O of Compound (I) and other solid forms of Compound (I). Such other solid forms can be, for example, other crystalline forms and/or amorphous Compound (I).

**Form P of Compound (I)**

In some embodiments, Compound (I) is provided as a crystalline material comprising Form P. The crystalline Form P of Compound (I) is a hydrobromic acid (HBr) salt crystalline form. The molar ratio of Compound (I) to bromide (Br) in Form P is approximately 1:2. Crystalline Form P may be hydrated.

**Table 5: Form P of Compound (I)**

| PXRD 2θ values (CuKα) | | | | | | |
|---|---|---|---|---|---|---|
| 7.4±0.2° | 8.6±0.2° | 11.4±0.2° | 14.9±0.2° | 15.6±0.2° | 16.2±0.2° | 17.3±0.2° |
| 18.8±0.2° | 19.8±0.2° | 20.8±0.2° | 21.7±0.2° | 22.5±0.2° | 22.9±0.2° | 23.5±0.2° |
| 24.0±0.2° | 24.8±0.2° | 25.1±0.2° | 25.7±0.2° | 26.6±0.2° | 27.1±0.2° | 27.7±0.2° |
| 28.6±0.2° | 29.0±0.2° | - | - | - | - | - |

In some embodiments, crystalline Form P of Compound (I) is characterized by a powder X-ray diffraction (PXRD) pattern comprising two or more 2θ values in degrees (CuKα) selected from: 7.4±0.2, 8.6±0.2, 14.9±0.2, 18.8±0.2, and 22.9±0.2, wherein the PXRD pattem of Form P is measured at room temperature.

In some embodiments, crystalline Form P of Compound (I) is characterized by a powder X-ray diffraction (PXRD) pattern comprising three or more 2θ values in degrees (CuKα) selected from: 7.4±0.2, 8.6±0.2, 14.9±0.2, 18.8±0.2, and

22.9±0.2, wherein the PXRD pattem of Form P is measured at room temperature.

In some embodiments, crystalline Form P of Compound (I) is characterized by a powder X-ray diffraction (PXRD) pattern comprising four or more 2θ values in degrees (CuKα) selected from: 7.4±0.2, 8.6±0.2, 14.9±0.2, 18.8±0.2, and 22.9±0.2, wherein the PXRD pattem of Form P is measured at room temperature.

In certain embodiments, crystalline Form P of Compound (I) is characterized by a powder X-ray diffraction (PXRD) pattern comprising 2θ values in degrees (CuKα) at 7.4±0.2 and 8.6±0.2, wherein the PXRD pattern of Form P is measured at room temperature. In further embodiments, the PXRD pattern further comprises one or more 2θ values in degrees selected from: 14.9±0.2, 18.8±0.2, and 22.9±0.2.

In certain embodiments, crystalline Form P of Compound (I) is characterized by a powder X-ray diffraction (PXRD) pattern comprising 2θ values in degrees (CuKα) at 7.4±0.2, 8.6±0.2, and 14.9±0.2, wherein the PXRD pattern of Form P is measured at room temperature.

In further embodiments, crystalline Form P of Compound (I) is characterized by a powder X-ray diffraction (PXRD) pattern comprising 2θ values in degrees (CuKα) at 7.4±0.2, 8.6±0.2, 14.9±0.2, and 18.8±0.2, wherein the PXRD pattern of Form P is measured at room temperature.

In further embodiments, crystalline Form P of Compound (I) is characterized by a powder X-ray diffraction (PXRD) pattern comprising 2θ values in degrees (CuKα) at 7.4±0.2, 8.6±0.2, 14.9±0.2, 18.8±0.2, and 22.9±0.2, wherein the PXRD pattern of Form P is measured at room temperature.

In certain embodiments, crystalline Form P of Compound (I) is characterized by an observed powder X-ray diffraction pattern (CuKα, measured at room temperature) substantially as shown in FIG. 8.

In some embodiments, the Form P of Compound (I) is substantially pure. For example, Form P of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form P. For such embodiments, crystalline Compound (I) may comprise at least about 90 weight %, preferably at least about 95 weight %, and more preferably at least about 99 weight %, of crystalline Form P.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form P.

In further embodiments, a pharmaceutical composition is provided comprising Form P of Compound (I), and at least one pharmaceutically-acceptable carrier and/or diluent.

In certain embodiments, a pharmaceutical composition comprises substantially pure Form P of Compound (I), and at least one pharmaceutically-acceptable carrier and/or diluent.

In certain embodiments, Form P of Compound (I) is combined with at least one pharmaceutically acceptable carrier and/or diluent to provide at least one pharmaceutical composition.

In some embodiments, a pharmaceutical composition comprises Form P of Compound (I) and other solid forms of Compound (I). Such other solid forms can be, for example, other crystalline forms and/or amorphous Compound (I).

**Form Q of Compound (I)**

In some embodiments, Compound (I) is provided as a crystalline material comprising Form Q. The crystalline Form Q of Compound (I) is a crystalline form comprising Compound (I) and malonic acid.

**Table 6: Form Q of Compound (I)**

| PXRD 2θ values (CuKα) | | | | | | |
|---|---|---|---|---|---|---|
| 5.4±0.2° | 5.9±0.2° | 9.6±0.2° | 10.4±0.2° | 10.9±0.2° | 11.6±0.2° | 11.9±0.2° |
| 14.4±0.2° | 14.8±0.2° | 15.4±0.2° | 15.9±0.2° | 16.4±0.2° | 17.5±0.2° | 18.0±0.2° |
| 18.4±0.2° | 18.7±0.2° | 19.5±0.2° | 19.8±0.2° | 20.2±0.2° | 21.2±0.2° | 21.9±0.2° |
| 22.7±0.2° | 23.8±0.2° | 25.6±0.2° | 26.1±0.2° | 26.9±0.2° | 27.7±0.2° | 28.5±0.2° |
| 29.0±0.2° | 29.7±0.2° | - | - | - | - | - |

In some embodiments, crystalline Form Q of Compound (I) is characterized by a powder X-ray diffraction (PXRD) pattern comprising two or more 2θ values in degrees (CuKα) selected from: 5.4±0.2, 9.6±0.2, 11.6±0.2, 20.2±0.2, 21.2±0.2, and 23.8±0.2, wherein the PXRD pattem of Form Q is measured at room temperature.

In some embodiments, crystalline Form Q of Compound (I) is characterized by a powder X-ray diffraction (PXRD) pattern comprising three or more 2θ values in degrees (CuKα) selected from: 5.4±0.2, 9.6±0.2, 11.6±0.2, 20.2±0.2, 21.2 ±0.2, and 23.8±0.2, wherein the PXRD pattem of Form Q is measured at room temperature.

In some embodiments, crystalline Form Q of Compound (I) is characterized by a powder X-ray diffraction (PXRD) pattern comprising four or more 2θ values in degrees (CuKα) selected from: 5.4±0.2, 9.6±0.2, 11.6±0.2, 20.2±0.2, 21.2 ±0.2, and 23.8±0.2, wherein the PXRD pattern of Form Q is measured at room temperature.

In some embodiments, crystalline Form Q of Compound (I) is characterized by a powder X-ray diffraction (PXRD) pattern comprising five or more 2θ values in degrees (CuKα) selected from: 5.4±0.2, 9.6±0.2, 11.6±0.2, 20.2±0.2, 21.2 ±0.2, and 23.8±0.2, wherein the PXRD pattern of Form Q is measured at room temperature.

In certain embodiments, crystalline Form Q of Compound (I) is characterized by a powder X-ray diffraction (PXRD) pattern comprising 2θ values in degrees (CuKα) at 9.6±0.2 and 11.6±0.2, wherein the PXRD pattern of Form Q is measured at room temperature. In further embodiments, the PXRD pattern further comprises one or more 2θ values in degrees selected from: 5.4±0.2, 20.2±0.2, 21.2±0.2, and 23.8±0.2.

In certain embodiments, crystalline Form Q of Compound (I) is characterized by a powder X-ray diffraction (PXRD) pattern comprising 2θ values in degrees (CuKα) at 5.4±0.2 and 9.6±0.2, wherein the PXRD pattern of Form Q is measured at room temperature. In further embodiments, the PXRD pattern further comprises one or more 2θ values in degrees selected from: 11.6±0.2, 20.2±0.2, 21.2±0.2, and 23.8±0.2

In certain embodiments, crystalline Form Q of Compound (I) is characterized by a powder X-ray diffraction (PXRD) pattern comprising 2θ values in degrees (CuKα) at 5.4±0.2, 9.6±0.2, and 11.6±0.2, wherein the PXRD pattern of Form Q is measured at room temperature.

In further embodiments, crystalline Form Q of Compound (I) is characterized by a powder X-ray diffraction (PXRD) pattern comprising 2θ values in degrees (CuKα) at 5.4±0.2, 9.6±0.2, 11.6±0.2, and 20.2±0.2, wherein the PXRD pattern of Form Q is measured at room temperature.

In further embodiments, crystalline Form Q of Compound (I) is characterized by a powder X-ray diffraction (PXRD) pattern comprising 2θ values in degrees (CuKα) at 5.4±0.2, 9.6±0.2, 11.6±0.2, 20.2±0.2, and 21.2±0.2, wherein the PXRD pattern of Form Q is measured at room temperature.

In further embodiments, crystalline Form Q of Compound (I) is characterized by a powder X-ray diffraction (PXRD) pattern comprising 2θ values in degrees (CuKα) at 5.4±0.2, 9.6±0.2, 11.6±0.2, 20.2±0.2, 21.2±0.2, and 23.8 ±0.2, wherein the PXRD pattern of Form Q is measured at room temperature.

In certain embodiments, crystalline Form Q of Compound (I) is characterized by an observed powder X-ray diffraction pattern (CuKα, measured at room temperature) substantially as shown in FIG. 9.

In some embodiments, the Form Q of Compound (I) is substantially pure. For example, Form Q of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form Q. For such embodiments, crystalline Compound (I) may comprise at least about 90 weight %, preferably at least about 95 weight %, and more preferably at least about 99 weight %, of crystalline Form Q.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form Q.

In further embodiments, a pharmaceutical composition is provided comprising Form Q of Compound (I), and at least one pharmaceutically-acceptable carrier and/or diluent.

In certain embodiments, a pharmaceutical composition comprises substantially pure Form Q of Compound (I), and at least one pharmaceutically-acceptable carrier and/or diluent.

In certain embodiments, Form Q of Compound (I) is combined with at least one pharmaceutically acceptable carrier and/or diluent to provide at least one pharmaceutical composition.

In some embodiments, a pharmaceutical composition comprises Form Q of Compound (I) and other solid forms of Compound (I). Such other solid forms can be, for example, other crystalline forms and/or amorphous Compound (I).

**Form R of Compound (I)**

In some embodiments, Compound (I) is provided as a crystalline material comprising Form R. The crystalline Form R of Compound (I) is a 1,2-ethanesulfonic acid salt crystalline form. The molar ratio of Compound (I) to 1,2-ethanesulfonic acid in Form R is approximately 2:1. Crystalline Form R may be non-solvated.

**Table 7: Form R of Compound (I)**

| PXRD 2θ values (CuKα) | | | | | | |
|---|---|---|---|---|---|---|
| 8.2±0.2° | 9.5±0.2° | 10.0±0.2° | 12.7±0.2° | 13.2±0.2° | 14.3±0.2° | 15.0±0.2° |
| 16.4±0.2° | 17.2±0.2° | 17.9±0.2° | 19.2±0.2° | 20.0±0.2° | 20.5±0.2° | 21.0±0.2° |
| 22.0±0.2° | 22.4±0.2° | 22.8±0.2° | 23.9±0.2° | 24.4±0.2° | 25.0±0.2° | 25.3±0.2° |
| 25.7±0.2° | 26.6±0.2° | 27.0±0.2° | 29.0±0.2° | 30.3±0.2° | - | - |

In some embodiments, crystalline Form R of Compound (I) is characterized by a powder X-ray diffraction (PXRD) pattern comprising two or more 2θ values in degrees (CuKα) selected from: 9.5±0.2, 10.0±0.2, 16.4±0.2, 17.2±0.2, 22.0 ±0.2, and 22.8±0.2, wherein the PXRD pattern of Form R is measured at room temperature.

In some embodiments, crystalline Form R of Compound (I) is characterized by a powder X-ray diffraction (PXRD) pattern comprising three or more 2θ values in degrees (CuKα) selected from: 9.5±0.2, 10.0±0.2, 16.4±0.2, 17.2±0.2, 22.0±0.2, and 22.8±0.2, wherein the PXRD pattern of Form R is measured at room temperature.

In some embodiments, crystalline Form R of Compound (I) is characterized by a powder X-ray diffraction (PXRD) pattern comprising four or more 2θ values in degrees (CuKα) selected from: 9.5±0.2, 10.0±0.2, 16.4±0.2, 17.2±0.2, 22.0 ±0.2, and 22.8±0.2, wherein the PXRD pattern of Form R is measured at room temperature.

In some embodiments, crystalline Form R of Compound (I) is characterized by a powder X-ray diffraction (PXRD) pattern comprising five or more 2θ values in degrees (CuKα) selected from: 9.5±0.2, 10.0±0.2, 16.4±0.2, 17.2±0.2, 22.0 ±0.2, and 22.8±0.2, wherein the PXRD pattern of Form R is measured at room temperature.

In certain embodiments, crystalline Form R of Compound (I) is characterized by a powder X-ray diffraction (PXRD) pattern comprising 2θ values in degrees (CuKα) at 9.5±0.2 and 10.0±0.2, wherein the PXRD pattern of Form R is measured at room temperature. In further embodiments, the PXRD pattern further comprises one or more 2θ values in degrees selected from: 16.4±0.2, 17.2±0.2, 22.0±0.2, and 22.8±0.2.

In certain embodiments, crystalline Form R of Compound (I) is characterized by a powder X-ray diffraction (PXRD) pattern comprising 2θ values in degrees (CuKα) at 9.5±0.2, 10.0±0.2, and 16.4±0.2, wherein the PXRD pattern of Form R is measured at room temperature.

In further embodiments, crystalline Form R of Compound (I) is characterized by a powder X-ray diffraction (PXRD) pattern comprising 2θ values in degrees (CuKα) at 9.5±0.2, 10.0±0.2, 16.4±0.2, and 17.2±0.2, wherein the PXRD pattern of Form R is measured at room temperature.

In further embodiments, crystalline Form R of Compound (I) is characterized by a powder X-ray diffraction (PXRD) pattern comprising 2θ values in degrees (CuKα) at 9.5±0.2, 10.0±0.2, 16.4±0.2, 17.2±0.2, and 22.0±0.2, wherein the PXRD pattern of Form R is measured at room temperature.

In further embodiments, crystalline Form R of Compound (I) is characterized by a powder X-ray diffraction (PXRD) pattern comprising 2θ values in degrees (CuKα) at 9.5±0.2, 10.0±0.2, 16.4±0.2, 17.2±0.2, 22.0±0.2, and 22.8 ±0.2, wherein the PXRD pattern of Form R is measured at room temperature.

In certain embodiments, crystalline Form R of Compound (I) is characterized by an observed powder X-ray diffraction pattern (CuKα, measured at room temperature) substantially as shown in FIG. 10.

In some embodiments, crystalline Form R of Compound (I) is characterized by an endotherm with a peak maximum in the approximate range of from 280 °C to 290 °C. For example, in some embodiments, crystalline Form R of Compound (I) is characterized by a differential scanning calorimetry (DSC) thermogram comprising an endotherm with a maximum in the range of from about 280 °C to about 290 °C. It should be understood that, in some cases, the endothermic event may not be detected.

In certain embodiments, crystalline Form R of Compound (I) is characterized by (i) a powder X-ray diffraction (PXRD) pattern comprising 2θ values in degrees (CuKα) at 9.5±0.2 and 10.0±0.2, wherein the PXRD pattern of Form R is measured at room temperature; and (ii) an endotherm with a peak maximum in the approximate range of from 280 °C to 290 °C.

In certain embodiments, crystalline Form R of Compound (I) is characterized by (i) a powder X-ray diffraction (PXRD) pattern comprising 2θ values in degrees (CuKα) at 9.5±0.2, 10.0±0.2, and 16.4±0.2, wherein the PXRD pattern of Form R is measured at room temperature; and (ii) an endotherm with a peak maximum in the approximate range of from 280 °C to 290 °C.

In some embodiments, crystalline Form R of Compound (I) is characterized by a differential scanning calorimetry (DSC) thermogram substantially in accordance with the thermogram shown in FIG. 11 (bottom graph).

In certain embodiments, crystalline Form R of Compound (I) is characterized by (i) a powder X-ray diffraction (PXRD) pattern comprising two or more 2θ values in degrees (CuKα) selected from: 9.5±0.2, 10.0±0.2, 16.4±0.2, 17.2 ±0.2, 22.0±0.2, and 22.8±0.2, wherein the PXRD pattern of Form R is measured at room temperature; and (ii) a

differential scanning calorimetry (DSC) thermogram substantially in accordance with the thermogram shown in FIG. 11 (bottom graph).

In some embodiments, crystalline Form R of Compound (I) is characterized by a thermogravimetric analysis (TGA) thermogram having weight loss of less than 0.1% upon being heated (e.g., from room temperature) to a temperature of about 150 °C. In certain embodiments, crystalline Form R of Compound (I) is characterized by a thermogravimetric analysis (TGA) thermogram having weight loss of less than 0.1% upon being heated to a temperature of about 175 °C. In certain embodiments, crystalline Form R of Compound (I) is characterized by a thermogravimetric analysis (TGA) thermogram showing less than about 1.0% weight loss, less than about 0.5% weight loss, or in further embodiments less than about 0.1% weight loss, up to a temperature of about 200 °C.

In certain embodiments, crystalline Form R of Compound (I) exhibits a thermogravimetric analysis (TGA) thermogram substantially as shown in FIG. 11 (top graph).

In some embodiments, the Form R of Compound (I) is substantially pure. For example, Form R of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form R. For such embodiments, crystalline Compound (I) may comprise at least about 90 weight %, preferably at least about 95 weight %, and more preferably at least about 99 weight %, of crystalline Form R.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form R.

In further embodiments, a pharmaceutical composition is provided comprising Form R of Compound (I), and at least one pharmaceutically-acceptable carrier and/or diluent.

In certain embodiments, a pharmaceutical composition comprises substantially pure Form R of Compound (I), and at least one pharmaceutically-acceptable carrier and/or diluent.

In certain embodiments, Form R of Compound (I) is combined with at least one pharmaceutically acceptable carrier and/or diluent to provide at least one pharmaceutical composition.

In some embodiments, a pharmaceutical composition comprises Form R of Compound (I) and other solid forms of Compound (I). Such other solid forms can be, for example, other crystalline forms and/or amorphous Compound (I).

**Form S of Compound (I)**

In some embodiments, Compound (I) is provided as a crystalline material comprising Form S. The crystalline Form S of Compound (I) is a 2-hydroxyethanesulfonic acid salt crystalline form. The molar ratio of Compound (I) to 2-hydroxyethanesulfonic acid in Form S is approximately 1:1. Crystalline Form S may be non-solvated.

**Table 8: Form S of Compound (I)**

| PXRD 2θ values (CuKα) | | | | | | |
|---|---|---|---|---|---|---|
| 7.9±0.2° | 8.4±0.2± | 9.6±0.2° | 10.1±0.2° | 11.4±0.2° | 11.8±0.2° | 13.2±0.2° |
| 14.0±0.2° | 14.3±0.2° | 14.9±0.2° | 15.6±0.2° | 16.0±0.2° | 16.5±0.2° | 16.9±0.2° |
| 17.4±0.2° | 18.3±0.2° | 18.6±0.2° | 19.3±0.2° | 20.3±0.2° | 21.6±0.2° | 22.3±0.2° |
| 23.1±0.2° | 23.7±0.2° | 24.7±0.2° | 25.2±0.2° | 26.8±0.2° | 27.3±0.2° | 28.3±0.2° |
| 28.8±0.2° | 29.6±0.2° | 30.3±0.2° | - | - | - | - |

In some embodiments, crystalline Form S of Compound (I) is characterized by a powder X-ray diffraction (PXRD) pattern comprising two or more 2θ values in degrees (CuKα) selected from: 8.4±0.2, 9.6±0.2, 19.3±0.2, 23.7±0.2, and 24.7±0.2, wherein the PXRD pattern of Form S is measured at room temperature.

In some embodiments, crystalline Form S of Compound (I) is characterized by a powder X-ray diffraction (PXRD) pattern comprising three or more 2θ values in degrees (CuKα) selected from: 8.4±0.2, 9.6±0.2, 19.3±0.2, 23.7±0.2, and 24.7±0.2, wherein the PXRD pattern of Form S is measured at room temperature.

In some embodiments, crystalline Form S of Compound (I) is characterized by a powder X-ray diffraction (PXRD) pattern comprising four or more 2θ values in degrees (CuKα) selected from: 8.4±0.2, 9.6±0.2, 19.3±0.2, 23.7±0.2, and 24.7±0.2, wherein the PXRD pattern of Form S is measured at room temperature.

In certain embodiments, crystalline Form S of Compound (I) is characterized by a powder X-ray diffraction (PXRD) pattern comprising 2θ values in degrees (CuKα) at 8.4±0.2 and 9.6±0.2, wherein the PXRD pattern of Form S is measured at room temperature. In further embodiments, the PXRD pattern further comprises one or more 2θ values in degrees selected from: 19.3±0.2, 23.7±0.2, and 24.7±0.2.

In certain embodiments, crystalline Form S of Compound (I) is characterized by a powder X-ray diffraction (PXRD) pattern comprising 2θ values in degrees (CuKα) at 8.4±0.2, 9.6±0.2, and 24.7±0.2, wherein the PXRD pattern of Form S is measured at room temperature.

In certain embodiments, crystalline Form S of Compound (I) is characterized by a powder X-ray diffraction (PXRD) pattern comprising 2θ values in degrees (CuKα) at 8.4±0.2, 9.6±0.2, and 19.3±0.2, wherein the PXRD pattern of Form S is measured at room temperature.

In further embodiments, crystalline Form S of Compound (I) is characterized by a powder X-ray diffraction (PXRD) pattern comprising 2θ values in degrees (CuKα) at 8.4±0.2, 9.6±0.2, 19.3±0.2, and 23.7±0.2, wherein the PXRD pattern of Form S is measured at room temperature.

In further embodiments, crystalline Form S of Compound (I) is characterized by a powder X-ray diffraction (PXRD) pattern comprising 2θ values in degrees (CuKα) at 8.4±0.2, 9.6±0.2, 19.3±0.2, 23.7±0.2, and 24.7±0.2, wherein the PXRD pattern of Form S is measured at room temperature.

In certain embodiments, crystalline Form S of Compound (I) is characterized by an observed powder X-ray diffraction pattern (CuKα, measured at room temperature) substantially as shown in FIG. 12.

In some embodiments, crystalline Form S of Compound (I) is characterized by an endotherm having a peak maximum at a temperature that is above about 180 °C. In certain embodiments, crystalline Form S of Compound (I) is characterized by an endotherm with a peak maximum in the approximate range of from 183 °C to 193 °C. For example, in some embodiments, crystalline Form S of Compound (I) is characterized by a differential scanning calorimetry (DSC) thermogram comprising an endotherm with a maximum in the range of from about 183 °C to about 193 °C. It should be understood that, in some cases, the endothermic event may not be detected.

In certain embodiments, crystalline Form S of Compound (I) is characterized by (i) a powder X-ray diffraction (PXRD) pattern comprising 2θ values in degrees (CuKα) at 8.4±0.2 and 9.6±0.2, wherein the PXRD pattern of Form S is measured at room temperature; and (ii) an endotherm with a peak maximum in the approximate range of from 183 °C to 193 °C.

In certain embodiments, crystalline Form S of Compound (I) is characterized by (i) a powder X-ray diffraction (PXRD) pattern comprising 2θ values in degrees (CuKα) at 8.4±0.2, 9.6±0.2, and 19.3±0.2, wherein the PXRD pattern of Form S is measured at room temperature; and (ii) an endotherm with a peak maximum in the approximate range of from 183 °C to 193 °C.

In certain embodiments, crystalline Form S of Compound (I) is characterized by (i) a powder X-ray diffraction (PXRD) pattern comprising 2θ values in degrees (CuKα) at 8.4±0.2, 9.6±0.2, and 24.7±0.2, wherein the PXRD pattern of Form S is measured at room temperature; and (ii) an endotherm with a peak maximum in the approximate range of from 183 °C to 193 °C.

In some embodiments, crystalline Form S of Compound (I) is characterized by a differential scanning calorimetry (DSC) thermogram substantially in accordance with the thermogram shown in FIG. 13 (bottom graph).

In certain embodiments, crystalline Form S of Compound (I) is characterized by (i) a powder X-ray diffraction (PXRD) pattern comprising two or more 2θ values in degrees (CuKα) selected from: 8.4±0.2, 9.6±0.2, 19.3±0.2, 23.7 ±0.2, and 24.7±0.2, wherein the PXRD pattern of Form S is measured at room temperature; and (ii) a differential scanning calorimetry (DSC) thermogram substantially in accordance with the thermogram shown in FIG. 13 (bottom graph).

In some embodiments, crystalline Form S of Compound (I) is characterized by a thermogravimetric analysis (TGA) thermogram having weight loss of less than 0.1% upon being heated (e.g., from room temperature) to a temperature of about 150 °C. In certain embodiments, crystalline Form S of Compound (I) is characterized by a thermogravimetric analysis (TGA) thermogram having weight loss of less than 0.1% upon being heated to a temperature of about 175 °C. In certain embodiments, crystalline Form S of Compound (I) is characterized by a thermogravimetric analysis (TGA) thermogram showing less than about 1.0% weight loss, less than about 0.5% weight loss, or in further embodiments less than about 0.1% weight loss, up to a temperature of about 200 °C.

In certain embodiments, crystalline Form S of Compound (I) exhibits a thermogravimetric analysis (TGA) thermogram substantially as shown in FIG. 13 (top graph).

In some embodiments, the Form S of Compound (I) is substantially pure. For example, Form S of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form S. For such embodiments, crystalline Compound (I) may comprise at least about 90 weight %, preferably at least about 95 weight %, and more preferably at least about 99 weight %, of crystalline Form S.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form S.

In further embodiments, a pharmaceutical composition is provided comprising Form S of Compound (I), and at least one pharmaceutically-acceptable carrier and/or diluent.

In certain embodiments, a pharmaceutical composition comprises substantially pure Form S of Compound (I), and at least one pharmaceutically-acceptable carrier and/or diluent.

In certain embodiments, Form S of Compound (I) is combined with at least one pharmaceutically acceptable carrier and/or diluent to provide at least one pharmaceutical composition.

In some embodiments, a pharmaceutical composition comprises Form S of Compound (I) and other solid forms of Compound (I). Such other solid forms can be, for example, other crystalline forms and/or amorphous Compound (I).

**Form T of Compound** (I)

In some embodiments, Compound (I) is provided as a crystalline material comprising Form T. The crystalline Form T of Compound (I) is a crystalline form comprising Compound (I) and maleic acid. The molar ratio of Compound (I) to maleic acid in Form T is approximately 1:1.

**Table 9: Form T of Compound (I)**

| PXRD 2θ values (CuKα) | | | | | | |
|---|---|---|---|---|---|---|
| 5.1±0.2° | 10.2±0.2° | 10.7±0.2° | 11.0±0.2° | 12.7±0.2° | 13.5±0.2° | 13.9±0.2° |
| 14.4±0.° | 15.4±0.2° | 15.6±0.2° | 16.1±0.2° | 17.2±0.2° | 18.5±0.2° | 19.4±0.2° |
| 20.4±0.2° | 22.0±0.2° | 22.7±0.2° | 23.5±0.2° | 23.9±0.2° | 24.2±0.2° | 24.9±0.2° |
| 25.5±0.2° | 26.5±0.2° | 28.3±0.2° | 29.1±0.2° | 29.7±0.2° | - | - |

In some embodiments, crystalline Form T of Compound (I) is characterized by a powder X-ray diffraction (PXRD) pattern comprising two or more 2θ values in degrees (CuKα) selected from: 10.7±0.2, 11.0±0.2, 12.7±0.2, 18.5±0.2, and 23.9±0.2, wherein the PXRD pattern of Form T is measured at room temperature.

In some embodiments, crystalline Form T of Compound (I) is characterized by a powder X-ray diffraction (PXRD) pattern comprising three or more 2θ values in degrees (CuKα) selected from: 10.7±0.2, 11.0±0.2, 12.7±0.2, 18.5±0.2, and 23.9±0.2, wherein the PXRD pattern of Form T is measured at room temperature.

In some embodiments, crystalline Form T of Compound (I) is characterized by a powder X-ray diffraction (PXRD) pattern comprising four or more 2θ values in degrees (CuKα) selected from: 10.7±0.2, 11.0±0.2, 12.7±0.2, 18.5±0.2, and 23.9±0.2, wherein the PXRD pattern of Form T is measured at room temperature.

In certain embodiments, crystalline Form T of Compound (I) is characterized by a powder X-ray diffraction (PXRD) pattern comprising 2θ values in degrees (CuKα) at 10.7±0.2 and 11.0±0.2, wherein the PXRD pattern of Form T is measured at room temperature. In further embodiments, the PXRD pattern further comprises one or more 2θ values in degrees selected from: 12.7±0.2, 18.5±0.2, and 23.9±0.2.

In certain embodiments, crystalline Form T of Compound (I) is characterized by a powder X-ray diffraction (PXRD) pattern comprising 2θ values in degrees (CuKα) at 10.7±0.2, 11.0±0.2, and 23.9±0.2, wherein the PXRD pattern of Form T is measured at room temperature.

In certain embodiments, crystalline Form T of Compound (I) is characterized by a powder X-ray diffraction (PXRD) pattern comprising 2θ values in degrees (CuKα) at 10.7±0.2, 11.0±0.2, and 12.7±0.2, wherein the PXRD pattern of Form T is measured at room temperature.

In further embodiments, crystalline Form T of Compound (I) is characterized by a powder X-ray diffraction (PXRD) pattern comprising 2θ values in degrees (CuKα) at 10.7±0.2, 11.0±0.2, 12.7±0.2, and 18.5±0.2, wherein the PXRD pattern of Form T is measured at room temperature.

In further embodiments, crystalline Form T of Compound (I) is characterized by a powder X-ray diffraction (PXRD) pattern comprising 2θ values in degrees (CuKα) at 10.7±0.2, 11.0±0.2, 12.7±0.2, 18.5±0.2, and 23.9±0.2, wherein the PXRD pattern of Form T is measured at room temperature.

In certain embodiments, crystalline Form T of Compound (I) is characterized by an observed powder X-ray diffraction pattern (CuKα, measured at room temperature) substantially as shown in FIG. 14.

In some embodiments, the Form T of Compound (I) is substantially pure. For example, Form T of Compound (I)

may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form T. For such embodiments, crystalline Compound (I) may comprise at least about 90 weight %, preferably at least about 95 weight %, and more preferably at least about 99 weight %, of crystalline Form T.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form T.

In further embodiments, a pharmaceutical composition is provided comprising Form T of Compound (I), and at least one pharmaceutically-acceptable carrier and/or diluent.

In certain embodiments, a pharmaceutical composition comprises substantially pure Form T of Compound (I), and at least one pharmaceutically-acceptable carrier and/or diluent.

In certain embodiments, Form T of Compound (I) is combined with at least one pharmaceutically acceptable carrier and/or diluent to provide at least one pharmaceutical composition.

In some embodiments, a pharmaceutical composition comprises Form T of Compound (I) and other solid forms of Compound (I). Such other solid forms can be, for example, other crystalline forms and/or amorphous Compound (I).

**Form U of Compound (I)**

In some embodiments, Compound (I) is provided as a crystalline material comprising Form U. The crystalline Form U of Compound (I) is a naphthalene-1,5-disulfonic acid salt crystalline form. The molar ratio of Compound (I) to naphthalene-1,5-disulfonic acid in Form U is approximately 1:2. Crystalline Form U may be hydrated.

**Table 10: Form U of Compound (I)**

| PXRD 2θ values (CuKα) | | | | | | |
|---|---|---|---|---|---|---|
| 7.2±0.2° | 8.3±0.2° | 9.1±0.2° | 10.2±0.2° | 10.6±0.2° | 10.9±0.2° | 11.8±0.2° |
| 12.7±0.2° | 13.4±0.2° | 14.3±0.2° | 14.7±0.2° | 15.0±0.2° | 15.6±0.2° | 16.4±0.2° |
| 16.8±0.2° | 17.5±0.2° | 17.9±0.2° | 18.5±0.2° | 19.6±0.2° | 20.2±0.2° | 20.9±0.2° |
| 21.5±0.2° | 21.8±0.2° | 22.4±0.2° | 22.8±0.2° | 23.1±0.2° | 23.4±0.2° | 23.8±0.2° |
| 24.3±0.2° | 25.0±0.2° | 26.4±0.2° | 26.8±0.2° | 27.5±0.2° | 28.4±0.2° | - |

In some embodiments, crystalline Form U of Compound (I) is characterized by a powder X-ray diffraction (PXRD) pattern comprising two or more 2θ values in degrees (CuKα) selected from: 7.2±0.2, 9.1±0.2, 10.2±0.2, 13.4±0.2, and 18.5±0.2, wherein the PXRD pattern of Form U is measured at room temperature.

In some embodiments, crystalline Form U of Compound (I) is characterized by a powder X-ray diffraction (PXRD) pattern comprising three or more 2θ values in degrees (CuKα) selected from: 7.2±0.2, 9.1±0.2, 10.2±0.2, 13.4±0.2, and 18.5±0.2, wherein the PXRD pattern of Form U is measured at room temperature.

In some embodiments, crystalline Form U of Compound (I) is characterized by a powder X-ray diffraction (PXRD) pattern comprising four or more 2θ values in degrees (CuKα) selected from: 7.2±0.2, 9.1±0.2, 10.2±0.2, 13.4±0.2, and 18.5±0.2, wherein the PXRD pattem of Form U is measured at room temperature.

In certain embodiments, crystalline Form U of Compound (I) is characterized by a powder X-ray diffraction (PXRD) pattern comprising 2θ values in degrees (CuKα) at 7.2±0.2 and 9.1±0.2, wherein the PXRD pattern of Form U is measured at room temperature. In further embodiments, the PXRD pattern further comprises one or more 2θ values in degrees selected from: 10.2±0.2, 13.4±0.2, and 18.5±0.2.

In certain embodiments, crystalline Form U of Compound (I) is characterized by a powder X-ray diffraction (PXRD) pattern comprising 2θ values in degrees (CuKα) at 7.2±0.2, 9.1±0.2, and 18.5±0.2, wherein the PXRD pattern of Form U is measured at room temperature.

In certain embodiments, crystalline Form U of Compound (I) is characterized by a powder X-ray diffraction (PXRD) pattern comprising 2θ values in degrees (CuKα) at 7.2±0.2, 9.1±0.2, and 10.2±0.2, wherein the PXRD pattern of Form U is measured at room temperature.

In further embodiments, crystalline Form U of Compound (I) is characterized by a powder X-ray diffraction (PXRD) pattern comprising 2θ values in degrees (CuKα) at 7.2±0.2, 9.1±0.2, 10.2±0.2, and 13.4±0.2, wherein the

PXRD pattern of Form U is measured at room temperature.

In further embodiments, crystalline Form U of Compound (I) is characterized by a powder X-ray diffraction (PXRD) pattern comprising 2θ values in degrees (CuKα) at 7.2±0.2, 9.1±0.2, 10.2±0.2, 13.4±0.2, and 18.5±0.2, wherein the PXRD pattern of Form U is measured at room temperature.

In certain embodiments, crystalline Form U of Compound (I) is characterized by an observed powder X-ray diffraction pattern (CuKα, measured at room temperature) substantially as shown in FIG. 15.

In some embodiments, the Form U of Compound (I) is substantially pure. For example, Form U of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form U. For such embodiments, crystalline Compound (I) may comprise at least about 90 weight %, preferably at least about 95 weight %, and more preferably at least about 99 weight %, of crystalline Form U.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form U.

In further embodiments, a pharmaceutical composition is provided comprising Form U of Compound (I), and at least one pharmaceutically-acceptable carrier and/or diluent.

In certain embodiments, a pharmaceutical composition comprises substantially pure Form U of Compound (I), and at least one pharmaceutically-acceptable carrier and/or diluent.

In certain embodiments, Form U of Compound (I) is combined with at least one pharmaceutically acceptable carrier and/or diluent to provide at least one pharmaceutical composition.

In some embodiments, a pharmaceutical composition comprises Form U of Compound (I) and other solid forms of Compound (I). Such other solid forms can be, for example, other crystalline forms and/or amorphous Compound (I).

**Form V of Compound (I)**

In some embodiments, Compound (I) is provided as a crystalline material comprising Form V. The crystalline Form V of Compound (I) is a nitrate salt crystalline form. The molar ratio of Compound (I) to $NO_3^-$ in Form V is approximately 1:2.

**Table 11: Form V** of Compound **(I)**

| PXRD 2θ values (CuKα) | | | | | | |
|---|---|---|---|---|---|---|
| 6.3±0.2° | 8.1±0.2° | 8.7±0.2° | 9.1±0.2° | 10.6±0.2° | 11.4±0.2° | 11.8±0.2° |
| 12.5±0.2° | 13.0±0.2° | 13.5±0.2° | 13.8±0.2° | 15.1±0.2° | 15.4±0.2° | 16.2±0.2° |
| 16.9±0.2° | 17.7±0.2° | 18.1±0.2° | 18.4±0.2° | 19.5±0.2° | 20.2±0.2° | 21.0±0.2° |
| 21.3±0.2° | 22.1±0.2° | 22.8±0.2° | 23.1±0.2° | 24.2±0.2° | 25.2±0.2° | 26.4±0.2° |
| 27.0±0.2° | 27.4±0.2° | 27.8±0.2° | 28.9±0.2° | - | - | - |

In some embodiments, crystalline Form V of Compound (I) is characterized by a powder X-ray diffraction (PXRD) pattern comprising two or more 2θ values in degrees (CuKα) selected from: 6.3±0.2, 9.1±0.2, 16.9±0.2, 25.2±0.2, 26.4 ±0.2, and 27.4±0.2, wherein the PXRD pattem of Form V is measured at room temperature.

In some embodiments, crystalline Form V of Compound (I) is characterized by a powder X-ray diffraction (PXRD) pattern comprising three or more 2θ values in degrees (CuKα) selected from: 6.3±0.2, 9.1±0.2, 16.9±0.2, 25.2±0.2, 26.4 ±0.2, and 27.4±0.2, wherein the PXRD pattem of Form V is measured at room temperature.

In some embodiments, crystalline Form V of Compound (I) is characterized by a powder X-ray diffraction (PXRD) pattern comprising four or more 2θ values in degrees (CuKα) selected from: 6.3±0.2, 9.1±0.2, 16.9±0.2, 25.2±0.2, 26.4 ±0.2, and 27.4±0.2, wherein the PXRD pattem of Form V is measured at room temperature.

In some embodiments, crystalline Form V of Compound (I) is characterized by a powder X-ray diffraction (PXRD) pattern comprising five or more 2θ values in degrees (CuKα) selected from: 6.3±0.2, 9.1±0.2, 16.9±0.2, 25.2±0.2, 26.4 ±0.2, and 27.4±0.2, wherein the PXRD pattem of Form V is measured at room temperature.

In certain embodiments, crystalline Form V of Compound (I) is characterized by a powder X-ray diffraction (PXRD) pattern comprising 2θ values in degrees (CuKα) at 6.3±0.2 and 9.1±0.2, wherein the PXRD pattern of Form V is

measured at room temperature. In further embodiments, the PXRD pattern further comprises one or more 2θ values in degrees selected from: 16.9±0.2, 25.2±0.2, 26.4±0.2, and 27.4±0.2.

In certain embodiments, crystalline Form V of Compound (I) is characterized by a powder X-ray diffraction (PXRD) pattern comprising 2θ values in degrees (CuKα) at 6.3±0.2, 9.1±0.2, and 16.9±0.2, wherein the PXRD pattern of Form V is measured at room temperature.

In further embodiments, crystalline Form V of Compound (I) is characterized by a powder X-ray diffraction (PXRD) pattern comprising 2θ values in degrees (CuKα) at 6.3±0.2, 9.1±0.2, 16.9±0.2, and 25.2±0.2, wherein the PXRD pattem of Form V is measured at room temperature.

In further embodiments, crystalline Form V of Compound (I) is characterized by a powder X-ray diffraction (PXRD) pattern comprising 2θ values in degrees (CuKα) at 6.3±0.2, 9.1±0.2, 16.9±0.2, 25.2±0.2, and 26.4±0.2, wherein the PXRD pattern of Form V is measured at room temperature.

In further embodiments, crystalline Form V of Compound (I) is characterized by a powder X-ray diffraction (PXRD) pattern comprising 2θ values in degrees (CuKα) at 6.3±0.2, 9.1±0.2, 16.9±0.2, 25.2±0.2, 26.4±0.2, and 27.4 ±0.2, wherein the PXRD pattem of Form V is measured at room temperature.

In certain embodiments, crystalline Form V of Compound (I) is characterized by an observed powder X-ray diffraction pattern (CuKα, measured at room temperature) substantially as shown in FIG. 16.

In some embodiments, the Form V of Compound (I) is substantially pure. For example, Form V of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form V. For such embodiments, crystalline Compound (I) may comprise at least about 90 weight %, preferably at least about 95 weight %, and more preferably at least about 99 weight %, of crystalline Form V.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form V.

In further embodiments, a pharmaceutical composition is provided comprising Form V of Compound (I), and at least one pharmaceutically-acceptable carrier and/or diluent.

In certain embodiments, a pharmaceutical composition comprises substantially pure Form V of Compound (I), and at least one pharmaceutically-acceptable carrier and/or diluent.

In certain embodiments, Form V of Compound (I) is combined with at least one pharmaceutically acceptable carrier and/or diluent to provide at least one pharmaceutical composition.

In some embodiments, a pharmaceutical composition comprises Form V of Compound (I) and other solid forms of Compound (I). Such other solid forms can be, for example, other crystalline forms and/or amorphous Compound (I).

**Form W of Compound** (I)

In some embodiments, Compound (I) is provided as a crystalline material comprising Form W. The crystalline Form W of Compound (I) is a benzenesulfonic acid salt crystalline form. The molar ratio of Compound (I) to benzenesulfonic acid in Form W is approximately 1:1. Crystalline Form W may be non-solvated.

**Table 12: Form W of Compound (I)**

| PXRD 2θ values (CuKα) | | | | | | |
|---|---|---|---|---|---|---|
| 7.3±0.2° | 7.7±0.2° | 8.5±0.2° | 8.8±0.2° | 9.6±0.2° | 10.1±0.2° | 10.4±0.2° |
| 11.2±0.2° | 12.3±0.2° | 13.5±0.2° | 14.7±0.2° | 15.6±0.2° | 16.9±0.2° | 17.3±0.2° |
| 17.8±0.2° | 18.6±0.2° | 19.4±0.2° | 20.8±0.2° | 21.5±0.2° | 22.0±0.2° | 22.4±0.2° |
| 22.7±0.2° | 23.5±0.2° | 23.9±0.2° | 25.2±0.2° | 25.5±0.2° | 25.8±0.2° | 26.2±0.2° |
| 27.1±0.2° | 28.3±0.2° | 29.2±0.2° | 29.7±0.2° | - | - | - |

In some embodiments, crystalline Form W of Compound (I) is characterized by a powder X-ray diffraction (PXRD) pattern comprising two or more 2θ values in degrees (CuKα) selected from: 7.7±0.2, 9.6±0.2, 14.7±0.2, 15.6±0.2, and 25.2±0.2, wherein the PXRD pattem of Form W is measured at room temperature.

In some embodiments, crystalline Form W of Compound (I) is characterized by a powder X-ray diffraction (PXRD)

pattern comprising three or more 2θ values in degrees (CuKα) selected from: 7.7±0.2, 9.6±0.2, 14.7±0.2, 15.6±0.2, and 25.2±0.2, wherein the PXRD pattem of Form W is measured at room temperature.

In some embodiments, crystalline Form W of Compound (I) is characterized by a powder X-ray diffraction (PXRD) pattern comprising four or more 2θ values in degrees (CuKα) selected from: 7.7±0.2, 9.6±0.2, 14.7±0.2, 15.6±0.2, and 25.2±0.2, wherein the PXRD pattem of Form W is measured at room temperature.

In certain embodiments, crystalline Form W of Compound (I) is characterized by a powder X-ray diffraction (PXRD) pattern comprising 2θ values in degrees (CuKα) at 7.7±0.2 and 9.6±0.2, wherein the PXRD pattern of Form W is measured at room temperature. In further embodiments, the PXRD pattern further comprises one or more 2θ values in degrees selected from: 14.7±0.2, 15.6±0.2, and 25.2±0.2.

In certain embodiments, crystalline Form W of Compound (I) is characterized by a powder X-ray diffraction (PXRD) pattern comprising 2θ values in degrees (CuKα) at 7.7±0.2, 9.6±0.2, and 15.6±0.2, wherein the PXRD pattern of Form W is measured at room temperature.

In certain embodiments, crystalline Form W of Compound (I) is characterized by a powder X-ray diffraction (PXRD) pattern comprising 2θ values in degrees (CuKα) at 7.7±0.2, 9.6±0.2, and 14.7±0.2, wherein the PXRD pattern of Form W is measured at room temperature.

In further embodiments, crystalline Form W of Compound (I) is characterized by a powder X-ray diffraction (PXRD) pattern comprising 2θ values in degrees (CuKα) at 7.7±0.2, 9.6±0.2, 14.7±0.2, and 15.6±0.2, wherein the PXRD pattem of Form W is measured at room temperature.

In further embodiments, crystalline Form W of Compound (I) is characterized by a powder X-ray diffraction (PXRD) pattern comprising 2θ values in degrees (CuKα) at 7.7±0.2, 9.6±0.2, 14.7±0.2, 15.6±0.2, and 25.2±0.2, wherein the PXRD pattern of Form W is measured at room temperature.

In certain embodiments, crystalline Form W of Compound (I) is characterized by an observed powder X-ray diffraction pattern (CuKα, measured at room temperature) substantially as shown in FIG. 17.

In some embodiments, crystalline Form W of Compound (I) is characterized by an endotherm with a peak having an onset temperature in the approximate range of from 176 °C to 186 °C. In further embodiments, the endotherm has a peak with an onset temperature that is about 181 °C. For example, in some embodiments, crystalline Form W of Compound (I) is characterized by a differential scanning calorimetry (DSC) thermogram comprising an endotherm with a peak having an onset temperature in the range of from about 176 °C to about 186 °C; in certain embodiments, the differential scanning calorimetry (DSC) thermogram comprises an endotherm with a peak having an onset temperature that is about 181 °C. It should be understood that, in some cases, the endothermic event may not be detected.

In certain embodiments, crystalline Form W of Compound (I) is characterized by (i) a powder X-ray diffraction (PXRD) pattem comprising 2θ values in degrees (CuKα) at 7.7±0.2 and 9.6±0.2, wherein the PXRD pattern of Form W is measured at room temperature; and (ii) an endotherm having a peak with an onset temperature that is about 181 °C.

In certain embodiments, crystalline Form W of Compound (I) is characterized by (i) a powder X-ray diffraction (PXRD) pattem comprising 2θ values in degrees (CuKα) at 7.7±0.2 and 9.6±0.2, wherein the PXRD pattern of Form W is measured at room temperature; and (ii) an endotherm with a peak having an onset temperature in the approximate range of from 176 °C to 186 °C. In further embodiments, the endotherm has a peak with an onset temperature that is about 181 °C.

In certain embodiments, crystalline Form W of Compound (I) is characterized by (i) a powder X-ray diffraction (PXRD) pattem comprising 2θ values in degrees (CuKα) at 7.7±0.2, 9.6±0.2, and 14.7±0.2, wherein the PXRD pattern of Form W is measured at room temperature; and (ii) an endotherm with a peak having an onset temperature in the approximate range of from 176 °C to 186 °C. In further embodiments, the endotherm has a peak with an onset temperature that is about 181 °C.

In certain embodiments, crystalline Form W of Compound (I) is characterized by (i) a powder X-ray diffraction (PXRD) pattem comprising 2θ values in degrees (CuKα) at 7.7±0.2, 9.6±0.2, and 15.6±0.2, wherein the PXRD pattern of Form W is measured at room temperature; and (ii) an endotherm with a peak having an onset temperature in the approximate range of from 176 °C to 186 °C. In further embodiments, the endotherm has a peak with an onset temperature that is about 181 °C.

In some embodiments, crystalline Form W of Compound (I) is characterized by a differential scanning calorimetry (DSC) thermogram substantially in accordance with the thermogram shown in FIG. 18 (bottom graph).

In certain embodiments, crystalline Form W of Compound (I) is characterized by (i) a powder X-ray diffraction (PXRD) pattem comprising two or more 2θ values in degrees (CuKα) selected from: 7.7±0.2, 9.6±0.2, 14.7±0.2, 15.6 ±0.2, and 25.2±0.2, wherein the PXRD pattem of Form W is measured at room temperature; and (ii) a differential scanning calorimetry (DSC) thermogram substantially in accordance with the thermogram shown in FIG. 18 (bottom graph).

In some embodiments, crystalline Form W of Compound (I) is characterized by a thermogravimetric analysis (TGA) thermogram having weight loss of less than 0.1% upon being heated (e.g., from room temperature) to a temperature of about 125 °C. In certain embodiments, crystalline Form W of Compound (I) is characterized by a thermogravimetric analysis (TGA) thermogram having weight loss of less than 0.1% upon being heated to a temperature of about 150 °C. In

certain embodiments, crystalline Form W of Compound (I) is characterized by a thermogravimetric analysis (TGA) thermogram showing less than about 1.0% weight loss, less than about 0.5% weight loss, or in further embodiments less than about 0.1% weight loss, up to a temperature of about 150 °C.

In certain embodiments, crystalline Form W of Compound (I) exhibits a thermogravimetric analysis (TGA) thermogram substantially as shown in FIG. 18 (top graph).

In some embodiments, the Form W of Compound (I) is substantially pure. For example, Form W of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form W. For such embodiments, crystalline Compound (I) may comprise at least about 90 weight %, preferably at least about 95 weight %, and more preferably at least about 99 weight %, of crystalline Form W.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form W.

In further embodiments, a pharmaceutical composition is provided comprising Form W of Compound (I), and at least one pharmaceutically-acceptable carrier and/or diluent.

In certain embodiments, a pharmaceutical composition comprises substantially pure Form W of Compound (I), and at least one pharmaceutically-acceptable carrier and/or diluent.

In certain embodiments, Form W of Compound (I) is combined with at least one pharmaceutically acceptable carrier and/or diluent to provide at least one pharmaceutical composition.

In some embodiments, a pharmaceutical composition comprises Form W of Compound (I) and other solid forms of Compound (I). Such other solid forms can be, for example, other crystalline forms and/or amorphous Compound (I).

**Form X of Compound** (I)

In some embodiments, Compound (I) is provided as a crystalline material comprising Form X. The crystalline Form X of Compound (I) is a naphthalene-1,5-disulfonic acid salt crystalline form. The molar ratio of Compound (I) to naphthalene-1,5-disulfonic acid in Form X is approximately 1:1.

**Table 13: Form X of Compound (I)**

| PXRD 2θ values (CuKα) | | | | | | |
|---|---|---|---|---|---|---|
| 5.7±0.2° | 7.6±0.2° | 9.1±0.2° | 10.3±0.2° | 10.7±0.2° | 11.3±0.2° | 14.2±0.2° |
| 14.7±0.2° | 15.2±0.2° | 15.6±0.2° | 16.2±0.2° | 17.2±0.2° | 18.0±0.2° | 18.7±0.2° |
| 19.2±0.2° | 19.7±0.2° | 20.4±0.2° | 21.0±0.2° | 21.4±0.2° | 22.5±0.2° | 23.0±0.2° |
| 23.5±0.2° | 23.8±0.2° | 24.5±0.2° | 24.9±0.2° | 25.2±0.2° | 26.0±0.2° | 26.4±0.2° |
| 27.9±0.2° | 28.7±0.2° | 29.4±0.2° | 29.8±0.2° | 30.8±0.2° | - | - |

In some embodiments, crystalline Form X of Compound (I) is characterized by a powder X-ray diffraction (PXRD) pattern comprising two or more 2θ values in degrees (CuKα) selected from: 7.6±0.2, 10.7±0.2, 14.7±0.2, 16.2±0.2, 23.8 ±0.2, and 26.4±0.2, wherein the PXRD pattem of Form X is measured at room temperature.

In some embodiments, crystalline Form X of Compound (I) is characterized by a powder X-ray diffraction (PXRD) pattern comprising three or more 2θ values in degrees (CuKα) selected from: 7.6±0.2, 10.7±0.2, 14.7±0.2, 16.2±0.2, 23.8±0.2, and 26.4±0.2, wherein the PXRD pattem of Form X is measured at room temperature.

In some embodiments, crystalline Form X of Compound (I) is characterized by a powder X-ray diffraction (PXRD) pattern comprising four or more 2θ values in degrees (CuKα) selected from: 7.6±0.2, 10.7±0.2, 14.7±0.2, 16.2±0.2, 23.8 ±0.2, and 26.4±0.2, wherein the PXRD pattem of Form X is measured at room temperature.

In some embodiments, crystalline Form X of Compound (I) is characterized by a powder X-ray diffraction (PXRD) pattern comprising five or more 2θ values in degrees (CuKα) selected from: 7.6±0.2, 10.7±0.2, 14.7±0.2, 16.2±0.2, 23.8 ±0.2, and 26.4±0.2, wherein the PXRD pattem of Form X is measured at room temperature.

In certain embodiments, crystalline Form X of Compound (I) is characterized by a powder X-ray diffraction (PXRD) pattern comprising 2θ values in degrees (CuKα) at 7.6±0.2 and 10.7±0.2, wherein the PXRD pattern of Form X is measured at room temperature. In further embodiments, the PXRD pattern further comprises one or more 2θ values in

degrees selected from: 14.7±0.2, 16.2±0.2, 23.8±0.2, and 26.4±0.2.

In further embodiments, crystalline Form X of Compound (I) is characterized by a powder X-ray diffraction (PXRD) pattern comprising 2θ values in degrees (CuKα) at 7.6±0.2, 10.7±0.2, and 14.7±0.2, wherein the PXRD pattern of Form X is measured at room temperature.

In further embodiments, crystalline Form X of Compound (I) is characterized by a powder X-ray diffraction (PXRD) pattern comprising 2θ values in degrees (CuKα) at 7.6±0.2, 10.7±0.2, 14.7±0.2, and 16.2±0.2, wherein the PXRD pattem of Form X is measured at room temperature.

In further embodiments, crystalline Form X of Compound (I) is characterized by a powder X-ray diffraction (PXRD) pattern comprising 2θ values in degrees (CuKα) at 7.6±0.2, 10.7±0.2, 14.7±0.2, 16.2±0.2, and 23.8±0.2, wherein the PXRD pattern of Form X is measured at room temperature.

In certain embodiments, crystalline Form X of Compound (I) is characterized by a powder X-ray diffraction (PXRD) pattern comprising 2θ values in degrees (CuKα) at 7.6±0.2, 10.7±0.2, 14.7±0.2, and 23.8±0.2, wherein the PXRD pattem of Form X is measured at room temperature.

In further embodiments, crystalline Form X of Compound (I) is characterized by a powder X-ray diffraction (PXRD) pattern comprising 2θ values in degrees (CuKα) at 7.6±0.2, 10.7±0.2, 14.7±0.2, 16.2±0.2, 23.8±0.2, and 26.4 ±0.2, wherein the PXRD pattem of Form X is measured at room temperature.

In certain embodiments, crystalline Form X of Compound (I) is characterized by an observed powder X-ray diffraction pattern (CuKα, measured at room temperature) substantially as shown in FIG. 19.

In some embodiments, the Form X of Compound (I) is substantially pure. For example, Form X of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form X. For such embodiments, crystalline Compound (I) may comprise at least about 90 weight %, preferably at least about 95 weight %, and more preferably at least about 99 weight %, of crystalline Form X.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form X.

In further embodiments, a pharmaceutical composition is provided comprising Form X of Compound (I), and at least one pharmaceutically-acceptable carrier and/or diluent.

In certain embodiments, a pharmaceutical composition comprises substantially pure Form X of Compound (I), and at least one pharmaceutically-acceptable carrier and/or diluent.

In certain embodiments, Form X of Compound (I) is combined with at least one pharmaceutically acceptable carrier and/or diluent to provide at least one pharmaceutical composition.

In some embodiments, a pharmaceutical composition comprises Form X of Compound (I) and other solid forms of Compound (I). Such other solid forms can be, for example, other crystalline forms and/or amorphous Compound (I).

## Form Y of Compound (I)

In some embodiments, Compound (I) is provided as a crystalline material comprising Form Y. The crystalline Form Y of Compound (I) is an ethanesulfonic acid salt crystalline form.

**Table 14: Form Y of Compound (I)**

| PXRD 2θ values (CuKα) | | | | | | |
|---|---|---|---|---|---|---|
| 8.5±0.2° | 9.1±0.2° | 10.1±0.2° | 11.9±0.2° | 12.6±0.2° | 15.0±0.2° | 16.0±0.2° |
| 16.7±0.2° | 17.1±0.2° | 17.6±0.2° | 18.4±0.2° | 18.7±0.2° | 19.1±0.2° | 19.7±0.2° |
| 20.3±0.2° | 21.4±0.2° | 22.3±0.2° | 22.9±0.2° | 23.3±0.2° | 24.6±0.2° | 25.5±0.2° |
| 26.3±0.2° | 28.0±0.2° | 29.5±0.2° | 29.9±0.2° | - | - | - |

In some embodiments, crystalline Form Y of Compound (I) is characterized by a powder X-ray diffraction (PXRD) pattern comprising two or more 2θ values in degrees (CuKα) selected from: 8.5±0.2, 9.1±0.2, 11.9±0.2, 19.7±0.2, and 24.6±0.2, wherein the PXRD pattern of Form Y is measured at room temperature.

In some embodiments, crystalline Form Y of Compound (I) is characterized by a powder X-ray diffraction (PXRD)

pattern comprising three or more 2θ values in degrees (CuKα) selected from: 8.5±0.2, 9.1±0.2, 11.9±0.2, 19.7±0.2, and 24.6±0.2, wherein the PXRD pattern of Form Y is measured at room temperature.

In some embodiments, crystalline Form Y of Compound (I) is characterized by a powder X-ray diffraction (PXRD) pattern comprising four or more 2θ values in degrees (CuKα) selected from: 8.5±0.2, 9.1±0.2, 11.9±0.2, 19.7±0.2, and 24.6±0.2, wherein the PXRD pattern of Form Y is measured at room temperature.

In certain embodiments, crystalline Form Y of Compound (I) is characterized by a powder X-ray diffraction (PXRD) pattern comprising 2θ values in degrees (CuKα) at 8.5±0.2 and 9.1±0.2, wherein the PXRD pattern of Form Y is measured at room temperature. In further embodiments, the PXRD pattern further comprises one or more 2θ values in degrees selected from: 11.9±0.2, 19.7±0.2, and 24.6±0.2.

In certain embodiments, crystalline Form Y of Compound (I) is characterized by a powder X-ray diffraction (PXRD) pattern comprising 2θ values in degrees (CuKα) at 8.5±0.2, 9.1±0.2, and 11.9+0.2, wherein the PXRD pattern of Form Y is measured at room temperature.

In certain embodiments, crystalline Form Y of Compound (I) is characterized by a powder X-ray diffraction (PXRD) pattern comprising 2θ values in degrees (CuKα) at 8.5±0.2, 9.1±0.2, and 19.7±0.2, wherein the PXRD pattern of Form Y is measured at room temperature.

In certain embodiments, crystalline Form Y of Compound (I) is characterized by a powder X-ray diffraction (PXRD) pattern comprising 2θ values in degrees (CuKα) at 9.1±0.2, 19.7±0.2, and 24.6±0.2, wherein the PXRD pattern of Form Y is measured at room temperature.

In certain embodiments, crystalline Form Y of Compound (I) is characterized by a powder X-ray diffraction (PXRD) pattern comprising 2θ values in degrees (CuKα) at 8.5±0.2, 9.1±0.2, 11.9±0.2, and 19.7±0.2, wherein the PXRD pattern of Form Y is measured at room temperature.

In further embodiments, crystalline Form Y of Compound (I) is characterized by a powder X-ray diffraction (PXRD) pattern comprising 2θ values in degrees (CuKα) at 8.5±0.2, 9.1±0.2, 11.9±0.2, 19.7±0.2, and 24.6±0.2, wherein the PXRD pattern of Form Y is measured at room temperature.

In certain embodiments, crystalline Form Y of Compound (I) is characterized by an observed powder X-ray diffraction pattern (CuKα, measured at room temperature) substantially as shown in FIG. 20.

In some embodiments, the Form Y of Compound (I) is substantially pure. For example, Form Y of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form Y. For such embodiments, crystalline Compound (I) may comprise at least about 90 weight %, preferably at least about 95 weight %, and more preferably at least about 99 weight %, of crystalline Form Y.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form Y.

In further embodiments, a pharmaceutical composition is provided comprising Form Y of Compound (I), and at least one pharmaceutically-acceptable carrier and/or diluent.

In certain embodiments, a pharmaceutical composition comprises substantially pure Form Y of Compound (I), and at least one pharmaceutically-acceptable carrier and/or diluent.

In certain embodiments, Form Y of Compound (I) is combined with at least one pharmaceutically acceptable carrier and/or diluent to provide at least one pharmaceutical composition.

In some embodiments, a pharmaceutical composition comprises Form Y of Compound (I) and other solid forms of Compound (I). Such other solid forms can be, for example, other crystalline forms and/or amorphous Compound (I).

**Form Z of Compound (I)**

In some embodiments, Compound (I) is provided as a crystalline material comprising Form Z. The crystalline Form Z of Compound (I) is a mono-sulfate salt trihydrate crystalline form.

In certain embodiments, crystalline Form Z of Compound (I) is characterized by unit cell parameters approximately equal to the following:

Unit cell dimensions (triclinic crystal system):

$$a = 8.76 \pm 0.10\ \text{Å}$$

$$b = 11.79 \pm 0.10\ \text{Å}$$

$$c = 12.68 \pm 0.10\ \text{Å}$$

$$\alpha = 77.5 \pm 1.0°$$

$$\beta = 89.9 \pm 1.0°$$

$$\gamma = 86.5 \pm 1.0°$$

Space group: P-1
Molecules per unit cell (Z): 2
Unit cell volume = 1277 ± 20 $Å^3$
Density (calculated) = 1.502 $g/cm^3$,
wherein the unit cell parameters of Form Z of Compound (I) are measured at a temperature of about 296 K.

**Table 15: Form Z of Compound (I)**

| PXRD 2θ values (CuKα) | | | | | | |
|---|---|---|---|---|---|---|
| 7.1±0.2° | 7.7±0.2° | 10.1±0.2° | 12.3±0.2° | 15.5±0.2° | 17.5±0.2° | 18.0±0.2° |
| 19.0±0.2° | 20.0±0.2° | 21.5±0.2° | 22.0±0.2° | 23.7±0.2° | 24.8±0.2° | 25.4±0.2° |
| 26.1±0.2° | 26.5±0.2° | 27.1±0.2° | 27.5±0.2° | 28.3±0.2° | - | - |

In some embodiments, crystalline Form Z of Compound (I) is characterized by a powder X-ray diffraction (PXRD) pattern comprising two or more 2θ values in degrees (CuKα) selected from: 7.7±0.2, 15.5±0.2, 19.0±0.2, and 20.0±0.2, wherein the PXRD pattern of Form Z is measured at room temperature.

In some embodiments, crystalline Form Z of Compound (I) is characterized by a powder X-ray diffraction (PXRD) pattern comprising three or more 2θ values in degrees (CuKα) selected from: 7.7±0.2, 15.5±0.2, 19.0±0.2, and 20.0 ±0.2, wherein the PXRD pattern of Form Z is measured at room temperature.

In certain embodiments, crystalline Form Z of Compound (I) is characterized by a powder X-ray diffraction (PXRD) pattern comprising 2θ values in degrees (CuKα) at 7.7±0.2 and 15.5±0.2, wherein the PXRD pattern of Form Z is measured at room temperature. In further embodiments, the PXRD pattern further comprises one or more 2θ values in degrees selected from: 19.0±0.2 and 20.0±0.2.

In certain embodiments, crystalline Form Z of Compound (I) is characterized by a powder X-ray diffraction (PXRD) pattern comprising 2θ values in degrees (CuKα) at 7.7±0.2, 15.5±0.2, and 19.0±0.2, wherein the PXRD pattern of Form Z is measured at room temperature.

In certain embodiments, crystalline Form Z of Compound (I) is characterized by a powder X-ray diffraction (PXRD) pattern comprising 2θ values in degrees (CuKα) at 7.7±0.2, 15.5±0.2, and 20.0±0.2, wherein the PXRD pattern of Form Z is measured at room temperature.

In certain embodiments, crystalline Form Z of Compound (I) is characterized by a powder X-ray diffraction (PXRD) pattern comprising 2θ values in degrees (CuKα) at 7.7±0.2, 19.0±0.2, and 20.0±0.2, wherein the PXRD pattern of Form Z is measured at room temperature.

In certain embodiments, crystalline Form Z of Compound (I) is characterized by a powder X-ray diffraction (PXRD) pattern comprising 2θ values in degrees (CuKα) at 7.7±0.2, 15.5±0.2, 19.0±0.2, and 20.0±0.2, wherein the PXRD pattem of Form Z is measured at room temperature.

In certain embodiments, crystalline Form Z of Compound (I) is characterized by an observed powder X-ray diffraction pattern (CuKα, measured at room temperature) substantially as shown in FIG. 21.

In some embodiments, the Form Z of Compound (I) is substantially pure. For example, Form Z of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form Z. For such

embodiments, crystalline Compound (I) may comprise at least about 90 weight %, preferably at least about 95 weight %, and more preferably at least about 99 weight %, of crystalline Form Z.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form Z.

In further embodiments, a pharmaceutical composition is provided comprising Form Z of Compound (I), and at least one pharmaceutically-acceptable carrier and/or diluent.

In certain embodiments, a pharmaceutical composition comprises substantially pure Form Z of Compound (I), and at least one pharmaceutically-acceptable carrier and/or diluent.

In certain embodiments, Form Z of Compound (I) is combined with at least one pharmaceutically acceptable carrier and/or diluent to provide at least one pharmaceutical composition.

In some embodiments, a pharmaceutical composition comprises Form Z of Compound (I) and other solid forms of Compound (I). Such other solid forms can be, for example, other crystalline forms and/or amorphous Compound (I).

**Form AA of Compound (I)**

In some embodiments, Compound (I) is provided as a crystalline material comprising Form AA. The crystalline Form AA of Compound (I) is a sulfate salt crystalline form.

**Table 16: Form AA of Compound** (I)

| PXRD 2θ values (CuKα) | | | | | | |
|---|---|---|---|---|---|---|
| 6.3±0.2° | 7.7±0.2° | 8.2±0.2° | 11.6±0.2° | 12.7±0.2° | 13.2±0.2° | 14.6±0.2° |
| 15.0±0.2° | 16.2±0.2° | 16.8±0.2° | 18.2±0.2° | 19.0±0.2° | 20.3±0.2° | 21.3±0.2° |
| 22.2±0.2° | 22.8±0.2° | 23.3±0.2° | 24.5±0.2° | 25.4±0.2° | 26.6±0.2° | 27.5±0.2° |
| 28.7±0.2° | 29.4±0.2° | - | - | - | - | - |

In some embodiments, crystalline Form AA of Compound (I) is characterized by a powder X-ray diffraction (PXRD) pattern comprising two or more 2θ values in degrees (CuKα) selected from: 6.3±0.2, 7.7±0.2, 8.2±0.2, 11.6 ±0.2, and 25.4±0.2, wherein the PXRD pattern of Form AA is measured at room temperature.

In some embodiments, crystalline Form AA of Compound (I) is characterized by a powder X-ray diffraction (PXRD) pattern comprising three or more 2θ values in degrees (CuKα) selected from: 6.3±0.2, 7.7±0.2, 8.2±0.2, 11.6 ±0.2, and 25.4±0.2, wherein the PXRD pattern of Form AA is measured at room temperature.

In some embodiments, crystalline Form AA of Compound (I) is characterized by a powder X-ray diffraction (PXRD) pattern comprising four or more 2θ values in degrees (CuKα) selected from: 6.3±0.2, 7.7±0.2, 8.2±0.2, 11.6 ±0.2, and 25.4±0.2, wherein the PXRD pattern of Form AA is measured at room temperature.

In certain embodiments, crystalline Form AA of Compound (I) is characterized by a powder X-ray diffraction (PXRD) pattern comprising 2θ values in degrees (CuKα) at 6.3±0.2 and 7.7±0.2, wherein the PXRD pattern of Form AA is measured at room temperature. In further embodiments, the PXRD pattern further comprises one or more 2θ values in degrees selected from: 8.2±0.2, 11.6±0.2, and 25.4±0.2.

In certain embodiments, crystalline Form AA of Compound (I) is characterized by a powder X-ray diffraction (PXRD) pattern comprising 2θ values in degrees (CuKα) at 6.3±0.2, 7.7±0.2, and 8.2±0.2, wherein the PXRD pattern of Form AA is measured at room temperature.

In certain embodiments, crystalline Form AA of Compound (I) is characterized by a powder X-ray diffraction (PXRD) pattern comprising 2θ values in degrees (CuKα) at 7.7±0.2 and 8.2±0.2, wherein the PXRD pattern of Form AA is measured at room temperature. In further embodiments, the PXRD pattern further comprises one or more 2θ values in degrees selected from: 6.3±0.2, 11.6±0.2, and 25.4±0.2.

In certain embodiments, crystalline Form AA of Compound (I) is characterized by a powder X-ray diffraction (PXRD) pattern comprising 2θ values in degrees (CuKα) at 6.3±0.2, 7.7±0.2, 8.2±0.2, and 11.6±0.2, wherein the PXRD pattern of Form AA is measured at room temperature.

In further embodiments, crystalline Form AA of Compound (I) is characterized by a powder X-ray diffraction (PXRD) pattern comprising 2θ values in degrees (CuKα) at 6.3±0.2, 7.7±0.2, 8.2±0.2, 11.6±0.2, and 25.4±0.2, wherein the PXRD pattern of Form AA is measured at room temperature.

In certain embodiments, crystalline Form AA of Compound (I) is characterized by an observed powder X-ray

diffraction pattern (CuKα, measured at room temperature) substantially as shown in FIG. 22.

In certain embodiments, crystalline Form AA of Compound (I) is characterized by an endotherm with a peak maximum in the approximate range of from 189 °C to 199 °C. In further embodiments, the endotherm maximum is at about 194 °C. For example, in some embodiments, crystalline Form AA of Compound (I) is characterized by a differential scanning calorimetry (DSC) thermogram comprising an endotherm with a maximum in the range of from about 189 °C to about 199 °C; in certain embodiments, the differential scanning calorimetry (DSC) thermogram comprises an endotherm with a maximum at about 194 °C. It should be understood that, in some cases, the endothermic event may not be detected.

In certain embodiments, crystalline Form AA of Compound (I) is characterized by (i) a powder X-ray diffraction (PXRD) pattern comprising 2θ values in degrees (CuKα) at 6.3±0.2 and 7.7±0.2, wherein the PXRD pattern of Form AA is measured at room temperature; and (ii) an endotherm with a peak maximum in the approximate range of from 189 °C to 199 °C. In further embodiments, the endotherm peak maximum is at about 194 °C.

In certain embodiments, crystalline Form AA of Compound (I) is characterized by (i) a powder X-ray diffraction (PXRD) pattern comprising 2θ values in degrees (CuKα) at 7.7±0.2 and 8.2±0.2, wherein the PXRD pattern of Form AA is measured at room temperature; and (ii) an endotherm with a peak maximum in the approximate range of from 189 °C to 199 °C. In further embodiments, the endotherm peak maximum is at about 194 °C.

In certain embodiments, crystalline Form AA of Compound (I) is characterized by (i) a powder X-ray diffraction (PXRD) pattern comprising 2θ values in degrees (CuKα) at 7.7±0.2, 8.2±0.2, and 11.6±0.2, wherein the PXRD pattern of Form AA is measured at room temperature; and (ii) an endotherm with a peak maximum in the approximate range of from 189 °C to 199 °C. In further embodiments, the endotherm peak maximum is at about 194 °C.

In certain embodiments, crystalline Form AA of Compound (I) is characterized by (i) a powder X-ray diffraction (PXRD) pattern comprising 2θ values in degrees (CuKα) at 6.3±0.2, 7.7±0.2, and 8.2±0.2, wherein the PXRD pattern of Form AA is measured at room temperature; and (ii) an endotherm with a peak maximum in the approximate range of from 189 °C to 199 °C. In further embodiments, the endotherm peak maximum is at about 194 °C.

In some embodiments, crystalline Form AA of Compound (I) is characterized by a differential scanning calorimetry (DSC) thermogram substantially in accordance with the thermogram shown in FIG. 23.

In certain embodiments, crystalline Form AA of Compound (I) is characterized by (i) a powder X-ray diffraction (PXRD) pattern comprising two or more 2θ values in degrees (CuKα) selected from: 6.3±0.2, 7.7±0.2, 8.2±0.2, 11.6 ±0.2, and 25.4±0.2, wherein the PXRD pattern of Form AA is measured at room temperature; and (ii) a differential scanning calorimetry (DSC) thermogram substantially in accordance with the thermogram shown in FIG. 23.

In some embodiments, the Form AA of Compound (I) is substantially pure. For example, Form AA of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form AA. For such embodiments, crystalline Compound (I) may comprise at least about 90 weight %, preferably at least about 95 weight %, and more preferably at least about 99 weight %, of crystalline Form AA.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form AA.

In further embodiments, a pharmaceutical composition is provided comprising Form AA of Compound (I), and at least one pharmaceutically-acceptable carrier and/or diluent.

In certain embodiments, a pharmaceutical composition comprises substantially pure Form AA of Compound (I), and at least one pharmaceutically-acceptable carrier and/or diluent.

In certain embodiments, Form AA of Compound (I) is combined with at least one pharmaceutically acceptable carrier and/or diluent to provide at least one pharmaceutical composition.

In some embodiments, a pharmaceutical composition comprises Form AA of Compound (I) and other solid forms of Compound (I). Such other solid forms can be, for example, other crystalline forms and/or amorphous Compound (I).

## Form AB of Compound (I)

In some embodiments, Compound (I) is provided as a crystalline material comprising Form AB. The crystalline Form AB of Compound (I) is a sulfate salt crystalline form. Crystalline Form AB may be non-solvated.

**Table 17: Form AB of Compound (I)**

| PXRD 2θ values (CuKα) | | | | | | |
|---|---|---|---|---|---|---|
| 7.8±0.2° | 9.7±0.2° | 10.3±0.2° | 11.4±0.2° | 13.8±0.2° | 14.3±0.2° | 15.6±0.2° |
| 16.6±0.2° | 17.7±0.2° | 18.1±0.2° | 18.6±0.2° | 19.1±0.2° | 19.8±0.2° | 20.2±0.2° |
| 20.7±0.2° | 21.6±0.2° | 22.5±0.2° | 23.8±0.2° | 25.0±0.2° | 25.5±0.2° | 26.2±0.2° |
| 27.2±0.2° | 28.5±0.2° | 29.7±0.2° | - | - | - | - |

In some embodiments, crystalline Form AB of Compound (I) is characterized by a powder X-ray diffraction (PXRD) pattern comprising two or more 2θ values in degrees (CuKα) selected from: 7.8±0.2, 9.7±0.2, 10.3±0.2, 11.4 ±0.2, and 23.8±0.2, wherein the PXRD pattern of Form AB is measured at room temperature.

In some embodiments, crystalline Form AB of Compound (I) is characterized by a powder X-ray diffraction (PXRD) pattern comprising three or more 2θ values in degrees (CuKα) selected from: 7.8±0.2, 9.7±0.2, 10.3±0.2, 11.4 ±0.2, and 23.8±0.2, wherein the PXRD pattern of Form AB is measured at room temperature.

In some embodiments, crystalline Form AB of Compound (I) is characterized by a powder X-ray diffraction (PXRD) pattern comprising four or more 2θ values in degrees (CuKα) selected from: 7.8±0.2, 9.7±0.2, 10.3±0.2, 11.4 ±0.2, and 23.8±0.2, wherein the PXRD pattern of Form AB is measured at room temperature.

In certain embodiments, crystalline Form AB of Compound (I) is characterized by a powder X-ray diffraction (PXRD) pattern comprising 2θ values in degrees (CuKα) at 7.8±0.2 and 9.7±0.2, wherein the PXRD pattern of Form AB is measured at room temperature. In further embodiments, the PXRD pattern further comprises one or more 2θ values in degrees selected from: 10.3±0.2, 11.4±0.2, and 23.8±0.2.

In certain embodiments, crystalline Form AB of Compound (I) is characterized by a powder X-ray diffraction (PXRD) pattern comprising 2θ values in degrees (CuKα) at 7.8±0.2, 9.7±0.2, and 23.8±0.2, wherein the PXRD pattern of Form AB is measured at room temperature.

In certain embodiments, crystalline Form AB of Compound (I) is characterized by a powder X-ray diffraction (PXRD) pattern comprising 2θ values in degrees (CuKα) at 7.8±0.2, 9.7±0.2, and 10.3±0.2, wherein the PXRD pattern of Form AB is measured at room temperature.

In further embodiments, crystalline Form AB of Compound (I) is characterized by a powder X-ray diffraction (PXRD) pattern comprising 2θ values in degrees (CuKα) at 7.8±0.2, 9.7±0.2, 10.3±0.2, and 11.4±0.2, wherein the PXRD pattern of Form AB is measured at room temperature.

In further embodiments, crystalline Form AB of Compound (I) is characterized by a powder X-ray diffraction (PXRD) pattern comprising 2θ values in degrees (CuKα) at 7.8±0.2, 9.7±0.2, 10.3±0.2, 11.4±0.2, and 23.8±0.2, wherein the PXRD pattern of Form AB is measured at room temperature.

In certain embodiments, crystalline Form AB of Compound (I) is characterized by an observed powder X-ray diffraction pattern (CuKα, measured at room temperature) substantially as shown in FIG. 24.

In certain embodiments, crystalline Form AB of Compound (I) is characterized by an endotherm with a peak maximum in the approximate range of from 217 °C to 227 °C. In further embodiments, the endotherm maximum is at about 222 °C. For example, in some embodiments, crystalline Form AB of Compound (I) is characterized by a differential scanning calorimetry (DSC) thermogram comprising an endotherm with a maximum in the range of from about 217 °C to about 227 °C; in certain embodiments, the differential scanning calorimetry (DSC) thermogram comprises an endotherm with a maximum at about 222 °C. It should be understood that, in some cases, the endothermic event may not be detected.

In certain embodiments, crystalline Form AB of Compound (I) is characterized by (i) a powder X-ray diffraction (PXRD) pattern comprising 2θ values in degrees (CuKα) at 7.8±0.2 and 9.7±0.2, wherein the PXRD pattern of Form AB is measured at room temperature; and (ii) an endotherm with a peak maximum in the approximate range of from 217 °C to 227 °C. In further embodiments, the endotherm peak maximum is at about 222 °C.

In certain embodiments, crystalline Form AB of Compound (I) is characterized by (i) a powder X-ray diffraction (PXRD) pattern comprising 2θ values in degrees (CuKα) at 7.8±0.2, 9.7±0.2, and 10.3±0.2, wherein the PXRD pattern of Form AB is measured at room temperature; and (ii) an endotherm with a peak maximum in the approximate range of from 217 °C to 227 °C. In further embodiments, the endotherm peak maximum is at about 222 °C.

In certain embodiments, crystalline Form AB of Compound (I) is characterized by (i) a powder X-ray diffraction (PXRD) pattern comprising 2θ values in degrees (CuKα) at 7.8±0.2, 9.7±0.2, and 23.8±0.2, wherein the PXRD pattern of Form AB is measured at room temperature; and (ii) an endotherm with a peak maximum in the approximate range of from 217 °C to 227 °C. In further embodiments, the endotherm peak maximum is at about 222 °C.

In some embodiments, crystalline Form AB of Compound (I) is characterized by a differential scanning calorimetry (DSC) thermogram substantially in accordance with the thermogram shown in FIG. 25.

In certain embodiments, crystalline Form AB of Compound (I) is characterized by (i) a powder X-ray diffraction

(PXRD) pattern comprising two or more 2θ values in degrees (CuKα) selected from: 7.8±0.2, 9.7±0.2, 10.3±0.2, 11.4 ±0.2, and 23.8±0.2, wherein the PXRD pattern of Form AB is measured at room temperature; and (ii) a differential scanning calorimetry (DSC) thermogram substantially in accordance with the thermogram shown in FIG. 25.

In some embodiments, crystalline Form AB of Compound (I) is characterized by a thermogravimetric analysis (TGA) thermogram having weight loss of less than 0.1% upon being heated (e.g., from room temperature) to a temperature of about 100 °C. In certain embodiments, crystalline Form AB of Compound (I) is characterized by a thermogravimetric analysis (TGA) thermogram having weight loss of less than 0.1% upon being heated to a temperature of about 120 °C. In certain embodiments, crystalline Form AB of Compound (I) is characterized by a thermogravimetric analysis (TGA) thermogram showing less than about 1.0% weight loss, less than about 0.5% weight loss, or in further embodiments less than about 0.1% weight loss, up to a temperature of about 120 °C.

In certain embodiments, crystalline Form AB of Compound (I) exhibits a thermogravimetric analysis (TGA) thermogram substantially as shown in FIG. 26.

In some embodiments, the Form AB of Compound (I) is substantially pure. For example, Form AB of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form AB. For such embodiments, crystalline Compound (I) may comprise at least about 90 weight %, preferably at least about 95 weight %, and more preferably at least about 99 weight %, of crystalline Form AB.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form AB.

In further embodiments, a pharmaceutical composition is provided comprising Form AB of Compound (I), and at least one pharmaceutically-acceptable carrier and/or diluent.

In certain embodiments, a pharmaceutical composition comprises substantially pure Form AB of Compound (I), and at least one pharmaceutically-acceptable carrier and/or diluent.

In certain embodiments, Form AB of Compound (I) is combined with at least one pharmaceutically acceptable carrier and/or diluent to provide at least one pharmaceutical composition.

In some embodiments, a pharmaceutical composition comprises Form AB of Compound (I) and other solid forms of Compound (I). Such other solid forms can be, for example, other crystalline forms and/or amorphous Compound (I).

**Form AC of Compound (I)**

In some embodiments, Compound (I) is provided as a crystalline material comprising Form AC. The crystalline Form AC of Compound (I) is a monohydrochloride salt trihydrate crystalline form.

In certain embodiments, crystalline Form AC of Compound (I) is characterized by unit cell parameters approximately equal to the following:

Unit cell dimensions (triclinic crystal system):

$$a = 8.76 \pm 0.10\ \text{Å}$$

$$b = 11.75 \pm 0.10\ \text{Å}$$

$$c = 12.31 \pm 0.10\ \text{Å}$$

$$\alpha = 105.5 \pm 1.0°$$

$$\beta = 96.3 \pm 1.0°$$

$$\gamma = 95.0 \pm 1.0°$$

Space group: P-1
Molecules per unit cell (Z): 2
Unit cell volume = 1205 ± 20 $Å^3$
Density (calculated) = 1.422 $g/cm^3$,
wherein the unit cell parameters of Form AC of Compound (I) are measured at a temperature of about 296 K.

**Table 18: Form AC of Compound (I)**

| PXRD 2θ values (CuKα) | | | | | | |
|---|---|---|---|---|---|---|
| 7.6±0.2° | 10.2±0.2° | 13.7±0.2° | 15.0±0.2° | 18.0±0.2° | 19.0±0.2° | 19.4±0.2° |
| 20.4±0.2° | 20.9±0.2° | 22.4±0.2° | 23.7±0.2° | 24.2±0.2° | 25.9±0.2° | 26.4±0.2° |
| 26.7±0.2° | 27.3±0.2° | 29.6±0.2° | - | - | - | - |

In some embodiments, crystalline Form AC of Compound (I) is characterized by a powder X-ray diffraction (PXRD) pattern comprising two or more 2θ values in degrees (CuKα) selected from: 7.6±0.2, 10.2±0.2, 13.7±0.2, 20.4 ±0.2, and 25.9±0.2, wherein the PXRD pattern of Form AC is measured at room temperature.

In some embodiments, crystalline Form AC of Compound (I) is characterized by a powder X-ray diffraction (PXRD) pattern comprising three or more 2θ values in degrees (CuKα) selected from: 7.6±0.2, 10.2±0.2, 13.7±0.2, 20.4 ±0.2, and 25.9±0.2, wherein the PXRD pattern of Form AC is measured at room temperature.

In some embodiments, crystalline Form AC of Compound (I) is characterized by a powder X-ray diffraction (PXRD) pattern comprising four or more 2θ values in degrees (CuKα) selected from: 7.6±0.2, 10.2±0.2, 13.7±0.2, 20.4 ±0.2, and 25.9±0.2, wherein the PXRD pattern of Form AC is measured at room temperature.

In certain embodiments, crystalline Form AC of Compound (I) is characterized by a powder X-ray diffraction (PXRD) pattern comprising 2θ values in degrees (CuKα) at 7.6±0.2 and 10.2±0.2, wherein the PXRD pattern of Form AC is measured at room temperature. In further embodiments, the PXRD pattern further comprises one or more 2θ values in degrees selected from: 13.7±0.2, 20.4±0.2, and 25.9±0.2.

In certain embodiments, crystalline Form AC of Compound (I) is characterized by a powder X-ray diffraction (PXRD) pattern comprising 2θ values in degrees (CuKα) at 7.6±0.2 and 13.7±0.2, wherein the PXRD pattern of Form AC is measured at room temperature. In further embodiments, the PXRD pattern further comprises one or more 2θ values in degrees selected from: 10.2±0.2, 20.4±0.2, and 25.9±0.2.

In certain embodiments, crystalline Form AC of Compound (I) is characterized by a powder X-ray diffraction (PXRD) pattern comprising 2θ values in degrees (CuKα) at 7.6±0.2 and 25.9±0.2, wherein the PXRD pattern of Form AC is measured at room temperature. In further embodiments, the PXRD pattern further comprises one or more 2θ values in degrees selected from: 10.2±0.2, 13.7±0.2, and 20.4±0.2.

In certain embodiments, crystalline Form AC of Compound (I) is characterized by a powder X-ray diffraction (PXRD) pattern comprising 2θ values in degrees (CuKα) at 7.6±0.2, 10.2±0.2, and 13.7±0.2, wherein the PXRD pattern of Form AC is measured at room temperature.

In further embodiments, crystalline Form AC of Compound (I) is characterized by a powder X-ray diffraction (PXRD) pattern comprising 2θ values in degrees (CuKα) at 7.6±0.2, 10.2±0.2, 13.7±0.2, and 20.4±0.2, wherein the PXRD pattern of Form AC is measured at room temperature.

In further embodiments, crystalline Form AC of Compound (I) is characterized by a powder X-ray diffraction (PXRD) pattern comprising 2θ values in degrees (CuKα) at 7.6±0.2, 10.2±0.2, 13.7±0.2, 20.4±0.2, and 25.9±0.2, wherein the PXRD pattern of Form AC is measured at room temperature.

In certain embodiments, crystalline Form AC of Compound (I) is characterized by an observed powder X-ray diffraction pattern (CuKα, measured at room temperature) substantially as shown in FIG. 27.

In some embodiments, the Form AC of Compound (I) is substantially pure. For example, Form AC of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form AC. For such embodiments, crystalline Compound (I) may comprise at least about 90 weight %, preferably at least about 95 weight %, and more preferably at least about 99 weight %, of crystalline Form AC.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in

crystalline Form AC.

In further embodiments, a pharmaceutical composition is provided comprising Form AC of Compound (I), and at least one pharmaceutically-acceptable carrier and/or diluent.

In certain embodiments, a pharmaceutical composition comprises substantially pure Form AC of Compound (I), and at least one pharmaceutically-acceptable carrier and/or diluent.

In certain embodiments, Form AC of Compound (I) is combined with at least one pharmaceutically acceptable carrier and/or diluent to provide at least one pharmaceutical composition.

In some embodiments, a pharmaceutical composition comprises Form AC of Compound (I) and other solid forms of Compound (I). Such other solid forms can be, for example, other crystalline forms and/or amorphous Compound (I).

**Form AD of Compound (I)**

In some embodiments, Compound (I) is provided as a crystalline material comprising Form AD. The crystalline Form AD of Compound (I) is a mesylate salt crystalline form.

**Table 19: Form AD of Compound (I)**

| PXRD 2θ values (CuKα) | | | | | | |
|---|---|---|---|---|---|---|
| 6.0±0.2° | 6.9±0.2° | 7.8±0.2° | 9.5±0.2° | 10.3±0.2° | 11.2+0.2° | 12.0±0.2° |
| 12.5±0.2° | 12.8±0.2° | 13.0±0.2° | 13.8±0.2° | 14.7±0.2° | 15.6±0.2° | 16.2±0.2° |
| 17.0±0.2° | 17.6±0.2° | 18.5±0.2° | 19.1±0.2° | 19.7±0.2° | 20.5±0.2° | 21.1±0.2° |
| 24.9±0.2° | 26.5±0.2° | 27.4±0.2° | 28.8±0.2° | - | - | - |

In some embodiments, crystalline Form AD of Compound (I) is characterized by a powder X-ray diffraction (PXRD) pattern comprising two or more 2θ values in degrees (CuKα) selected from: 6.0±0.2, 6.9±0.2, 7.8±0.2, 10.3 ±0.2, and 13.8±0.2, wherein the PXRD pattern of Form AD is measured at room temperature.

In some embodiments, crystalline Form AD of Compound (I) is characterized by a powder X-ray diffraction (PXRD) pattern comprising three or more 2θ values in degrees (CuKα) selected from: 6.0±0.2, 6.9±0.2, 7.8±0.2, 10.3 ±0.2, and 13.8±0.2, wherein the PXRD pattern of Form AD is measured at room temperature.

In some embodiments, crystalline Form AD of Compound (I) is characterized by a powder X-ray diffraction (PXRD) pattern comprising four or more 2θ values in degrees (CuKα) selected from: 6.0±0.2, 6.9±0.2, 7.8±0.2, 10.3 ±0.2, and 13.8±0.2, wherein the PXRD pattern of Form AD is measured at room temperature.

In certain embodiments, crystalline Form AD of Compound (I) is characterized by a powder X-ray diffraction (PXRD) pattern comprising 2θ values in degrees (CuKα) at 6.0±0.2 and 6.9±0.2, wherein the PXRD pattern of Form AD is measured at room temperature. In further embodiments, the PXRD pattern further comprises one or more 2θ values in degrees selected from: 7.8±0.2, 10.3±0.2, and 13.8±0.2.

In certain embodiments, crystalline Form AD of Compound (I) is characterized by a powder X-ray diffraction (PXRD) pattern comprising 2θ values in degrees (CuKα) at 6.0±0.2, 6.9±0.2, and 10.3±0.2, wherein the PXRD pattern of Form AD is measured at room temperature.

In further embodiments, crystalline Form AD of Compound (I) is characterized by a powder X-ray diffraction (PXRD) pattern comprising 2θ values in degrees (CuKα) at 6.0±0.2, 6.9±0.2, 10.3±0.2, and 13.8±0.2, wherein the PXRD pattern of Form AD is measured at room temperature.

In certain embodiments, crystalline Form AD of Compound (I) is characterized by a powder X-ray diffraction (PXRD) pattern comprising 2θ values in degrees (CuKα) at 6.0±0.2, 6.9±0.2, and 7.8±0.2, wherein the PXRD pattern of Form AD is measured at room temperature.

In further embodiments, crystalline Form AD of Compound (I) is characterized by a powder X-ray diffraction (PXRD) pattern comprising 2θ values in degrees (CuKα) at 6.0±0.2, 6.9±0.2, 7.8±0.2, and 10.3±0.2, wherein the PXRD pattern of Form AD is measured at room temperature.

In further embodiments, crystalline Form AD of Compound (I) is characterized by a powder X-ray diffraction (PXRD) pattern comprising 2θ values in degrees (CuKα) at 6.0±0.2, 6.9±0.2, 7.8±0.2, 10.3±0.2, and 13.8±0.2, wherein the PXRD pattern of Form AD is measured at room temperature.

In certain embodiments, crystalline Form AD of Compound (I) is characterized by an observed powder X-ray diffraction pattern (CuKα, measured at room temperature) substantially as shown in FIG. 28.

In some embodiments, the Form AD of Compound (I) is substantially pure. For example, Form AD of Compound (I) may be present in a sample at a purity of greater than 90 weight %, greater than 95 weight %, or greater than 99 weight %, while the remaining material comprises other form(s) of the compound and/or reaction impurities and/or processing

impurities.

In certain embodiments, the crystalline form of Compound (I) consists essentially of Form AD. For such embodiments, crystalline Compound (I) may comprise at least about 90 weight %, preferably at least about 95 weight %, and more preferably at least about 99 weight %, of crystalline Form AD.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form AD.

In further embodiments, a pharmaceutical composition is provided comprising Form AD of Compound (I), and at least one pharmaceutically-acceptable carrier and/or diluent.

In certain embodiments, a pharmaceutical composition comprises substantially pure Form AD of Compound (I), and at least one pharmaceutically-acceptable carrier and/or diluent.

In certain embodiments, Form AD of Compound (I) is combined with at least one pharmaceutically acceptable carrier and/or diluent to provide at least one pharmaceutical composition.

In some embodiments, a pharmaceutical composition comprises Form AD of Compound (I) and other solid forms of Compound (I). Such other solid forms can be, for example, other crystalline forms and/or amorphous Compound (I).

**Form AE of Compound (I)**

In some embodiments, Compound (I) is provided as a crystalline material comprising Form AE. The crystalline Form AE of Compound (I) is a phosphate salt crystalline form.

**Table 20: Form AE of Compound (I)**

| PXRD 2θ values (CuKα) | | | | | | |
|---|---|---|---|---|---|---|
| 4.8±0.2$^0$ | 6.2±0.2$^0$ | 7.3±0.2$^0$ | 9.6±0.2$^0$ | 10.0±0.2$^0$ | 12.5±0.2$^0$ | 14.4±0.2$^0$ |
| 17.4±0.2$^0$ | 18.7±0.2$^0$ | 20.7±0.2$^0$ | 22.8±0.2$^0$ | 24.3±0.2$^0$ | 26.2±0.2$^0$ | - |

In some embodiments, crystalline Form AE of Compound (I) is characterized by a powder X-ray diffraction (PXRD) pattern comprising two or more 2θ values in degrees (CuKα) selected from: 4.8±0.2, 6.2±0.2, 7.3±0.2, 9.6±0.2, and 12.5±0.2, wherein the PXRD pattern of Form AE is measured at room temperature.

In some embodiments, crystalline Form AE of Compound (I) is characterized by a powder X-ray diffraction (PXRD) pattern comprising three or more 2θ values in degrees (CuKα) selected from: 4.8±0.2, 6.2±0.2, 7.3±0.2, 9.6 ±0.2, and 12.5±0.2, wherein the PXRD pattern of Form AE is measured at room temperature.

In some embodiments, crystalline Form AE of Compound (I) is characterized by a powder X-ray diffraction (PXRD) pattern comprising four or more 2θ values in degrees (CuKα) selected from: 4.8±0.2, 6.2±0.2, 7.3±0.2, 9.6±0.2, and 12.5±0.2, wherein the PXRD pattern of Form AE is measured at room temperature.

In certain embodiments, crystalline Form AE of Compound (I) is characterized by a powder X-ray diffraction (PXRD) pattern comprising 2θ values in degrees (CuKα) at 4.8±0.2 and 6.2±0.2, wherein the PXRD pattern of Form AE is measured at room temperature. In further embodiments, the PXRD pattern further comprises one or more 2θ values in degrees selected from: 7.3±0.2, 9.6±0.2, and 12.5±0.2.

In certain embodiments, crystalline Form AE of Compound (I) is characterized by a powder X-ray diffraction (PXRD) pattern comprising 2θ values in degrees (CuKα) at 4.8±0.2 and 9.6±0.2, wherein the PXRD pattern of Form AE is measured at room temperature. In further embodiments, the PXRD pattern further comprises one or more 2θ values in degrees selected from: 6.2±0.2, 7.3±0.2, and 12.5±0.2. For example, in some embodiments, crystalline Form AE of Compound (I) is characterized by a powder X-ray diffraction (PXRD) pattern comprising 2θ values in degrees (CuKα) at 4.8 ±0.2, 6.2±0.2, and 9.6±0.2, wherein the PXRD pattern of Form AE is measured at room temperature.

In certain embodiments, crystalline Form AE of Compound (I) is characterized by a powder X-ray diffraction (PXRD) pattern comprising 2θ values in degrees (CuKα) at 4.8±0.2, 6.2±0.2, and 7.3±0.2, wherein the PXRD pattern of Form AE is measured at room temperature.

In further embodiments, crystalline Form AE of Compound (I) is characterized by a powder X-ray diffraction (PXRD) pattern comprising 2θ values in degrees (CuKα) at 4.8±0.2, 6.2±0.2, 7.3±0.2, and 9.6±0.2, wherein the PXRD pattern of Form AE is measured at room temperature.

In further embodiments, crystalline Form AE of Compound (I) is characterized by a powder X-ray diffraction (PXRD) pattern comprising 2θ values in degrees (CuKα) at 4.8±0.2, 6.2±0.2, 7.3±0.2, 9.6±0.2, and 12.5±0.2, wherein the PXRD pattern of Form AE is measured at room temperature.

In certain embodiments, crystalline Form AE of Compound (I) is characterized by an observed powder X-ray diffraction pattern (CuKα, measured at room temperature) substantially as shown in FIG. 29.

In certain embodiments, crystalline Form AE of Compound (I) is characterized by an endotherm with a peak maximum in the approximate range of from 186 °C to 196 °C. In further embodiments, the endotherm maximum is at about 191 °C. For example, in some embodiments, crystalline Form AE of Compound (I) is characterized by a differential scanning calorimetry (DSC) thermogram comprising an endotherm with a maximum in the range of from about 186 °C to about 196 °C; in certain embodiments, the differential scanning calorimetry (DSC) thermogram comprises an endotherm with a maximum at about 191 °C. It should be understood that, in some cases, the endothermic event may not be detected.

In certain embodiments, crystalline Form AE of Compound (I) is characterized by (i) a powder X-ray diffraction (PXRD) pattern comprising 2θ values in degrees (CuKα) at 4.8±0.2 and 6.2±0.2, wherein the PXRD pattern of Form AE is measured at room temperature; and (ii) an endotherm with a peak maximum in the approximate range of from 186 °C to 196 °C. In further embodiments, the endotherm peak maximum is at about 191 °C.

In certain embodiments, crystalline Form AE of Compound (I) is characterized by (i) a powder X-ray diffraction (PXRD) pattern comprising 2θ values in degrees (CuKα) at 4.8±0.2 and 9.6±0.2, wherein the PXRD pattern of Form AE is measured at room temperature; and (ii) an endotherm with a peak maximum in the approximate range of from 186 °C to 196 °C. In further embodiments, the endotherm peak maximum is at about 191 °C.

In certain embodiments, crystalline Form AE of Compound (I) is characterized by (i) a powder X-ray diffraction (PXRD) pattern comprising 2θ values in degrees (CuKα) at 4.8±0.2, 6.2±0.2, and 7.3±0.2, wherein the PXRD pattern of Form AE is measured at room temperature; and (ii) an endotherm with a peak maximum in the approximate range of from 186 °C to 196 °C. In further embodiments, the endotherm peak maximum is at about 191 °C.

In some embodiments, crystalline Form AE of Compound (I) is characterized by a differential scanning calorimetry (DSC) thermogram substantially in accordance with the thermogram shown in FIG. 30.

In certain embodiments, crystalline Form AE of Compound (I) is characterized by (i) a powder X-ray diffraction (PXRD) pattern comprising two or more 2θ values in degrees (CuKα) selected from: 4.8±0.2, 6.2±0.2, 7.3±0.2, 9.6±0.2, and 12.5±0.2, wherein the PXRD pattern of Form AE is measured at room temperature; and (ii) a differential scanning calorimetry (DSC) thermogram substantially in accordance with the thermogram shown in FIG. 30.

In some embodiments, the Form AE of Compound (I) is substantially pure. For example, Form AE of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form AE. For such embodiments, crystalline Compound (I) may comprise at least about 90 weight %, preferably at least about 95 weight %, and more preferably at least about 99 weight %, of crystalline Form AE.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form AE.

In further embodiments, a pharmaceutical composition is provided comprising Form AE of Compound (I), and at least one pharmaceutically-acceptable carrier and/or diluent.

In certain embodiments, a pharmaceutical composition comprises substantially pure Form AE of Compound (I), and at least one pharmaceutically-acceptable carrier and/or diluent.

In certain embodiments, Form AE of Compound (I) is combined with at least one pharmaceutically acceptable carrier and/or diluent to provide at least one pharmaceutical composition.

In some embodiments, a pharmaceutical composition comprises Form AE of Compound (I) and other solid forms of Compound (I). Such other solid forms can be, for example, other crystalline forms and/or amorphous Compound (I).

**Form AF of Compound** (I)

In some embodiments, Compound (I) is provided as a crystalline material comprising Form AF. The crystalline Form AF of Compound (I) is a hydrochloride (HCl) salt solvated crystalline form, wherein the solvate comprises ethanol or further comprises ethanol and water.

**Table 21: Form AF** of Compound (I)

| PXRD 2θ values (CuKα) | | | | | | |
|---|---|---|---|---|---|---|
| 7.4±0.2$^0$ | 10.3±0.2$^0$ | 13.7±0.2$^0$ | 14.8±0.2$^0$ | 16.8±0.2$^0$ | 18.2±0.2$^0$ | 19.0±0.2$^0$ |
| 19.4±0.2$^0$ | 19.9±0.2$^0$ | 20.5±0.2$^0$ | 21.3±0.2$^0$ | 22.5±0.2$^0$ | 22.9±0.2$^0$ | 23.8±0.2$^0$ |

(continued)

| PXRD 2θ values (CuKα) | | | | | | |
|---|---|---|---|---|---|---|
| 24.6±0.2$^0$ | 25.2±0.2$^0$ | 25.8±0.2$^0$ | 26.2±0.2$^0$ | 27.5±0.2$^0$ | 29.6±0.2$^0$ | - |

In some embodiments, crystalline Form AF of Compound (I) is characterized by a powder X-ray diffraction (PXRD) pattern comprising two or more 2θ values in degrees (CuKα) selected from: 7.4±0.2, 13.7±0.2, 18.2±0.2, 20.5 ±0.2, 21.3±0.2, 22.5±0.2, 23.8±0.2, and 26.2±0.2, wherein the PXRD pattern of Form AF is measured at room temperature.

In some embodiments, crystalline Form AF of Compound (I) is characterized by a powder X-ray diffraction (PXRD) pattern comprising three or more 2θ values in degrees (CuKα) selected from: 7.4±0.2, 13.7±0.2, 18.2±0.2, 20.5 ±0.2, 21.3±0.2, 22.5±0.2, 23.8±0.2, and 26.2±0.2, wherein the PXRD pattern of Form AF is measured at room temperature.

In some embodiments, crystalline Form AF of Compound (I) is characterized by a powder X-ray diffraction (PXRD) pattern comprising four or more 2θ values in degrees (CuKα) selected from: 7.4±0.2, 13.7±0.2, 18.2±0.2, 20.5 ±0.2, 21.3±0.2, 22.5±0.2, 23.8±0.2, and 26.2±0.2, wherein the PXRD pattern of Form AF is measured at room temperature.

In some embodiments, crystalline Form AF of Compound (I) is characterized by a powder X-ray diffraction (PXRD) pattern comprising five or more 2θ values in degrees (CuKα) selected from: 7.4±0.2, 13.7±0.2, 18.2±0.2, 20.5 ±0.2, 21.3±0.2, 22.5±0.2, 23.8±0.2, and 26.2±0.2, wherein the PXRD pattern of Form AF is measured at room temperature.

In certain embodiments, crystalline Form AF of Compound (I) is characterized by a powder X-ray diffraction (PXRD) pattern comprising 2θ values in degrees (CuKα) at 7.4±0.2 and 13.7±0.2, wherein the PXRD pattern of Form AF is measured at room temperature. In further embodiments, the PXRD pattern further comprises one or more 2θ values in degrees selected from: 18.2±0.2, 20.5±0.2, 21.3±0.2, 22.5±0.2, 23.8±0.2, and 26.2±0.2.

In certain embodiments, crystalline Form AF of Compound (I) is characterized by a powder X-ray diffraction (PXRD) pattern comprising 2θ values in degrees (CuKα) at 7.4±0.2, 13.7±0.2, and 26.2±0.2, wherein the PXRD pattern of Form AF is measured at room temperature.

In certain embodiments, crystalline Form AF of Compound (I) is characterized by a powder X-ray diffraction (PXRD) pattern comprising 2θ values in degrees (CuKα) at 7.4±0.2, 13.7±0.2, and 18.2±0.2, wherein the PXRD pattern of Form AF is measured at room temperature.

In certain embodiments, crystalline Form AF of Compound (I) is characterized by a powder X-ray diffraction (PXRD) pattern comprising 2θ values in degrees (CuKα) at 7.4±0.2, 13.7±0.2, and 23.8±0.2, wherein the PXRD pattern of Form AF is measured at room temperature.

In further embodiments, crystalline Form AF of Compound (I) is characterized by a powder X-ray diffraction (PXRD) pattern comprising 2θ values in degrees (CuKα) at 7.4±0.2, 13.7±0.2, 18.2±0.2, and 23.8±0.2, wherein the PXRD pattern of Form AF is measured at room temperature.

In further embodiments, crystalline Form AF of Compound (I) is characterized by a powder X-ray diffraction (PXRD) pattern comprising 2θ values in degrees (CuKα) at 7.4±0.2, 13.7±0.2, 18.2±0.2, 23.8±0.2, and 26.2±0.2, wherein the PXRD pattern of Form AF is measured at room temperature.

In further embodiments, crystalline Form AF of Compound (I) is characterized by a powder X-ray diffraction (PXRD) pattern comprising 2θ values in degrees (CuKα) at 7.4±0.2, 13.7±0.2, 18.2±0.2, 20.5±0.2, 21.3±0.2, 22.5±0.2, 23.8±0.2, and 26.2±0.2, wherein the PXRD pattern of Form AF is measured at room temperature.

In certain embodiments, crystalline Form AF of Compound (I) is characterized by an observed powder X-ray diffraction pattern (CuKα, measured at room temperature) substantially as shown in FIG. 31.

In some embodiments, crystalline Form AF of Compound (I) is characterized by a broad endotherm in the approximate range of from 95 °C to 105 °C. In certain embodiments, the broad endotherm is at about 100 °C. In certain embodiments, crystalline Form AF of Compound (I) is characterized by an endotherm with a peak maximum in the approximate range of from 232 °C to 242 °C; in further embodiments, the endotherm maximum is at about 237 °C. For example, in some embodiments, crystalline Form AF of Compound (I) is characterized by a differential scanning calorimetry (DSC) thermogram comprising an endotherm with a maximum in the range of from about 232 °C to about 242 °C; in certain embodiments, the differential scanning calorimetry (DSC) thermogram comprises an endotherm with a maximum at about 237 °C. In further embodiments, the differential scanning calorimetry (DSC) thermogram also comprises a broad endotherm at about 100 °C. It should be understood that, in some cases, one or more of the endothermic events may not be detected.

In certain embodiments, crystalline Form AF of Compound (I) is characterized by (i) a powder X-ray diffraction (PXRD) pattern comprising 2θ values in degrees (CuKα) at 7.4±0.2 and 13.7±0.2, wherein the PXRD pattern of Form AF

is measured at room temperature; and (ii) an endotherm with a peak maximum in the approximate range of from 232 °C to 242 °C. In further embodiments, the endotherm peak maximum is at about 237 °C.

In certain embodiments, crystalline Form AF of Compound (I) is characterized by (i) a powder X-ray diffraction (PXRD) pattern comprising 2θ values in degrees (CuKα) at 7.4±0.2, 13.7±0.2, and 18.2±0.2, wherein the PXRD pattern of Form AF is measured at room temperature; and (ii) an endotherm with a peak maximum in the approximate range of from 232 °C to 242 °C. In further embodiments, the endotherm peak maximum is at about 237 °C.

In certain embodiments, crystalline Form AF of Compound (I) is characterized by (i) a powder X-ray diffraction (PXRD) pattern comprising 2θ values in degrees (CuKα) at 7.4±0.2, 13.7±0.2, and 23.8±0.2, wherein the PXRD pattern of Form AF is measured at room temperature; and (ii) an endotherm with a peak maximum in the approximate range of from 232 °C to 242 °C. In further embodiments, the endotherm peak maximum is at about 237 °C.

In the aforementioned embodiments describing an endotherm, crystalline Form AF of Compound (I) may be further characterized by a broad endotherm in the approximate range of from 95 °C to 105 °C; in further embodiments, this endotherm is at about 100 °C.

In some embodiments, crystalline Form AF of Compound (I) is characterized by a differential scanning calorimetry (DSC) thermogram substantially in accordance with the thermogram shown in FIG. 32.

In certain embodiments, crystalline Form AF of Compound (I) is characterized by (i) a powder X-ray diffraction (PXRD) pattern comprising two or more 2θ values in degrees (CuKα) selected from: 7.4±0.2, 13.7±0.2, 18.2±0.2, 20.5 ±0.2, 21.3±0.2, 22.5±0.2, 23.8±0.2, and 26.2±0.2, wherein the PXRD pattern of Form AF is measured at room temperature; and (ii) a differential scanning calorimetry (DSC) thermogram substantially in accordance with the thermogram shown in FIG. 32.

In some embodiments, crystalline Form AF of Compound (I) is characterized by a thermogravimetric analysis (TGA) thermogram showing weight loss of no more than about 10% upon being heated (e.g., from room temperature) to a temperature of about 100 °C. In certain embodiments, crystalline Form AF of Compound (I) is characterized by a thermogravimetric analysis (TGA) thermogram showing weight loss of about 10% upon being heated (e.g., from room temperature) to a temperature of about 105 °C.

In certain embodiments, crystalline Form AF of Compound (I) exhibits a thermogravimetric analysis (TGA) thermogram substantially as shown in FIG. 33.

In some embodiments, the Form AF of Compound (I) is substantially pure. For example, Form AF of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form AF. For such embodiments, crystalline Compound (I) may comprise at least about 90 weight %, preferably at least about 95 weight %, and more preferably at least about 99 weight %, of crystalline Form AF.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form AF.

In further embodiments, a pharmaceutical composition is provided comprising Form AF of Compound (I), and at least one pharmaceutically-acceptable carrier and/or diluent.

In certain embodiments, a pharmaceutical composition comprises substantially pure Form AF of Compound (I), and at least one pharmaceutically-acceptable carrier and/or diluent.

In certain embodiments, Form AF of Compound (I) is combined with at least one pharmaceutically acceptable carrier and/or diluent to provide at least one pharmaceutical composition.

In some embodiments, a pharmaceutical composition comprises Form AF of Compound (I) and other solid forms of Compound (I). Such other solid forms can be, for example, other crystalline forms and/or amorphous Compound (I).

**Form AG of Compound (I)**

In some embodiments, Compound (I) is provided as a crystalline material comprising Form AG. The crystalline Form AG of Compound (I) is a hydrochloride (HCl) salt solvated crystalline form, wherein the solvate comprises acetic acid.

**Table 22: Form AG of Compound (I)**

| PXRD 2θ values (CuKα) | | | | | | |
|---|---|---|---|---|---|---|
| 7.2±0.2$^0$ | 10.5±0.2$^0$ | 11.8±0.2$^0$ | 13.8±0.2$^0$ | 16.5±0.2$^0$ | 18.5±0.2$^0$ | 19.0±0.2$^0$ |
| 19.8±0.2$^0$ | 20.9±0.2$^0$ | 21.2±0.2$^0$ | 21.8±0.2$^0$ | 22.6±0.2$^0$ | 23.3±0.2$^0$ | 24.2±0.2$^0$ |

(continued)

| PXRD 2θ values (CuKα) | | | | | | |
|---|---|---|---|---|---|---|
| 24.8±0.2$^0$ | 25.3±0.2$^0$ | 27.3±0.2$^0$ | 28.0±0.2$^0$ | - | - | - |

In some embodiments, crystalline Form AG of Compound (I) is characterized by a powder X-ray diffraction (PXRD) pattern comprising two or more 2θ values in degrees (CuKα) selected from: 7.2±0.2, 13.8±0.2, 18.5±0.2, 21.8 ±0.2, 24.2±0.2, 25.3±0.2, and 26.8±0.2, wherein the PXRD pattern of Form AG is measured at room temperature.

In some embodiments, crystalline Form AG of Compound (I) is characterized by a powder X-ray diffraction (PXRD) pattern comprising three or more 2θ values in degrees (CuKα) selected from: 7.2±0.2, 13.8±0.2, 18.5±0.2, 21.8 ±0.2, 24.2±0.2, 25.3±0.2, and 26.8±0.2, wherein the PXRD pattern of Form AG is measured at room temperature.

In some embodiments, crystalline Form AG of Compound (I) is characterized by a powder X-ray diffraction (PXRD) pattern comprising four or more 2θ values in degrees (CuKα) selected from: 7.2±0.2, 13.8±0.2, 18.5±0.2, 21.8 ±0.2, 24.2±0.2, 25.3±0.2, and 26.8±0.2, wherein the PXRD pattern of Form AG is measured at room temperature.

In some embodiments, crystalline Form AG of Compound (I) is characterized by a powder X-ray diffraction (PXRD) pattern comprising five or more 2θ values in degrees (CuKα) selected from: 7.2±0.2, 13.8±0.2, 18.5±0.2, 21.8 ±0.2, 24.2±0.2, 25.3±0.2, and 26.8±0.2, wherein the PXRD pattern of Form AG is measured at room temperature.

In certain embodiments, crystalline Form AG of Compound (I) is characterized by a powder X-ray diffraction (PXRD) pattern comprising 2θ values in degrees (CuKα) at 7.2±0.2 and 13.8±0.2, wherein the PXRD pattern of Form AG is measured at room temperature. In further embodiments, the PXRD pattern further comprises one or more 2θ values in degrees selected from: 18.5±0.2, 21.8±0.2, 24.2±0.2, 25.3±0.2, and 26.8±0.2. For example, in certain embodiments, the PXRD pattern comprises 2θ values in degrees (CuKα) at 7.2±0.2, 13.8±0.2, and 25.3±0.2, wherein the PXRD pattern is measured at room temperature.

In certain embodiments, crystalline Form AG of Compound (I) is characterized by a powder X-ray diffraction (PXRD) pattern comprising 2θ values in degrees (CuKα) at 7.2±0.2, 13.8±0.2, and 18.5±0.2, wherein the PXRD pattern of Form AG is measured at room temperature.

In further embodiments, crystalline Form AG of Compound (I) is characterized by a powder X-ray diffraction (PXRD) pattern comprising 2θ values in degrees (CuKα) at 7.2±0.2, 13.8±0.2, 18.5±0.2, and 21.8±0.2, wherein the PXRD pattern of Form AG is measured at room temperature.

In further embodiments, crystalline Form AG of Compound (I) is characterized by a powder X-ray diffraction (PXRD) pattern comprising 2θ values in degrees (CuKα) at 7.2±0.2, 13.8±0.2, 18.5±0.2, 21.8±0.2, and 24.2±0.2, wherein the PXRD pattern of Form AG is measured at room temperature.

In further embodiments, crystalline Form AG of Compound (I) is characterized by a powder X-ray diffraction (PXRD) pattern comprising 2θ values in degrees (CuKα) at 7.2±0.2, 13.8±0.2, 18.5±0.2, 21.8±0.2, 24.2±0.2, and 25.3 ±0.2, wherein the PXRD pattern of Form AG is measured at room temperature.

In further embodiments, crystalline Form AG of Compound (I) is characterized by a powder X-ray diffraction (PXRD) pattern comprising 2θ values in degrees (CuKα) at 7.2±0.2, 13.8±0.2, 18.5±0.2, 21.8±0.2, 24.2±0.2, 25.3±0.2, and 26.8±0.2, wherein the PXRD pattern of Form AG is measured at room temperature.

In certain embodiments, crystalline Form AG of Compound (I) is characterized by an observed powder X-ray diffraction pattern (CuKα, measured at room temperature) substantially as shown in FIG. 34.

In some embodiments, crystalline Form AG of Compound (I) is characterized by an endotherm with a peak maximum in the approximate range of from 146 °C to 156 °C. In certain embodiments, the endotherm maximum is at about 151 °C. For example, in some embodiments, crystalline Form AG of Compound (I) is characterized by a differential scanning calorimetry (DSC) thermogram comprising an endotherm with a maximum in the range of from about 146 °C to about 156 °C; in certain embodiments, the differential scanning calorimetry (DSC) thermogram comprises an endotherm with a maximum at about 151 °C. In certain embodiments, crystalline Form AG of Compound (I) is characterized by an endotherm with a peak maximum in the approximate range of from 231 °C to 241 °C. In certain embodiments, the endotherm maximum is at about 236 °C. For example, in some embodiments, crystalline Form AG of Compound (I) is characterized by a differential scanning calorimetry (DSC) thermogram comprising an endotherm with a maximum in the range of from about 231 °C to about 241 °C; in certain embodiments, the differential scanning calorimetry (DSC) thermogram comprises an endotherm with a maximum at about 236 °C. In certain embodiments, crystalline Form AG of Compound (I) is characterized by a differential scanning calorimetry (DSC) thermogram comprising both of the endotherms described above. It should be also understood that, in some cases, one or more of the endothermic events may not be detected.

In certain embodiments, crystalline Form AG of Compound (I) is characterized by (i) a powder X-ray diffraction (PXRD) pattern comprising 2θ values in degrees (CuKα) at 7.2±0.2 and 13.8±0.2, wherein the PXRD pattern of Form AG is measured at room temperature; and (ii) an endotherm with a peak maximum in the approximate range of from 231 °C to

241 °C. In further embodiments, the endotherm peak maximum is at about 236 °C. In some embodiments, crystalline Form AG of Compound (I) is further characterized by an endotherm with a peak maximum in the approximate range of from 146 °C to 156 °C; in further embodiments, this endotherm peak maximum is at about 151 °C.

In certain embodiments, crystalline Form AG of Compound (I) is characterized by (i) a powder X-ray diffraction (PXRD) pattern comprising 2$\theta$ values in degrees (CuK$\alpha$) at 7.2$\pm$0.2, 13.8$\pm$0.2, and 18.5$\pm$0.2, wherein the PXRD pattern of Form AG is measured at room temperature; and (ii) one or more of the following endotherms: an endotherm with a peak maximum in the approximate range of from 146 °C to 156 °C, and an endotherm with a peak maximum in the approximate range of from 231 °C to 241 °C.

In some embodiments, crystalline Form AG of Compound (I) is characterized by a differential scanning calorimetry (DSC) thermogram substantially in accordance with the thermogram shown in FIG. 35.

In certain embodiments, crystalline Form AG of Compound (I) is characterized by (i) a powder X-ray diffraction (PXRD) pattern comprising two or more 2$\theta$ values in degrees (CuK$\alpha$) selected from: 7.2$\pm$0.2, 13.8$\pm$0.2, 18.5$\pm$0.2, 21.8 $\pm$0.2, 24.2$\pm$0.2, 25.3$\pm$0.2, and 26.8$\pm$0.2, wherein the PXRD pattern of Form AG is measured at room temperature; and (ii) a differential scanning calorimetry (DSC) thermogram substantially in accordance with the thermogram shown in FIG. 35.

In some embodiments, crystalline Form AG of Compound (I) is characterized by a thermogravimetric analysis (TGA) thermogram showing weight loss of no more than about 10% upon being heated (e.g., from room temperature) to a temperature of about 100 °C. In certain embodiments, crystalline Form AG of Compound (I) is characterized by a thermogravimetric analysis (TGA) thermogram showing weight loss of about 11% upon being heated (e.g., from room temperature) to a temperature of about 160 °C.

In certain embodiments, crystalline Form AG of Compound (I) exhibits a thermogravimetric analysis (TGA) thermogram substantially as shown in FIG. 36.

In some embodiments, the Form AG of Compound (I) is substantially pure. For example, Form AG of Compound (I) may be present in a sample at a purity of greater than 90 weight %, greater than 95 weight %, or greater than 99 weight %, while the remaining material comprises other form(s) of the compound and/or reaction impurities and/or processing impurities.

In certain embodiments, the crystalline form of Compound (I) consists essentially of Form AG. For such embodiments, crystalline Compound (I) may comprise at least about 90 weight %, preferably at least about 95 weight %, and more preferably at least about 99 weight %, of crystalline Form AG.

Certain embodiments also provide a composition comprising 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, wherein at least 95 weight %, preferably at least 97 weight %, and more preferably at least 99 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in crystalline Form AG.

In further embodiments, a pharmaceutical composition is provided comprising Form AG of Compound (I), and at least one pharmaceutically-acceptable carrier and/or diluent.

In certain embodiments, a pharmaceutical composition comprises substantially pure Form AG of Compound (I), and at least one pharmaceutically-acceptable carrier and/or diluent.

In certain embodiments, Form AG of Compound (I) is combined with at least one pharmaceutically acceptable carrier and/or diluent to provide at least one pharmaceutical composition.

In some embodiments, a pharmaceutical composition comprises Form AG of Compound (I) and other solid forms of Compound (I). Such other solid forms can be, for example, other crystalline forms and/or amorphous Compound (I).

Crystalline forms may be prepared by a variety of methods, including for example, crystallization or recrystallization from a suitable solvent, sublimation, growth from a melt, solid state transformation from another phase, crystallization from a supercritical fluid, and jet spraying. Techniques for crystallization or recrystallization of crystalline forms from a solvent mixture include, for example, evaporation of the solvent, decreasing the temperature of the solvent mixture, crystal seeding a supersaturated solvent mixture of the molecule and/or salt, freeze drying the solvent mixture, and addition of antisolvents (countersolvents) to the solvent mixture. High throughput crystallization techniques may be employed to prepare crystalline forms including polymorphs.

Crystals of drugs, including polymorphs, methods of preparation, and characterization of drug crystals are discussed in Solid-State Chemistry of Drugs, S.R. Byrn, R.R. Pfeiffer, and J.G. Stowell, 2nd Edition, SSCI, West Lafayette, Indiana (1999).

For crystallization techniques that employ solvent, the choice of solvent or solvents typically depends on one or more factors, such as solubility of the compound, crystallization technique, and vapor pressure of the solvent. Combinations of solvents may be employed, for example, the compound may be solubilized into a first solvent to afford a solution, followed by the addition of an antisolvent to decrease the solubility of the compound in the solution and to afford the formation of crystals. An antisolvent is a solvent in which the compound has low solubility.

In one method to prepare crystals, a compound is suspended and/or stirred in a suitable solvent to afford a slurry,

which may be heated to promote dissolution. The term "slurry", as used herein, means a saturated solution of the compound, which may also contain an additional amount of the compound to afford a heterogeneous mixture of the compound and a solvent at a given temperature.

Seed crystals may be added to any crystallization mixture to promote crystallization. Seeding may be employed to control growth of a particular polymorph or to control the particle size distribution of the crystalline product. Accordingly, calculation of the amount of seeds needed depends on the size of the seed available and the desired size of an average product particle as described, for example, in "Programmed Cooling of Batch Crystallizers," J.W. Mullin and J. Nyvlt, Chemical Engineering Science, 1971,26, 369-377. In general, seeds of small size are needed to effectively control the growth of crystals in the batch. Seeds of small size may be generated by sieving, milling, or micronizing of large crystals, or by micro-crystallization of solutions. Care should be taken that milling or micronizing of crystals does not result in any change in crystallinity from the desired crystal form (i.e., a change to amorphous or to another polymorph).

A cooled crystallization mixture may be filtered under vacuum, and the isolated solids may be washed with a suitable solvent, such as cold recrystallization solvent, and dried under a nitrogen purge to afford the desired crystalline form. The isolated solids may be analyzed by one or more suitable spectroscopic or analytical techniques, such as solid state nuclear magnetic resonance, differential scanning calorimetry, X-ray powder diffraction, or the like, to assess formation of the preferred crystalline form of the product. The resulting crystalline form is typically produced in an amount of greater than about 70 weight % isolated yield, preferably greater than 90 weight % isolated yield, based on the weight of the compound originally employed in the crystallization procedure. The product may be co-milled or passed through a mesh screen to delump the product, if necessary.

Crystalline forms may be prepared directly from the reaction medium of the final process for preparing Compound (I). Such preparation may be achieved, for example, by employing in the final process step a solvent or a mixture of solvents from which Compound (I) may be crystallized. Alternatively, crystalline forms may be obtained by distillation or solvent addition techniques. Suitable solvents for this purpose include, for example, the aforementioned nonpolar solvents and polar solvents, including protic polar solvents such as alcohols, and aprotic polar solvents such as ketones.

The presence of more than one polymorph in a sample may be determined by techniques such as powder X-ray diffraction (PXRD) or solid state nuclear magnetic resonance spectroscopy (ssNMR). For example, the presence of extra peaks in the comparison of an experimentally measured PXRD pattern with a simulated PXRD pattern may indicate the presence of more than one polymorph in the sample. The simulated PXRD may be calculated from single crystal X-ray data. See Smith, D.K., "A FORTRAN Program for Calculating X-Ray Powder Diffraction Patterns," Lawrence Radiation Laboratory, Livermore, California, UCRL-7196 (April 1963).

The forms of Compound (I) described herein may be characterized using various techniques, the operation of which are well known to those of ordinary skill in the art. The forms may be characterized and distinguished using single crystal X-ray diffraction, which is based on unit cell measurements of a single crystal at a fixed analytical temperature. A detailed description of unit cells is provided in Stout & Jensen, X-Ray Structure Determination: A Practical Guide, Macmillan Co., New York (1968), Chapter 3. Alternatively, the unique arrangement of atoms in spatial relation within the crystalline lattice may be characterized according to the observed fractional atomic coordinates. Another means of characterizing the crystalline structure is by powder X-ray diffraction analysis in which the diffraction profile is compared to a simulated profile representing pure powder material, both run at the same analytical temperature, and measurements for the subject form are characterized as a series of 2$\theta$ values (e.g., two, three, four or more).

Other methods of characterizing the form may be used. Such methods include solid state nuclear magnetic resonance (ssNMR), differential scanning calorimetry (DSC), thermography, and gross examination of the crystalline or amorphous morphology. Two or more of these parameters may also be used in combination to characterize the subject form.

## UTILITY

The crystalline forms of Compound (I) described herein can be used to isolate Compound (I) from other components at the completion of the synthesis process; and/or to purify Compound (I) by one or a series of crystallization steps. The isolation and the purification steps can be combined or practiced as separate process steps. Each of the crystalline forms described herein can also be used to make other solid forms of Compound (I), including, for example, amorphous Compound (I). In further embodiments, amorphous Compound (I) is then used to make a dosage form (e.g., a dosage form comprising a solid dispersion of amorphous Compound (I)) for clinical use.

Each of the crystalline forms of Compound (I) described herein may be used alone or in combination with other forms of Compound (I) (including the other crystalline forms described herein), and/or formulated with one or more excipients or other active pharmaceutical ingredients to make pharmaceutical compositions.

## EXAMPLES

Examples related to Crystalline Form R of Compound (I) are working examples of the claimed invention. Other examples are reference examples. All temperatures are in degrees Celsius (°C) unless otherwise indicated. These examples are illustrative.

For ease of reference, the following abbreviations may be used herein.

### Abbreviations

| | |
|---|---|
| ACN or MeCN | acetonitrile |
| AP | area percent |
| DBU | 1,8-diazabicyclo[5.4.0]undec-7-ene |
| DIPEA | N,N-diisopropylethylamine (Hunig's base) |
| EDC HCl | 1-(dimethylaminopropyl)-3-ethylcarbodiimide hydrochloride |
| equiv. | molar equivalents |
| Et | ethyl |
| h | hour(s) |
| HOBt | 1-hydroxy benzotriazole |
| IPA | isopropyl alcohol |
| IPAc | isopropyl acetate |
| min | minute(s) |
| Me | methyl |
| MTBE | methyl *tert*-butyl ether |
| NMP | *n*-methylpyrrolidone |
| Pd/C | palladium on carbon |
| RH | relative humidity |
| THF | tetrahydrofuran |
| TsCl | p-toluenesulfonyl chloride |

### Example 1: Preparation of Crystalline Form L of Compound (I)

The addition of an equimolar amount of gentisic acid (32.9 mg; 0.2 mmole) to Compound (I) (84.4 mg; 0.2 mmole) in acetonitrile (1.0 mL) resulted in a suspension, which was slurried at 75 °C for 1 day. After 1 day, the resulting suspension was composed of particles with birefringence. The suspension was hot filtered with positive pressure yielding solids. The solids comprised Form L.

### Example 2: Preparation of Crystalline Form M of Compound (I)

A suspension of Compound (I) (84.4 mg; 0.2 mmole) in acetonitrile (1.0 mL) was placed at 75 °C. To this suspension, 2 mole equivalents of HBr (47 μL) were added, resulting in a solution. The solution then quickly precipitated solids, forming a suspension. The suspension was hot filtered with positive pressure yielding solids. The solids comprised Form M.

### Example 3: Preparation of Crystalline Form N of Compound (I)

Compound (I) (79.6 mg; 0.19 mmole) was suspended in water and an approximately equimolar amount of nitric acid (15 μL; 0.25 mmole) was added, producing a solution. Evaporation with a nitrogen purge resulted in solids. The solids comprised Form N.

**Example 4: Preparation of Crystalline Form O of Compound (I)**

p-Toluenesulfonic acid (38.8 mg, 0.20 mmole) was dissolved in ethyl acetate (1 mL) and added to approximately an equimolar amount of Compound (I) (74.1 mg, 0.17 mmole) producing a suspension. After slurrying overnight a gel had formed that contained small equants with birefringence. The clear supernatant was decanted and left to sit at room temperature for 10 days, after which solids with birefringence had precipitated. The solids comprised Form O.

**Example 5: Preparation of Crystalline Form P of Compound (I)**

A suspension of Compound (I) (84.4 mg; 0.2 mmole) in acetonitrile (1.0 mL) was placed at 75 °C. To this suspension, 2 mole equivalents of HBr (47 μL) were added, resulting in a solution. The solution then quickly precipitated solids, forming a suspension. The suspension was hot filtered with positive pressure. The filtrate was fast cooled to room temperature, resulting in solids. The solids comprised Form P.

**Example 6: Preparation of Crystalline Form Q of Compound (I)**

An aliquot of ethyl acetate (1 mL) was saturated with malonic acid at 60 °C. The saturated solution was hot filtered into a sample of Compound (I) (70.9 mg; 0.17 mmole). The resulting suspension was slurried at 60 °C for 8 days, resulting in a suspension composed of particles with birefringence. The suspension was hot filtered with positive pressure yielding solids. The solids comprised Form Q.

**Example 7: Preparation of Crystalline Form R of Compound (I)**

1,2-ethanedisulfonic acid dihydrate (19.7 mg, 0.085 mmole) was dissolved in acetone at 50 °C. This solution was added to 2 mole equivalents of Compound (I) (67.7 mg, 0.16 mmole) and the resulting suspension was slurried at 50 °C for 90 minutes, during which the suspension was composed of particles with birefringence. The suspension was hot filtered with positive pressure yielding solids. The solids comprised Form R.

**Example 8: Preparation of Crystalline Form S of Compound (I)**

In one experiment, Compound (I) (88.7 mg, 0.21 mmole) was suspended in chloroform (0.75 mL) and 2 mole equivalents of 2-hydroxyethanesulfonic acid (35 μL, 0.42 mmole) were added and the resulting solution was stirred at room temperature for a week before evaporation. The resulting gel was vacuum dried and then suspended in acetone, resulting in a suspension composed of small particles with birefringence. The suspension was slurried for 4 days before filtration to isolate solids. The solids comprised Form S.

Form S was also isolated from acetonitrile and an equimolar amount of 2-hydroxyethanesulfonic acid. A suspension of Compound (I) (71.3 mg, 0.17 mmole) in acetonitrile (1 mL) was slurried at 70 °C for 30 minutes before an equimolar amount of 2-hydroxyethanesulfonic acid (16.4 μL, 0.17 mmole) was added, producing a suspension. This suspension was slurried for 1 week at 70 °C, forming a solution that was cooled to room temperature and evaporated. The resulting solids comprised Form S.

**Example 9: Preparation of Crystalline Form T of Compound (I)**

A suspension of maleic acid (43.5 mg, 0.36 mmole) in 1 mL of chloroform: ethyl acetate 1:1 (v/v) was added to 0.5 molar equivalents of Compound (I) (73.6 mg; 0.17 mmole), producing a suspension. Slurrying of the sample for 2 weeks at room temperature resulted in a suspension composed of particles with birefringence. The suspension was filtered with positive pressure yielding solids. The solids comprised Form T.

**Example 10: Preparation of Crystalline Form U of Compound (I)**

Compound (I) (73.7 mg; 0.17 mmole) was suspended in p-dioxane (1 mL) at 75 °C. Approximately two mole equivalents of naphthalene-1,5-disulfonic acid (128.5 mg, 0.45 mmole) were added to the suspension at 75 °C, resulting in a gel with birefringence particles. The sample was slurried at ambient temperature for 6 days, resulting in a gel with no birefringent particles. To this gel toluene (2x1 mL) was added, which resulted in a suspension. This suspension was slurried for 2 days at room temperature before being filtered with positive pressure to yield solids. The solids comprised Form U.

**Example 11: Preparation of Crystalline Form V of Compound (I)**

Compound (I) (83.5 mg, 0.20 mmole) was suspended in water (1 mL) and placed at 80 °C, where 2 mole equivalents of nitric acid (25 μL, 0.40 mmole) were added to the suspension, resulting in a solution with scant solids. The reaction was hot filtered, cooled to room temperature, and evaporated resulting in a gel. Diisopropyl ether (1 mL) was added, producing a suspension composed of small particles with birefringence. This suspension was slurried at room temperature for 2 days prior to filtration with positive pressure, yielding solids. The solids comprised Form V.

**Example 12: Preparation of Crystalline Form W of Compound (I)**

Compound (I) (70.1 mg, 0.16 mmole) and approximately an equimolar amount of benzenesulfonic acid (32.0 mg; 0.20 mmole) were combined and an aliquot of ethyl acetate (1 mL) was added resulting in a suspension. The suspension was slurried at 60 °C for 1 week, resulting in a suspension composed of particles with birefringence. The suspension was filtered with positive pressure, resulting in solids. The solids comprised Form W.

**Example 13: Preparation of Crystalline Form X of Compound (I)**

Compound (I) (62.9 mg, 0.15 mmole) and approximately an equimolar amount of naphthalene-1,5-disulfonic acid (56.1 mg, 0.19 mmole) were suspended in acetone (1 mL) and slurried at 50 °C for one week, resulting in a suspension composed of particles with birefringence. The suspension was filtered with positive pressure to yield solids. The solids comprised Form X.

**Example 14: Preparation of Crystalline Form Y of Compound (I)**

Compound (I) (67.1 mg, 0.16 mmole) was suspended in 2-methyltetrahydrofuran (1 mL) and a 1.5 molar amount of ethanesulfonic acid (20.6 μL) was added. Solids remained after addition of the acid, and the sample was placed at 70 °C and stirred for 1 week, producing a gel. Methyl tert-butyl ether (1 mL) was added and the sample was triturated for 2 days at room temperature, resulting in a suspension. The suspension was filtered with positive pressure to yield solids. The solids comprised Form Y.

**Example 15: Preparation of Crystalline Form Z of Compound (I)**

360 mg of Compound (I) were dissolved in 24 mL of tetrahydrofuran: water 95:5 (v/v) at ambient temperature. 47 μL of 96% sulfuric acid were added. The solids were isolated by quick evaporation. 90 mg of these isolated solids were suspended in 1 mL of ethanol: water 1:2 (v/v) at 60 °C and agitated overnight. The solids suspended in the slurry comprised Form Z.

**Example 16: Preparation of Crystalline Form AA of Compound (I)**

360 mg of Compound (I) were dissolved in 24 mL of tetrahydrofuran: water 95:5 (v/v) at ambient temperature. 47 μL of 96% sulfuric acid were added. The solids were isolated by quick evaporation. 90 mg of these isolated solids were suspended in 1 mL of butyl acetate at 60 °C and agitated overnight. The suspension was filtered and the filtered solids were dried at 50 °C in a vacuum oven overnight. The dried solids comprised Form AA.

**Example 17: Preparation of Crystalline Form AB of Compound (I)**

360 mg of Compound (I) were dissolved in 24 mL of tetrahydrofuran: water 95:5 (v/v) at ambient temperature. 47 μL of 96% sulfuric acid were added. The solids were isolated by quick evaporation. 90 mg of these isolated solids were suspended in 1 mL of IPAc at 60 °C and agitated overnight. The suspension was filtered and the filtered solids were dried at 50 °C in a vacuum oven overnight. The dried solids comprised Form AB.

**Example 18: Preparation of Crystalline Form AC of Compound (I)**

360 mg of Compound (I) were dissolved in 24 mL of tetrahydrofuran: water 95:5 (v/v) at ambient temperature. 142 μL of 37% hydrochloric acid were added. The solids were isolated by quick evaporation. 90 mg of these isolated solids were stirred in 1 mL of ethanol: water 1:2 (v/v) at 60 °C and agitated overnight. The solids suspended in the slurry comprised Form AC.

**Example 19: Preparation of Crystalline Form AD of Compound (I)**

360 mg of Compound (I) were dissolved in 24 mL of tetrahydrofuran: water 95:5 (v/v) at ambient temperature. 55 μL of methanesulfonic acid were added. The solids were isolated by quick evaporation. 90 mg of these isolated solids were suspended in 1 mL of IPAc at 60 °C and agitated overnight. The suspension was filtered and the filtered solids were dried at 50 °C in a vacuum oven overnight. The dried solids comprised Form AD.

**Example 20: Preparation of Crystalline Form AE of Compound (I)**

360 mg of Compound (I) were dissolved in 24 mL of tetrahydrofuran: water 95:5 (v/v) at ambient temperature. 58 μL of 85% phosphoric acid were added. The solids were isolated by quick evaporation. 90 mg of these isolated solids were suspended in 1 mL of isopropyl acetate at 60 °C and agitated overnight. The suspension was filtered and the filtered solids were dried at 50 °C in a vacuum oven overnight. The dried solids comprised Form AE.

In another preparation, 90 mg of the isolated solids obtained by quick evaporation described in the first preparation above were suspended in 1 mL of butyl acetate at 60 °C and agitated overnight. The suspension was filtered and the filtered solids were dried at 50 °C in a vacuum oven overnight. The dried solids comprised Form AE.

**Example 21: Preparation of Crystalline Form AF of Compound (I)**

Compound (I) was dissolved in ethanol:water 90:10 (v:v) at 19 mg/mL concentration at 70 °C. The solution was hot filtered into a new vial and cooled to 2-8 °C. The precipitated solids comprised Form AF.

Form AF was also prepared by several other methods. In one preparation, Compound (I) (Form B) was suspended in ethanol:water 90:10 (v:v) at 2-8 °C for 14 days. The resulting solids comprised Form AF. In another preparation, Compound (I) (Form B) was suspended in ethanol:water 80:20 (v:v) at 2-8 °C and at room temperature for 14 days. The resulting solids comprised Form AF. In yet another preparation, Compound (I) (Form B) was suspended in ethanol:water 64:36 (v:v) at room temperature for 14 days. The resulting solids comprised Form AF.

**Example 22: Preparation of Crystalline Form AG of Compound (I)**

Compound (I) (Form B) was suspended in acetic acid at room temperature for 14 days. The resulting solids comprised Form AG.

In another preparation, Compound (I) was dissolved in acetic acid at 42 mg/mL concentration at 70 °C. The solution was hot filtered and the filtered solution was cooled to room temperature, at a rate of 20 °C/hour. The resulting clear solution was further cooled to 2-8 °C and then to -15 to -25 °C. The solution was equilibrated at room temperature and ethyl acetate was added, to reach a final acetic acid:ethyl acetate ratio of 1:3 (v:v). Solids were observed within 10 minutes after the addition of ethyl acetate. The solids comprised Form AG.

<u>Synthesis of 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide</u>

**Step 1: Preparation of Compound 2**

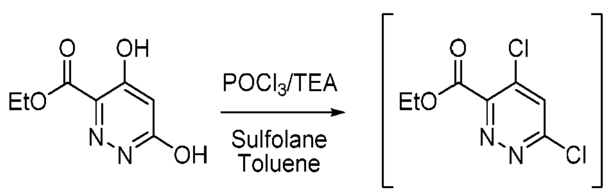


**1** **2**

To a glass lined reactor were charged toluene (0.26 kg), sulfolane (3.4 kg), Compound 1 (1.0 kg) and $POCl_3$ (2.7 kg). The crude was cooled to 0 °C. Triethylamine (0.89 kg) was charged, and the resulting crude mixture was heated to 65 °C and aged till the reaction reached completion. The reaction mass was cooled to 5 °C.

In a separate reactor, water (7.5 kg) was charged and cooled to 5 °C. The reaction mass was added slowly to the water solution, maintaining the internal temperature below 5 °C. Additional water (0.5 kg) was used to rinse the reactor and

aid the transfer. The resulting mixture was agitated at 5 °C for 3 hours, then extracted with MTBE three times (3 x 4.5 kg). The combined organic layers were washed sequentially with aqueous pH 7 buffer solution (5.0 L/kg, 15 wt% $KH_2PO_4/K_2HPO_4$) and water (2.5 kg). The crude was distilled under vacuum until total volume became approximately 3 L/kg. ACN (2 x 6.3 kg) was added followed by additional distillations back to ~3 L/kg. The crude was cooled to 20 °C to afford Compound 2 as a 30-36 wt% solution in 90-95% yield.

**Step 2: Preparation of Compound 3**

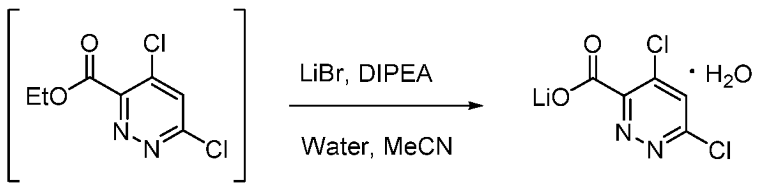


2 3

ACN (2.7 kg), lithium bromide (1.18 kg) and water (0.65 kg) were charged to a glass-lined reactor at 25 °C. Compound 2 crude solution prepared above (limiting reagent) was added, followed by DIPEA (1.82 kg). The resulting slurry was agitated at 25 °C until the reaction reached completion. The product was isolated by filtration. The crude solid was washed with ACN (1.6 kg). The cake was dried under vacuum at 45 °C. Compound 3 was isolated in 98 AP and 83% yield.

**Step 3: Preparation of Compound 8**

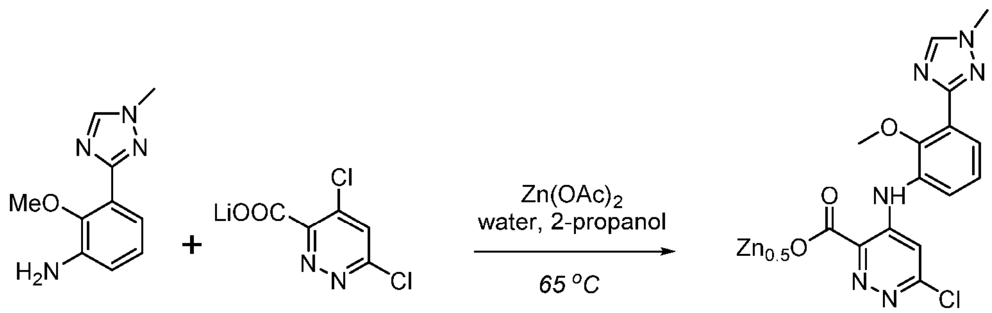


7 3 8

Water (6.0 kg, 6.0 L/kg) and Compound 7 (1.0 kg) were charged to a glass-lined reactor at 25 °C. Zinc acetate dehydrate (1.08 kg, 1.0 equiv) was added, followed by Compound 3 (1.28 kg, 1.20 equiv). The reactor line was rinsed with 2-propanol (0.79 kg, 1.0 L/kg) and water (1.50 kg, 1.50 L/kg). The resulting homogeneous solution was heated to 65 °C and aged until the reaction reached completion. Water (7.0 kg, 7.0 L/kg) was added, and the crude mixture was cooled to 20 °C and aged for 30 min. The product was isolated by filtration. The crude solid was washed sequentially with water (6.0 kg, 6.0 L/kg), water (6.0 kg, 6.0 L/kg), THF (5.3 kg, 6.0 L/kg) and THF (5.3 kg, 6.0 L/kg). The cake was dried under vacuum at 70 °C. Compound 8 was isolated in 98 AP and 94% yield.

**Step 4: Preparation of Compound 9**

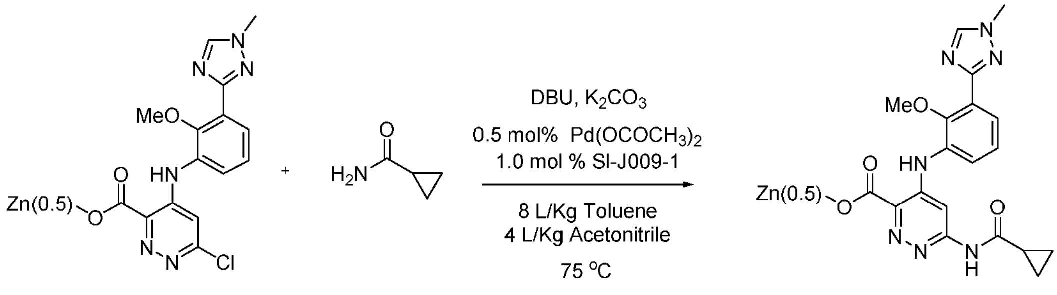


8 10 9

A separate glass-lined reactor was flushed with nitrogen. Toluene (0.87 kg, 1.0 L/kg) and MeCN (0.79 kg, 1.0 L/kg) were charged, followed by (2R)-1-[(1R)-1-[bis(1,1-dimethylethyl) phosphino] ethyl]-2-(dicyclohenxyphosphino) ferrocene (Josiphos SL-009-01) (14.1 g, 1.0 mol%) and palladium acetate (2.9 g, 0.5 mol%). The reactor line was rinsed with toluene (0.43 kg, 0.5 L/kg). The resulting pre-formed catalyst solution was kept under nitrogen until further usage.

At 20 °C, toluene (3.46 Kg, 4.0 L/kg) and ACN (1.57 kg, 2.0 L/kg) were charged to a glass-lined reactor flushed with nitrogen. Compound 8 (1.00 kg) was added, followed by DBU (0.39 kg, 1.00 equiv). The reactor line was rinsed with toluene (0.43 kg, 0.5 L/kg). Compound 10 (0.54 kg, 2.5 equiv) and $K_2CO_3$ (325 mesh grade, 0.70 kg, 2.0 equiv) were added to the reaction mixture, followed by toluene (1.30 kg, 1.5 L/kg) and ACN (0.79 kg, 1.0 L/kg). The pre-formed catalyst solution was transferred into the reaction mixture, which was then heated to 75 °C and agitated until the reaction reached completion.

The reaction crude was cooled to 20 °C. Aqueous acetic acid (50 Volume %, 4.0 kg, 4.0 L/kg) was charged slowly over the course of 1 h. Glacial acetic acid (10.5 kg, 10.0 L/kg) was then added. The resulting homogeneous solution was washed twice with heptane (2 × 3.42 kg, 2 × 5.0 L/kg). The bottom aqueous layer was collected and transferred to a clean reactor. Water (5.0 kg, 5.0 L/kg) was added, followed by Compound 9 seeds (0.01 kg, 1.0 wt%). The slurry was aged for 2 h at 20 °C. Additional water (2.0 kg, 2.0 L/kg) was added, and the slurry was further aged for 6 h. The product was isolated by filtration. The crude cake was washed with aqueous ACN (50 Volume %, 4.5 kg, 5.0 L/kg) followed by ACN (3.9 kg, 5.0 L/kg). The cake was dried under vacuum at 65 °C. Compound 9 was isolated in 98.5 AP and 84% yield.

**Step 5: Preparation of the Compound (I)**

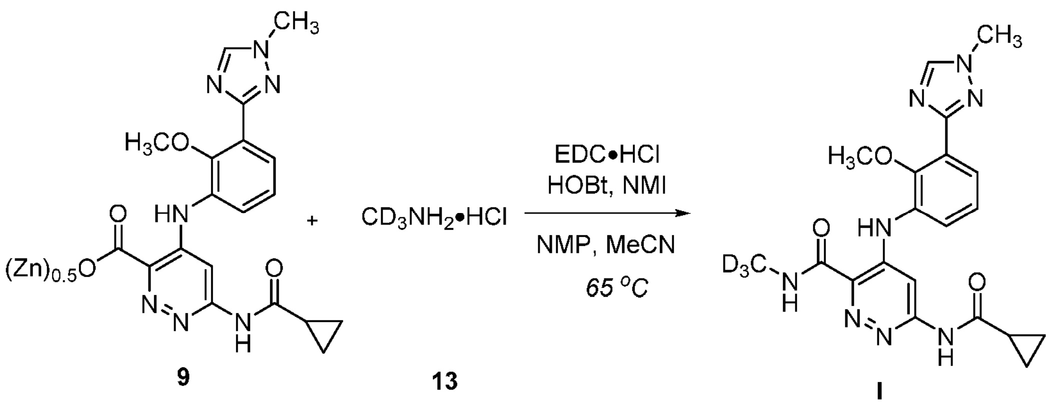


9 13 I

NMP (2.06 kg, 2.0 L/kg) and ACN (0.78 kg, 1.0 L/kg) were charged to a glass-lined reactor and agitated at 20 °C. N-Methylimidazole (0.13 kg, 0.7 equiv.), Compound 13 (0.17 kg, 1.2 equiv.) and Compound 9 (1.00 kg) were charged to the reaction mixture. The mixture was heated to 65 °C and aged until homogeneous. HOBt 20% wet (0.17 kg, 0.5 eq), followed by EDC HCl (0.54 kg, 1.4 eq) were then charged to the reaction mixture. The reactor was rinsed with ACN (0.78 kg, 1.0 L/kg), then the resulting mixture was aged at 65 °C until the reaction reached completion. The reaction was quenched by charging water (1.0 kg, 1 L/kg), then diluted with ACN (3.0 kg, 3 L/kg). The reaction mixture was aged at 65 °C for 1 h, before cooling to 0 °C, and aged for an additional 12 h at 0 °C. The product was isolated by filtration. The wet cake was washed with 2:1 Water:ACN (2.8 kg, 3 L/kg) then ACN (2.4 kg, 3 L/kg), before drying under full vacuum at 65 °C. Compound (I) was isolated in >99.5% purity and 91% yield.

NMP (6.2 kg, 6.0 L/kg) and Compound (I) (1.0 kg) were charged to a glass-lined reactor. The batch was heated to 70 °C to form a solution, which was then transferred through a polish filter to a clean vessel at 70 °C. 2-Propanol (2.4 kg, 3

L/kg) was added, followed by Compound I seeds (0.005 kg, 0.005 kg/kg). After aging for 1 h, additional 2-propanol (4.8 kg, 6 L/kg) was charged over the course of 2 h (3 L/kg/hr). The slurry was aged for 1 h at 70 °C, cooled slowly to 0 °C and aged for additional 12 h at 0 °C. Product was isolated by filtration. The wet cake was washed with 2-propanol (2 x 3.1 kg, 2 x 4 L/kg) before drying under full vacuum at 65 °C. Compound (I) was isolated in >99.9% purity and 83% yield.

**Preparation of Compound 7**

Step 1: Preparation of N-methyl-N-formyl hydrazine

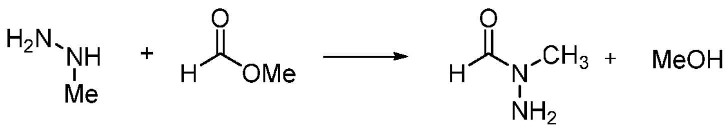

To a glass lined reactor were charged methanol (1.6 kg/kg, 2.0 L/kg) and methyl hydrazine (1 kg) at 0 °C. Methyl formate (0.57 kg/kg, 1.1 equiv.) was added drop-wise. The crude was warmed up to 20 °C and aged for additional 6 h. The crude was distilled under vacuum until total volume became approximately 0.5 L/kg. Five put/take distillations with 2-MeTHF (5 x 3.6 kg/kg) were undertaken for the purpose of azeotropic drying. The crude was cooled to 20 °C. N-Methyl-N-formyl hydrazine was isolated as 89-90 wt% solution in 89-91% yield.

Step 2: Preparation of Compound 5

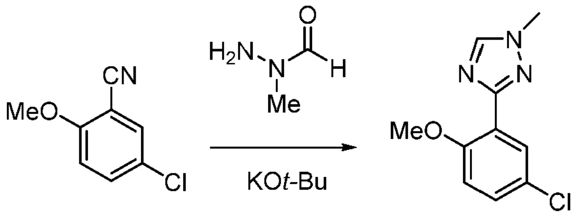


4 5

To a glass lined reactor were charged potassium tert-butoxide (1.5 kg/kg, 2.4 equiv) and THF (12.2 kg/kg) at 0 °C. A mixture of Compound 4 (1.0 kg), N-methyl-N-formyl hydrazine (1.0 kg/kg, 2.30 equiv) and THF (5.3 kg/kg, 6.0 L/kg) was added slowly. The reactor line was rinsed with THF (0.5 kg/kg). The reaction crude was aged at 0 °C until reaction reached completion. Water (5.0 kg/kg) was added, and the resulting mixture was aged at 0 °C for 30 min, heated to 40 °C and aged for additional 30 min. The layers were separated and the aqueous layer was discarded. The organic layer was washed with brine (15 wt%, 5.7 kg/kg) before distilling under vacuum until total volume became approximately 5 L/kg. Four put/take distillations with ethyl acetate (4 x 10 L/kg) were undertaken for the purpose of azeotropic drying. The crude was cooled to 20 °C. Sulfuric acid (0.66 kg/kg, 1.10 equiv.) was added, and the slurry was agitated for 2-3 h. Product was isolated by filtration. The cake was consecutively washed with ethyl acetate (2 × 6.5 L/kg) and heptane (8 L/kg), and dried under vacuum at 45 °C. Compound 5 was isolated in 99 AP and 83% yield.

Step 3: Preparation of Compound 6

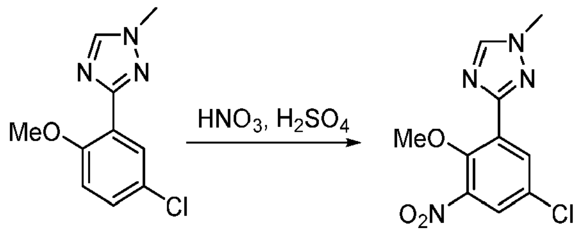


5 6

To a glass lined reactor were charged concentrated sulfuric acid (4.5 kg/kg) and Compound 5 (1.0 kg) at 0-5 °C. Nitric acid (68 wt%, 0.35 kg/kg, 1.2 equiv) was added drop-wise. The mixture was agitated at 0-5 °C until reaction reached completion.

In a separate reactor, water (12 kg/kg) and methanol (6.5 kg/kg, 8.3 L/kg) were mixed well at 20 °C. The nitration crude was transferred slowly into the methanol water mixture. The reactor line was rinsed with methanol (0.5 kg/kg). The crude was heated to 40-45 °C. Aqueous ammonium hydroxide (25 wt%, 7.4 kg/kg) was added slowly. The resulting slurry was cooled to 20 °C and agitated for 3 h. Product was isolated by filtration. The cake was washed with water (2 x 6 L/kg), and dried under vacuum at 45 °C. Compound 6 was isolated in 99 AP and 95% yield.

Step 4: Preparation of Compound 7

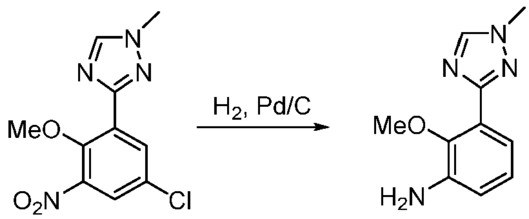


6 7

To a high pressure reactor flushed with nitrogen were charged methanol (8.0 kg/kg) and Compound 6 (1.0 kg). With careful exclusion of oxygen, sodium bicarbonate (0.6 kg/kg, 2.0 equiv.) and Pd/C (10% loading, 50 % wet, 0.02 kg/kg) were added. The reactor was pressurized with hydrogen (41-46 psi), and the reaction mixture was aged at 20 °C for 6 h then heated to 45 °C and aged till reaction reached completion. The reactor was flushed with nitrogen, and the reaction crude was filtered to remove Pd/C. Methanol (5 kg/kg) was used to aid the transfer. The combined filtrates were distilled under vacuum until total volume became approximately 2.5 L/kg. Water (10 kg/kg) was added, and the crude was distilled under vacuum until total volume became approximately 2.5 L/kg. The crude was heated to 70 °C. Brine (25 wt%, 9.0 kg/kg) was added, and the resulting crude was agitated for 6 h at 70 °C. After cooling down to 0 °C, the crude was further aged for 6 h. Product was isolated by filtration. The cake was washed with brine (pre-cooled to 0 °C, 25 wt%, 2.0 kg/kg), and dried under vacuum at 45 °C. Compound 7 was isolated in 99 AP and 88% yield.

**Preparation of Compound 13**

Step 1: Preparation of Compound 11 and Compound 12

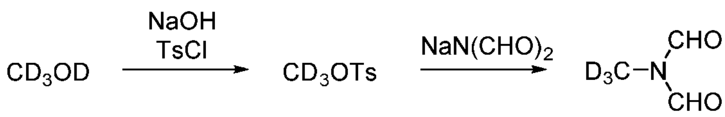


11 12

To a glass lined reactor flushed with nitrogen were charged water (16.3 L/kg) and sodium hydroxide (3.3 kg, 3.0 equiv). The mixture was aged till sodium hydroxide reached full dissolution. The crude was cooled to 0 °C. $d_4$-Methanol (1.0 kg) and THF (4.5 L/kg) were charged. A solution of TsCl (6.3 kg, 1.2 equiv) in THF (6.3 kg, 7.1 L/kg) was added over the

course of 2 h. The crude was agitated at 0 °C until reaction reached completion. The batch was warmed to 20 °C. The layers were separated. The collected organic layer was diluted with MTBE (4.0 kg, 5.4 L/kg), washed with brine twice (25 wt%, 4.0 kg followed by 12 kg). The organic layer was distilled under vacuum until total volume became approximately 10 L/kg. Two put/take distillations with ACN (2 × 10 L/kg) were undertaken for the purpose of azeotropic drying. The crude was cooled to 20°C. ACN (10.0 kg, 12.8 L/kg) and $NaN(CHO)_2$ (3.3 kg, 1.2 equiv.) were added. The crude was heated to 65 °C and agitated until reaction reached completion. After cooling down to 5 °C, the mixture was filtered, and the crude cake was washed with ACN twice (2 × 2.5 kg, 2 × 3.2 L/kg). The combined filtrates were distilled under vacuum until total volume became approximately 3 L/kg. The crude was cooled to 20 °C. Compound 12 was isolated as an oil with 80-85 wt% in 60-70% yield.

Step 2: Preparation of Compound 13

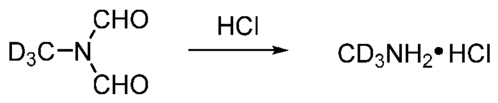


12 13

To a glass lined reactor were charged Compound 12 (1.0 kg) and methanol (3.9 kg, 5.0 L/kg) at 20 °C. A solution of HCl in IPA (5-6 Normal, 4.5 kg, 1.5 equiv) was added. The resulting mixture was heated to 50 °C and agitated until reaction reached completion. THF (10 kg, 11.2 L/kg) was added slowly and the crude was cooled to 0 °C over 2 h to afford a slurry. The product was isolated by filtration. The cake was washed with THF (3.7 kg, 4.1 L/kg), and dried under vacuum at 45 °C. Compound 13 was isolated in 80% yield.

Optional recrystallization of Compound 13:

Methanol (5.6 kg, 8.3 L/kg) and Compound 13 (1.0 kg) were charged to a glass-lined reactor. DBU (0.1 kg) was added slowly. The crude was agitated for 1 h. THF (12.4 kg, 13.9 L/kg) was added slowly, and the resulting slurry was aged for 2 h. The product was isolated by filtration. The cake was washed with THF (2.6 kg, 2.9 L/kg), and dried under vacuum at 45 °C. Compound 13 was isolated in 60% yield (1st crop). The mother liquor was distilled under vacuum until total volume became approximately 1 L/kg. Two put/take distillations with methanol (2 × 2.8 kg, 2 × 3.6 L/kg) were performed and the solution was concentrated back to ~ 1 L/kg. The crude was cooled to 20 °C. THF (4.8 kg, 5.4 L/kg) was added, and the resulting slurry was aged for 2 h. The product was isolated by filtration. The cake was washed with THF (1.0 kg), and dried under vacuum at 45 °C. Compound 13 was isolated in 25% yield (2nd crop).

**1. Single Crystal X-Ray Measurements**

Form Z: Single crystal X-ray data of Form Z were collected using a Bruker Kappa diffractometer equipped with a APEX II CCD detector and a MICROSTAR microfocus rotating anode X-ray generator of monochromatic Cu Kα radiation. The single crystal was at room temperature during data collection.

Indexing and processing of the measured intensity data were carried out with the APEX2 program suite (Bruker AXS, Inc., 5465 East Cheryl Parkway, Madison, WI 53711 USA).

The final unit cell parameters were determined using the full data set. The structures were solved by direct methods and refined by full-matrix least-squares approach using the SHELXTL software package (G. M. Sheldrick, SHELXTL v6.14, Bruker AXS, Madison, WI USA.). Structure refinements involved minimization of the function defined by $\sum w(|F_o| - |F_c|)^2$, where w is an appropriate weighting factor based on errors in the observed intensities, Fo is the structure factor based on measured reflections, and Fc is the structure factor based on calculated reflections. Agreement between the refined crystal structure model and the experimental X-ray diffraction data is assessed by using the residual factors $R = \sum||F_o|-|F_c||/\sum|F_o|$ and $wR = [\sum w(|F_o|-|F_c|)^2/\sum w|F_o|]^{1/2}$. Difference Fourier maps were examined at all stages of refinement. All non-hydrogen atoms were refined with anisotropic thermal displacement parameters. Hydrogen atoms were introduced using idealized geometry with isotropic temperature factors and included in structure factor calculations with fixed parameters.

Form AC: Single crystal X-ray data of Form AC were collected using a Bruker X8-Proteum diffractometer equipped with a APEX II CCD detector and a MICROSTAR microfocus rotating anode X-ray generator of monochromatic Cu Kα radiation. The single crystal was at room temperature during data collection.

Indexing and processing of the measured intensity data were carried out with the APEX2 program suite (Bruker

AXS, Inc., 5465 East Cheryl Parkway, Madison, WI 53711 USA).

The final unit cell parameters were determined using the full data set. The structures were solved by direct methods and refined by full-matrix least-squares approach using the SHELXTL software package (G. M. Sheldrick, SHELXTL v6.14, Bruker AXS, Madison, WI USA.). Structure refinements involved minimization of the function defined by $\sum w(|F_o| - |F_c|)^2$, where w is an appropriate weighting factor based on errors in the observed intensities, Fo is the structure factor based on measured reflections, and Fc is the structure factor based on calculated reflections. Agreement between the refined crystal structure model and the experimental X-ray diffraction data is assessed by using the residual factors $R = \sum||F_o|-|F_c||/\sum|F_o|$ and $wR = [\sum w(|F_o|-|F_c|)^2/\sum w|F_o|]^{1/2}$. Difference Fourier maps were examined at all stages of refinement. All non-hydrogen atoms were refined with anisotropic thermal displacement parameters. Hydrogen atoms were introduced using idealized geometry with isotropic temperature factors and included in structure factor calculations with fixed parameters.

**2. Powder X-Ray Diffraction**

Selected PXRD patterns of Forms L, M, N, O, P, Q, R, U, V, and W were collected with a PANalytical X'Pert PRO MPD diffractometer using an incident beam of Cu radiation produced by an Optix long, fine-focus source. An elliptically graded multilayer mirror was used to focus Cu K$\alpha$ X-rays through the specimen and onto the detector. Prior to the analysis, a silicon specimen (NIST SRM 640e) was analyzed to verify the Si 111 peak position. A specimen of the sample was sandwiched between 3 $\mu$m thick films and analyzed in transmission geometry. A beam-stop and short antiscatter extension were used to minimize the background generated by air. Soller slits for the incident and diffracted beams were used to minimize broadening from axial divergence. Diffraction patterns were collected using a scanning position-sensitive detector (X'Celerator) located 240 mm from the specimen and Data Collector software v.5.5.

PXRD patterns of Forms S, T, X and Y were collected with a PANalytical Empyrean diffractometer using an incident beam of Cu radiation produced using a long, fine-focus source. An elliptically graded multilayer mirror was used to focus Cu K$\alpha$ X-rays through the specimen and onto the detector. Prior to the analysis, a silicon specimen (NIST SRM 640e) was analyzed to verify the observed position of the Si 111 peak is consistent with the NIST-certified position. A specimen of the sample was sandwiched between 3-$\mu$m-thick films and analyzed in transmission geometry. A beam-stop, short antiscatter extension, and antiscatter knife edge were used to minimize the background generated by air. Soller slits for the incident and diffracted beams were used to minimize broadening and asymmetry from axial divergence. Diffraction patterns were collected using a scanning position-sensitive detector (X'Celerator) located 240 mm from the specimen and Data Collector software v. 5.5.

PXRD data of Forms Z, AA, AB, AC, AD, and AE were obtained using a Bruker C2 GADDS. The radiation was Cu K$\alpha$ (40 KV, 40 mA). The sample-detector distance was 15 cm. Samples were placed in sealed glass capillaries with diameters of $\leq$ 1mm. The capillary was rotated during data collection. Data were collected for approximately $2 \leq 2\theta \leq 32°$ with a sample exposure time of at least 1000 seconds. The resulting two dimensional diffraction arcs were integrated to create a traditional 1-dimensional PXRD pattern with a step size of 0.05 degrees 2$\theta$ in the approximate range of 2 to 32 degrees 2$\theta$.

**3. Differential Scanning Calorimetry**

Differential scanning calorimetry (DSC) experiments of Forms AA, AB, and AE were performed using a TA Instrument - model Q1000. The sample (about 1-10 mg) was weighed in an aluminum pan and the weight recorded accurately to a hundredth of a milligram before transferring the sample to the DSC. The instrument was purged with nitrogen gas at 50 mL/min. Data were collected between room temperature and 300 °C at a heating rate of 10 °C/min. DSC plots were generated such that the endothermic peaks pointed down.

**4. Thermal Gravimetric Analysis**

Thermal gravimetric analysis (TGA) experiment of Form AB was performed using a TA Instrument - model Q500. The sample (about 10-30 mg) was placed in a previously tarred platinum pan. The weight of the sample was measured accurately and recorded to a thousandth of a milligram by the instrument. The furnace was purged with nitrogen gas at 100 mL/min. Data were collected between room temperature and 300 °C at a heating rate of 10 °C/min.

**5. Thermogravimetric Analysis-Differential Scanning Calorimetry (TGA-DSC)**

DSC/TGA analysis of forms L, M, N, R, S and W was performed using a Mettler-Toledo TGA/DSC3+ analyzer. Temperature calibration was performed using calcium oxalate, indium, tin, and zinc. The sample was placed in an aluminum pan. The sample was sealed, the lid pierced, then inserted into the TG furnace. The furnace was heated under nitrogen with flow of 50 mL/min. The method included heating from room temperature to 350 °C with heating rate 10

°C/min.

## Claims

1. Crystalline Form R of 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide **characterized by** at least one of the following:

   (i) a powder X-ray diffraction (PXRD) pattern comprising two or more 2θ values in degrees (CuKα) selected from: 9.5±0.2, 10.0±0.2, 16.4±0.2, 17.2±0.2, 22.0±0.2, and 22.8±0.2, wherein the PXRD pattern is measured at room temperature;
   (ii) an observed powder X-ray diffraction (PXRD) pattern substantially as shown in FIG. 10, wherein the PXRD pattern is measured at room temperature;
   (iii) a differential scanning calorimetry (DSC) thermogram substantially as shown in FIG. 11 (bottom graph);
   (iv) a thermogravimetric analysis (TGA) thermogram comprising a weight loss of less than about 1.0% when heated from about room temperature to about 200 °C;
   (v) a thermogravimetric analysis (TGA) thermogram substantially as shown in FIG. 11 (top graph).

2. The crystalline form according to claim 1 **characterized by** a powder X-ray diffraction (PXRD) pattern comprising three or more 2θ values in degrees (CuKα) selected from: 9.5±0.2, 10.0±0.2, 16.4±0.2, 17.2±0.2, 22.0±0.2, and 22.8±0.2, wherein the PXRD pattern is measured at room temperature

3. The crystalline form according to claim 1 **characterized by** a powder X-ray diffraction (PXRD) pattern comprising four or more 2θ values in degrees (CuKα) selected from: 9.5±0.2, 10.0±0.2, 16.4±0.2, 17.2±0.2, 22.0±0.2, and 22.8 ±0.2, wherein the PXRD pattern is measured at room temperature

4. The crystalline form according to claim 1 **characterized by** a powder X-ray diffraction (PXRD) pattern comprising five or more 2θ values in degrees (CuKα) selected from: 9.5±0.2, 10.0±0.2, 16.4±0.2, 17.2±0.2, 22.0±0.2, and 22.8 ±0.2, wherein the PXRD pattern is measured at room temperature

5. The crystalline form according to claim 1 **characterized by** a powder X-ray diffraction pattern (PXRD) comprising 2θ values in degrees (CuKα) at 9.5±0.2 and 10.0±0.2, wherein the PXRD pattern of the crystalline form is measured at room temperature.

6. The crystalline form according to claim 5 wherein the PXRD pattern further comprises one or more 2θ values in degrees selected from: 16.4±0.2, 17.2±0.2, 22.0±0.2, and 22.8±0.2.

7. The crystalline form according to claim 1 **characterized by** a powder X-ray diffraction pattern (PXRD) comprising 2θ values in degrees (CuKα) at 9.5±0.2, 10.0±0.2, and 16.4±0.2, wherein the PXRD pattern of the crystalline form is measured at room temperature.

8. The crystalline form according to claim 1 **characterized by** a powder X-ray diffraction pattern (PXRD) comprising 2θ values in degrees (CuKα) at 9.5±0.2, 10.0±0.2, 16.4±0.2, and 17.2±0.2, wherein the PXRD pattern of the crystalline form is measured at room temperature.

9. The crystalline form according to claim 1 **characterized by** a powder X-ray diffraction pattern (PXRD) comprising 2θ values in degrees (CuKα) at 9.5±0.2, 10.0±0.2, 16.4±0.2, 17.2±0.2, and 22.0±0.2, wherein the PXRD pattern of the crystalline form is measured at room temperature.

10. The crystalline form according to claim 1 **characterized by** a powder X-ray diffraction pattern (PXRD) comprising 2θ values in degrees (CuKα) at 9.5±0.2, 10.0±0.2, 16.4±0.2, 17.2±0.2, 22.0±0.2, and 22.8±0.2, wherein the PXRD pattern of the crystalline form is measured at room temperature.

11. The crystalline form according to claim 1 **characterized by** (i) a powder X-ray diffraction (PXRD) pattern comprising 2θ values in degrees (CuKα) at 9.5±0.2 and 10.0±0.2, wherein the PXRD pattern is measured at room temperature; and (ii) a differential scanning calorimetry (DSC) thermogram substantially as shown in FIG. 11 (bottom graph).

12. A composition comprising 6-(cyclopropanecarboxamido)-4-((2-methoxy-3-(1-methyl-1H-1,2,4-triazol-3-yl)phenyl)

amino)-N-(methyl-$d_3$)pyridazine-3-carboxamide, wherein at least 90 weight % of said 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide is in the crystalline Form R according to any one of claims 1 to 11.

**13.** A pharmaceutical composition comprising the crystalline Form R according to any one of claims 1 to 11.

**14.** A method of preparing the crystalline form according to claim 1, comprising the steps of

(a) dissolving 1,2-ethanedisulfonic acid dihydrate in acetone at 50 °C, to form a solution;
(b) adding the solution to 2 mole equivalents of 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide, to form a suspension;
(c) slurrying the suspension at 50 °C for 90 minutes; and
(d) hot filtering the suspension with positive pressure, to yield solids comprising crystalline Form R of 6-(cyclopropanecarboxamido)-4-((2-methoxy-3-(1-methyl-1H-1,2,4-triazol-3-yl)phenyl) amino)-N-(methyl-$d_3$)pyridazine-3-carboxamide.

**15.** Use of the crystalline Form R according to any one of claims 1 to 11 in a method of preparing amorphous Compound (I).

## Patentansprüche

**1.** Kristalline Form R von 6-(Cyclopropancarboxamido)-4-((2-methoxy-3-(1-methyl-1H-1,2,4-triazol-3-yl)phenyl)amino)-N-(methyl-$d_3$)pyridazin-3-carboxamid, **gekennzeichnet durch** mindestens eines der folgenden Merkmale:

(i) ein Pulver-Röntgenbeugungsmuster (PXRD-Muster), das zwei oder mehr 2θ-Werte in Grad (CuKα) umfasst, ausgewählt aus: 9,5 ± 0,2, 10,0 ± 0,2, 16,4 ± 0,2, 17,2 ± 0,2, 22,0 ± 0,2 und 22,8 ± 0,2, wobei das PXRD-Muster bei Raumtemperatur gemessen wird;
(ii) ein beobachtetes Pulver-Röntgenbeugungsmuster (PXRD-Muster), das im Wesentlichen dem in FIG. 10 gezeigten entspricht, wobei das PXRD-Muster bei Raumtemperatur gemessen wird;
(iii) ein Differential-Scanning-Kalorimetrie-Thermogramm (DSC-Thermogramm), das im Wesentlichen dem in FIG. 11 (unteres Diagramm) gezeigten entspricht;
(iv) ein Thermogramm einer thermogravimetrischen Analyse (TGA), das einen Gewichtsverlust von weniger als etwa 1,0 % bei Erhitzung von etwa Raumtemperatur auf etwa 200 °C umfasst;
(v) ein Thermogramm einer thermogravimetrischen Analyse (TGA), das im Wesentlichen dem in FIG. 11 (oberes Diagramm) gezeigten entspricht.

**2.** Kristalline Form nach Anspruch 1, **gekennzeichnet durch** ein Pulver-Röntgenbeugungsmuster (PXRD-Muster), das drei oder mehr 2θ-Werte in Grad (CuKα) umfasst, ausgewählt aus: 9,5±0,2, 10,0±0,2, 16,4±0,2, 17,2±0,2, 22,0 ±0,2 und 22,8±0,2, wobei das PXRD-Muster bei Raumtemperatur gemessen wird;

**3.** Kristalline Form nach Anspruch 1, **gekennzeichnet durch** ein Pulver-Röntgenbeugungsmuster (PXRD-Muster), das vier oder mehr 2θ-Werte in Grad (CuKα) umfasst, ausgewählt aus: 9,5 ± 0,2, 10,0 ± 0,2, 16,4 ± 0,2, 17,2 ± 0,2, 22,0 ± 0,2 und 22,8 ± 0,2, wobei das PXRD-Muster bei Raumtemperatur gemessen wird;

**4.** Kristalline Form nach Anspruch 1, **gekennzeichnet durch** ein Pulver-Röntgenbeugungsmuster (PXRD-Muster), das fünf oder mehr 2θ-Werte in Grad (CuKα) umfasst, ausgewählt aus: 9,5 ± 0,2, 10,0 ± 0,2, 16,4 ± 0,2, 17,2 ± 0,2, 22,0 ± 0,2 und 22,8 ± 0,2, wobei das PXRD-Muster bei Raumtemperatur gemessen wird;

**5.** Kristalline Form nach Anspruch 1, **gekennzeichnet durch** ein Pulver-Röntgenbeugungsmuster (PXRD), das 2θ-Werte in Grad (CuKα) bei 9,5 ± 0,2 und 10,0 ± 0,2 umfasst, wobei das PXRD-Muster der kristallinen Form bei Raumtemperatur gemessen wird.

**6.** Kristalline Form nach Anspruch 5, wobei das PXRD-Muster ferner einen oder mehr 2θ-Werte in Grad (CuKα) umfasst, ausgewählt aus: 16,4 ± 0,2, 17,2 ± 0,2, 22,0 ± 0,2, und 22,8 ± 0,2.

**7.** Kristalline Form nach Anspruch 1, **gekennzeichnet durch** ein Pulver-Röntgenbeugungsmuster (PXRD), das 2θ-Werte in Grad (CuKα) bei 9,5 ± 0,2, 10,0±,2 und 16,4 ± 0,2 umfasst, wobei das PXRD-Muster der kristallinen Form bei Raumtemperatur gemessen wird.

8. Kristalline Form nach Anspruch 1, **gekennzeichnet durch** ein Pulver-Röntgenbeugungsmuster (PXRD), das 2θ-Werte in Grad (CuKα) bei 9,5 ± 0,2, 10,0±,2 und 16,4 ± 0,2 und 17,2 ± 0,2 umfasst, wobei das PXRD-Muster der kristallinen Form bei Raumtemperatur gemessen wird.

9. Kristalline Form nach Anspruch 1, **gekennzeichnet durch** ein Pulver-Röntgenbeugungsmuster (PXRD), das 2θ-Werte in Grad (CuKα) bei 9,5 ± 0,2, 10,0±,2 und 16,4 ± 0,2, 17,2 ± 0,2 und 22,0 ± 0,2 umfasst, wobei das PXRD-Muster der kristallinen Form bei Raumtemperatur gemessen wird.

10. Kristalline Form nach Anspruch 1, **gekennzeichnet durch** ein Pulver-Röntgenbeugungsmuster (PXRD), das 2θ-Werte in Grad (CuKα) bei 9,5 ± 0,2, 10,0 ± 0,2, 16,4 ± 0,2, 17,2 ± 0,2, 22,0 ± 0,2 und 22,8 ± 0,2 umfasst, wobei das PXRD-Muster der kristallinen Form bei Raumtemperatur gemessen wird.

11. Kristalline Form nach Anspruch 1, **gekennzeichnet durch** (i) ein Pulver-Röntgenbeugungsmuster (PXRD-Muster), das 2θ-Werte in Grad (CuKα) bei 9,5 ± 0,2 und 10,0 ± 0,2 umfasst, wobei das PXRD-Muster bei Raumtemperatur gemessen wird; und (ii) ein Differential-Scanning-Kalorimetrie-Thermogramm (DSC-Thermogramm), das im Wesentlichen dem in FIG. 11 gezeigten (unteres Diagramm) entspricht.

12. Zusammensetzung, umfassend 6-(Cyclopropancarboxamido)-4-((2-methoxy-3-(1-methyl-1H-1,2,4-triazol-3-yl)phenyl)amino)-N-(methyl-$d_3$)pyridazin-3-carboxamid, wobei mindestens 90 Gew.-% des 6-(Cyclopropancarboxamido)-4-((2-methoxy-3-(1-methyl-1H-1,2,4-triazol-3-yl)phenyl)amino)-N-(methyl-$d_3$)pyridazin-3-carboxamid in der kristallinen Form R nach einem der Ansprüche 1 bis 11 vorliegt.

13. Pharmazeutische Zusammensetzung, umfassend die kristalline Form R nach einem der Ansprüche 1 bis 11.

14. Verfahren zum Herstellen der kristallinen Form nach Anspruch 1, folgende Schritte umfassend:

(a) Lösen von 1,2-Ethandisulfonsäuredihydrat in Aceton bei 50 °C, um eine Lösung zu bilden;
(b) Zugeben der Lösung zu 2 Moläquivalenten 6-(Cyclopropancarboxamido)-4-((2-methoxy-3-(1-methyl-1H-1,2,4-triazol-3-yl)phenyl)amino)-N-(methyl-$d_3$)pyridazin-3-carboxamid, um eine Suspension zu bilden;
(c) Aufschlämmen der Suspension bei 50 °C über 90 Minuten; und
(d) Heißfiltrieren der Suspension unter Überdruck, um Feststoffe zu erhalten, welche die kristalline Form R von 6-(Cyclopropancarboxamido)-4-((2-methoxy-3-(1-methyl-1H-1,2,4-triazol-3-yl)phenyl)amino)-N-(methyl-$d_3$)pyridazin-3-carboxamid umfassen.

15. Verwendung der kristallinen Form R nach einem der Ansprüche 1 bis 11 in einem Verfahren zur Herstellung der amorphen Verbindung (I).

## Revendications

1. Forme cristalline R de 6-(cyclopropanecarboxamido)-4-((2-méthoxy-3-(1-méthyl-1H-1,2,4-triazol-3-yl)phényl)amino)-N-(méthyl-$d_3$)pyridazine-3-carboxamide **caractérisée par** au moins une caractéristique parmi:

(i) un diagramme de diffraction des rayons X de poudre (PXRD) comprenant deux valeurs 2θ ou plus en degrés (CuKα) choisies parmi: 9,5 ± 0,2, 10,0 ± 0,2, 16,4 ± 0,2, 17,2 ± 0,2, 22,0 ± 0,2 et 22,8 ± 0,2, dans lequel le diagramme PXRD est mesuré à la température ambiante;
(ii) un diagramme de diffraction des rayons X de poudre (PXRD) observé essentiellement comme il est montré dans la FIG. 10, dans lequel le diagramme PXRD est mesuré à la température ambiante;
(iii) un thermogramme de calorimétrie différentielle à balayage (DSC) essentiellement comme il est montré dans la FIG. 11 (graphique du bas);
(iv) un thermogramme d'analyse thermogravimétrique (TGA) comprenant une perte de poids de moins d'environ 1,0% lors d'un chauffage d'environ la température ambiante à environ 200°C;
(v) un thermogramme d'analyse thermogravimétrique (TGA) essentiellement comme il est montré dans la FIG. 11 (graphique du haut).

2. Forme cristalline selon la revendication 1, **caractérisée par** un diagramme de diffraction des rayons X de poudre (PXRD) comprenant trois valeurs 2θ ou plus en degrés (CuKα) choisies parmi: 9,5 ± 0,2, 10,0 ± 0,2, 16,4 ± 0,2, 17,2 ± 0,2, 22,0 ± 0,2 et 22,8 ± 0,2, où le diagramme PXRD est mesuré à la température ambiante.

3. Forme cristalline selon la revendication 1, **caractérisée par** un diagramme de diffraction des rayons X de poudre (PXRD) comprenant quatre valeurs 2θ ou plus en degrés (CuKα) choisies parmi: 9,5 ± 0,2, 10,0 ± 0,2, 16,4 ± 0,2, 17,2 ± 0,2, 22,0 ± 0,2 et 22,8 ± 0,2, où le diagramme PXRD est mesuré à la température ambiante.

4. Forme cristalline selon la revendication 1, **caractérisée par** un diagramme de diffraction des rayons X de poudre (PXRD) comprenant cinq valeurs 2θ ou plus en degrés (CuKα) choisies parmi: 9,5 ± 0,2, 10,0 ± 0,2, 16,4 ± 0,2, 17,2 ± 0,2, 22,0 ± 0,2 et 22,8 ± 0,2, où le diagramme PXRD est mesuré à la température ambiante.

5. Forme cristalline selon la revendication 1, **caractérisée par** un diagramme de diffraction des rayons X de poudre (PXRD) comprenant des valeurs 2θ en degrés (CuKα) à 9,5 ± 0,2 et 10,0 ± 0,2, où le diagramme PXRD de la forme cristalline est mesuré à la température ambiante.

6. Forme cristalline selon la revendication 5, où le diagramme PXRD comprend en outre une ou plusieurs valeurs 2θ en degrés (CuKα) choisies parmi: 16,4 ± 0,2, 17,2 ± 0,2, 22,0 ± 0,2 et 22,8 ± 0,2.

7. Forme cristalline selon la revendication 1, **caractérisée par** un diagramme de diffraction des rayons X de poudre (PXRD) comprenant des valeurs 2θ en degrés (CuKα) à 9,5 ± 0,2, 10,0 ± 0,2 et 16,4 ± 0,2, où le diagramme PXRD de la forme cristalline est mesuré à la température ambiante.

8. Forme cristalline selon la revendication 1, **caractérisée par** un diagramme de diffraction des rayons X de poudre (PXRD) comprenant des valeurs 2θ en degrés (CuKα) à 9,5 ± 0,2, 10,0 ± 0,2, 16,4 ± 0,2 et 17,2 ± 0,2, où le diagramme PXRD de la forme cristalline est mesuré à la température ambiante.

9. Forme cristalline selon la revendication 1, **caractérisée par** un diagramme de diffraction des rayons X de poudre (PXRD) comprenant des valeurs 2θ en degrés (CuKα) à 9,5 ± 0,2, 10,0 ± 0,2, 16,4 ± 0,2, 17,2 ± 0,2 et 22,0 ± 0,2, où le diagramme PXRD de la forme cristalline est mesuré à la température ambiante.

10. Forme cristalline selon la revendication 1, **caractérisée par** un diagramme de diffraction des rayons X de poudre (PXRD) comprenant des valeurs 2θ en degrés (CuKα) à 9,5 ± 0,2, 10,0 ± 0,2, 16,4 ± 0,2, 17,2 ± 0,2, 22,0 ± 0,2 et 22,8 ± 0,2, où le diagramme PXRD de la forme cristalline est mesuré à la température ambiante.

11. Forme cristalline selon la revendication 1, **caractérisée par** (i) un diagramme de diffraction des rayons X de poudre (PXRD) comprenant des valeurs 2θ en degrés (CuKα) à 9,5 ± 0,2 et 10,0 ± 0,2, où le diagramme PXRD est mesuré à la température ambiante; et (ii) un thermogramme de calorimétrie différentielle à balayage (DSC) essentiellement comme il est montré dans la FIG. 11 (graphique du bas).

12. Composition comprenant le 6-(cyclopropanecarboxamido)-4-((2-méthoxy-3-(1-méthyl-1H-1,2,4-triazol-3-yl)phényl) amino)-N-(méthyl-$d_3$)pyridazine-3-carboxamide, dans laquelle au moins 90% en poids dudit 6-(cyclopropanecarboxamido)-4-((2-méthoxy-3-(1-méthyl-1H-1,2,4-triazol-3-yl)phényl)amino)-N-(méthyl-$d_3$)pyridazine-3-carboxamide est sous la forme cristalline R selon l'une quelconque des revendications 1 à 11.

13. Composition pharmaceutique comprenant la forme cristalline R selon l'une quelconque des revendications 1 à 11.

14. Procédé de préparation de la forme cristalline selon la revendication 1, comprenant les étapes de:

(a) dissolution de dihydrate d'acide 1,2-éthanedisulfonique dans de l'acétone à 50°C, pour former une solution;
(b) ajout de la solution à 2 équivalents molaires de 6-(cyclopropanecarboxamido)-4-((2-méthoxy-3-(1-méthyl-1H-1,2,4-triazol-3-yl)phényl)-amino)-N-(méthyl-$d_3$)pyridazine-3-carboxamide, pour former une suspension;
(c) épaississement de la suspension à 50°C pendant 90 minutes; et
(d) filtration à chaud de la suspension avec une pression positive, pour donner des solides comprenant la forme cristalline R du 6-(cyclopropanecarboxamido)-4-((2-méthoxy-3-(1-méthyl-1H-1,2,4-triazol-3-yl)phényl)amino)-N-(méthyl-$d_3$)pyridazine-3-carboxamide.

15. Utilisation de la forme cristalline R selon l'une quelconque des revendications 1 à 11 dans un procédé de préparation d'un composé (I) amorphe.

FIG. 1

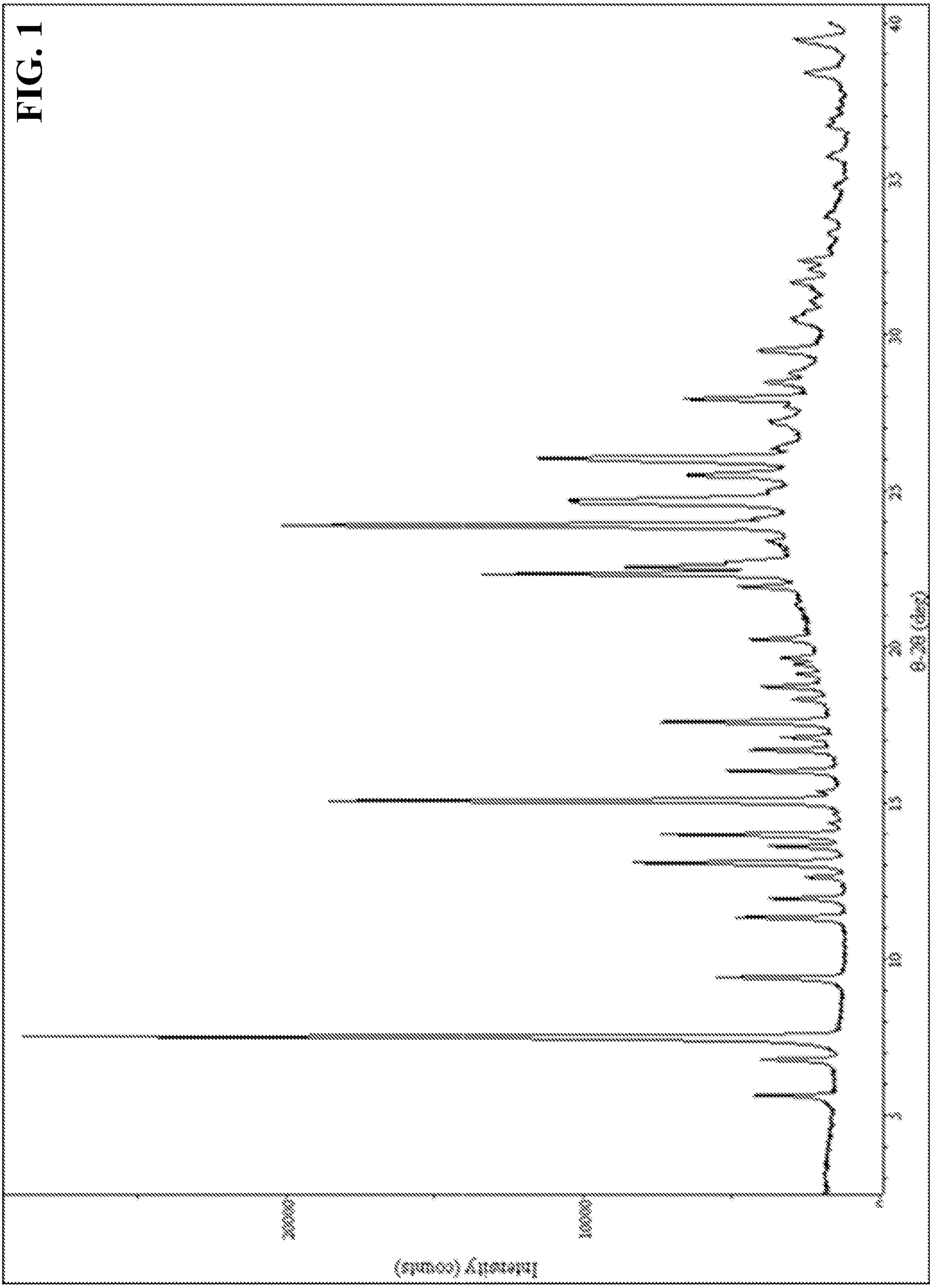

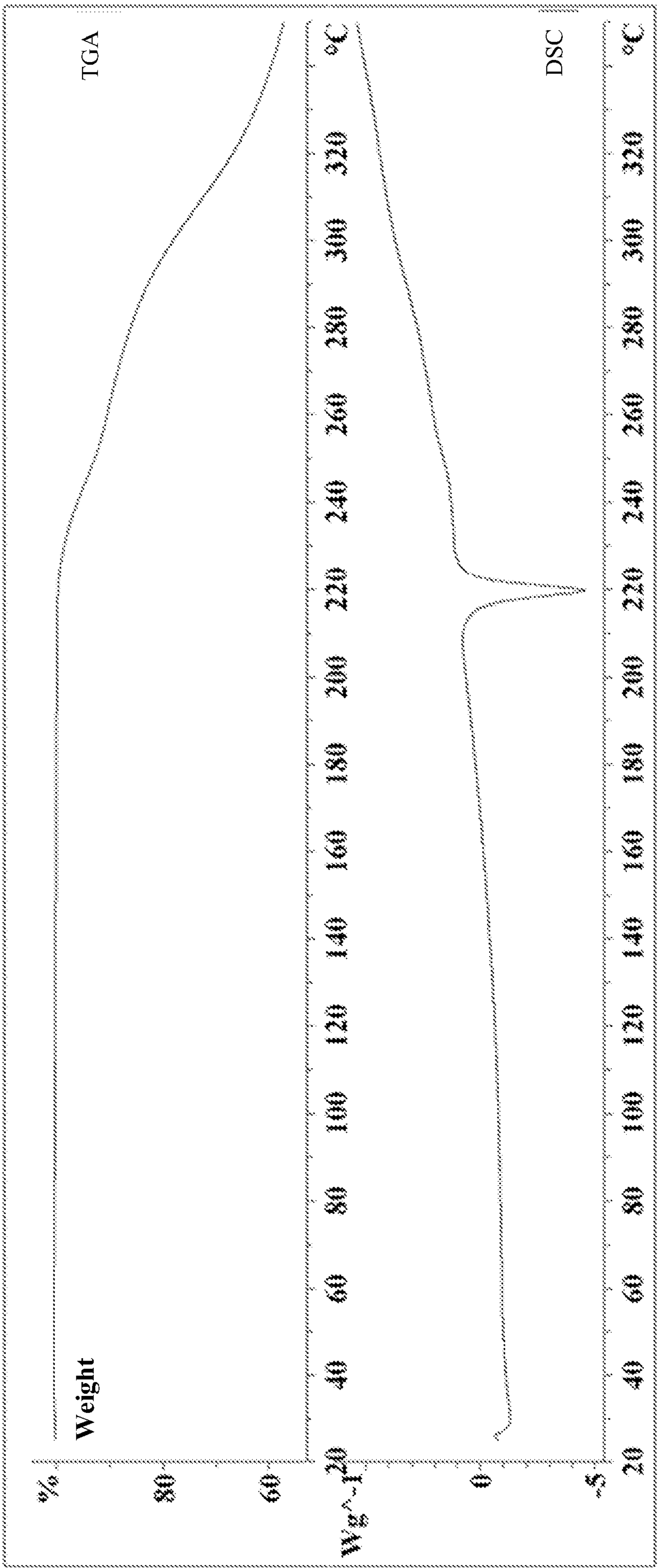
TGA
Weight
%
80
60
DSC
Wg^-1
0
-5
°C
20
40
60
80
100
120
140
160
180
200
220
240
260
280
300
320

FIG. 2

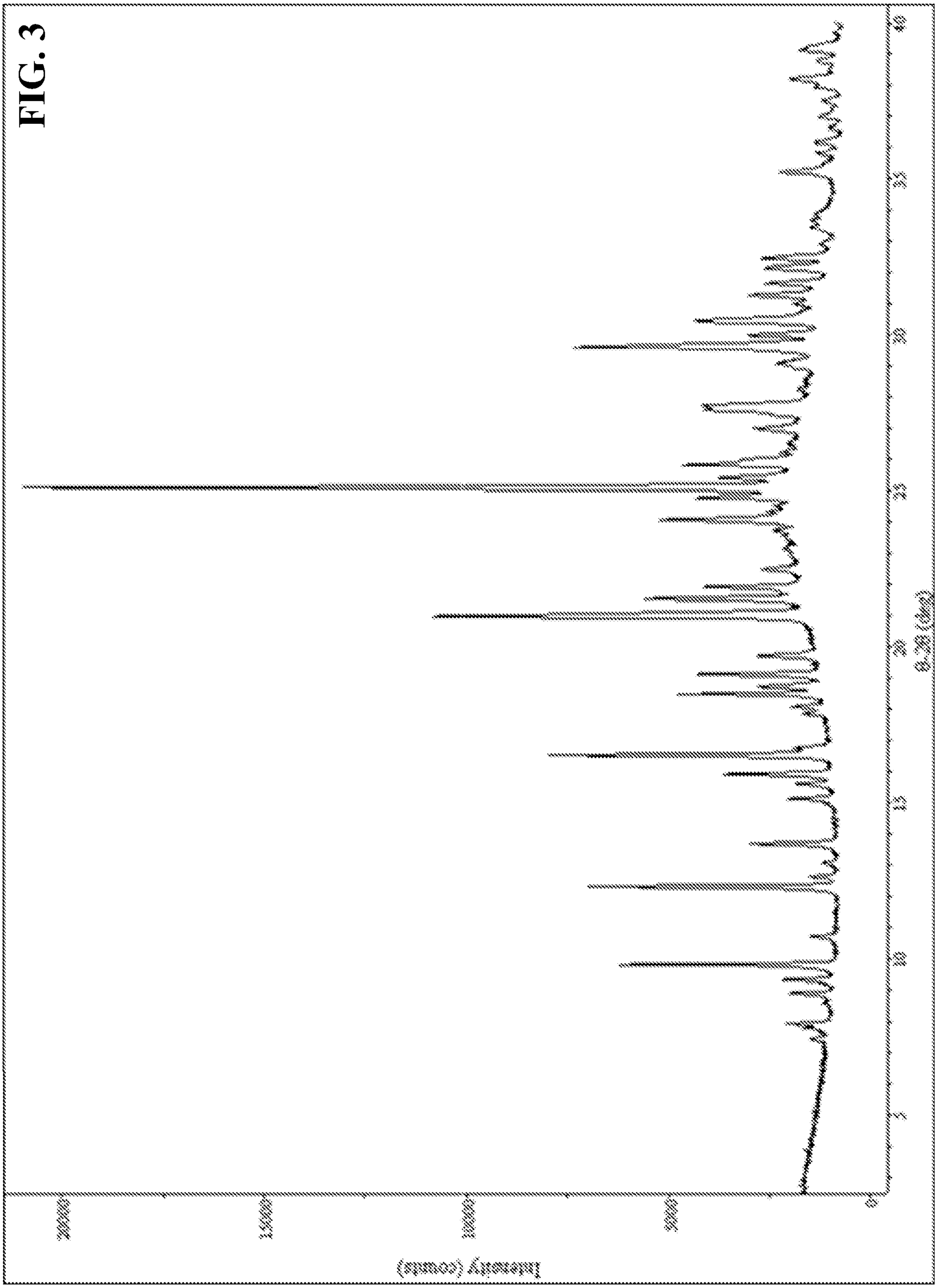
Intensity (counts)
0
5000
10000
15000
20000
5
10
15
20
25
30
35
40

FIG. 3

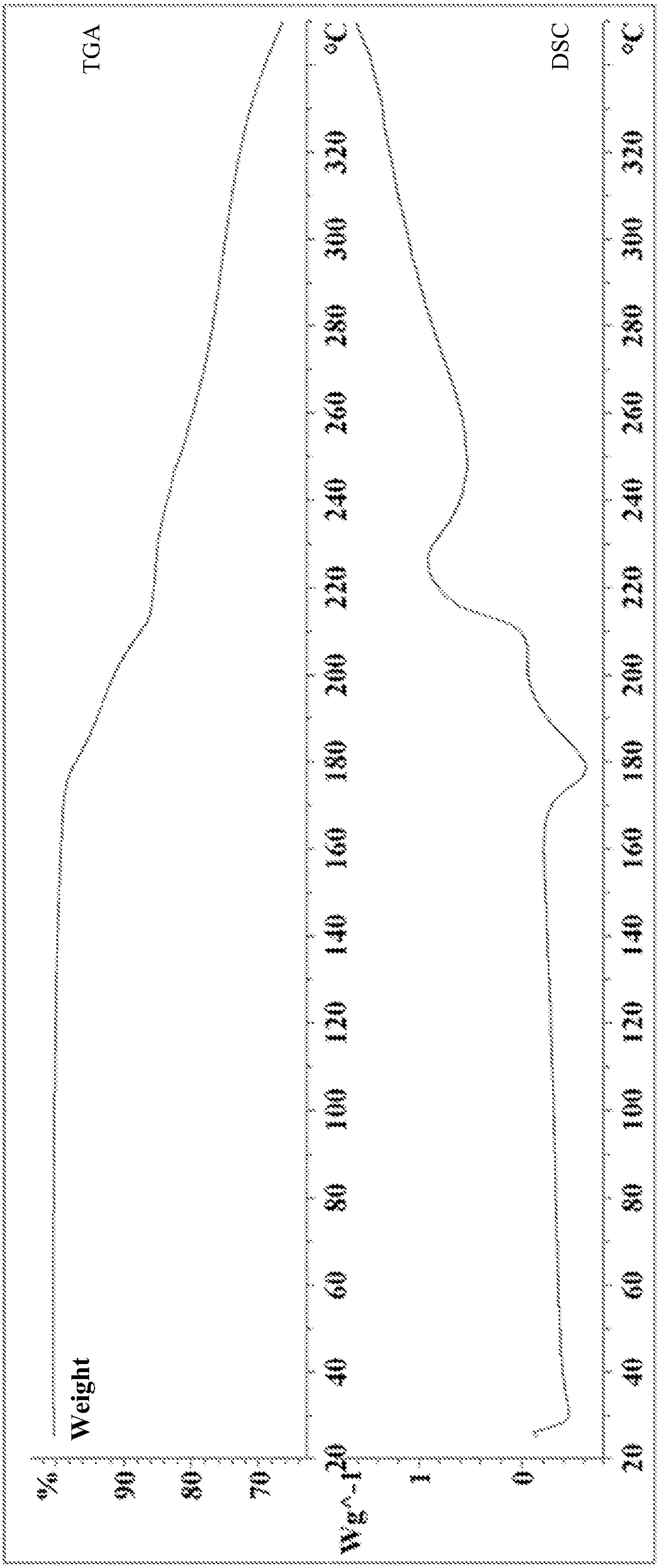
TGA
Weight
%
90
80
70
DSC
Wg^-1
1
0
20
40
60
80
100
120
140
160
180
200
220
240
260
280
300
320
°C

FIG. 4

FIG. 5

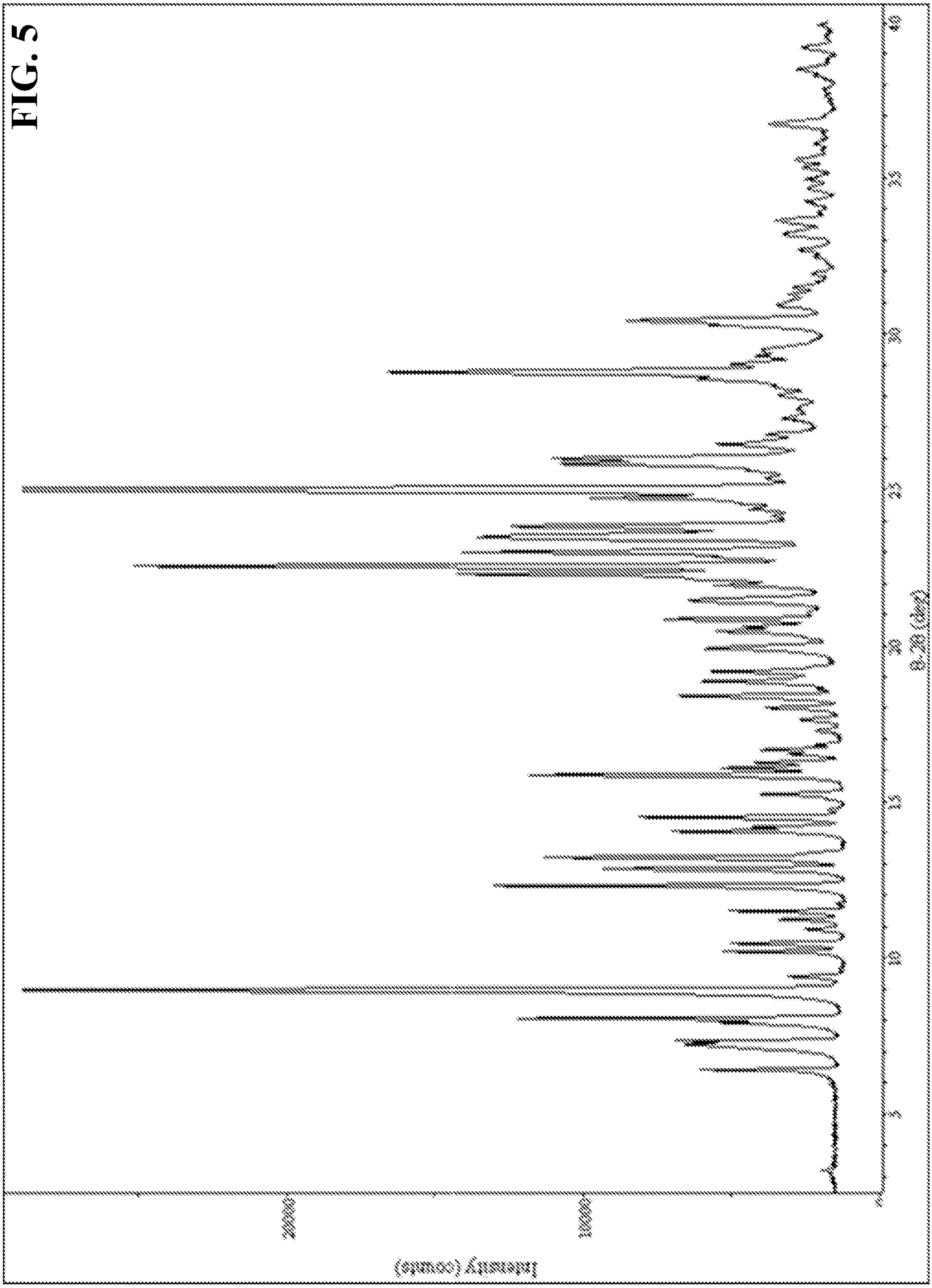

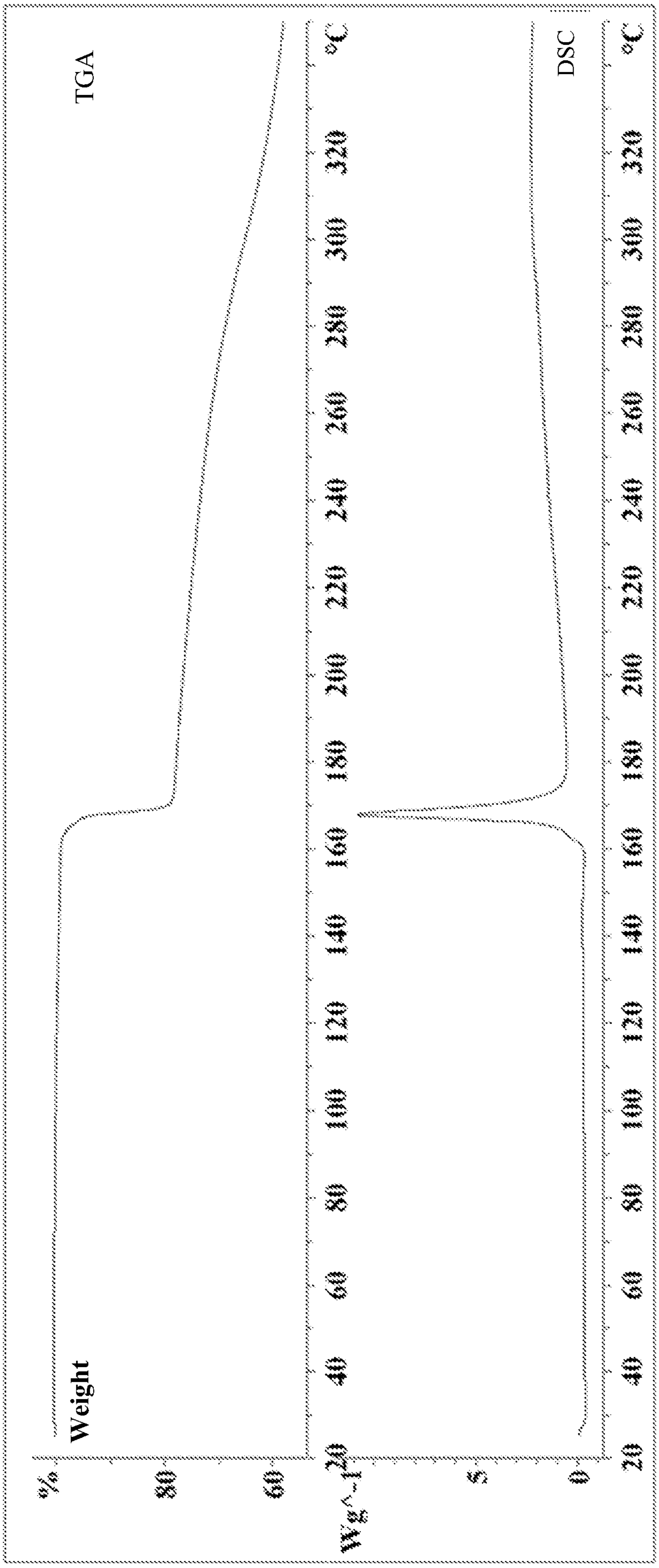
TGA
Weight
%
80
60
Wg^-1
5
0
DSC
°C
20
40
60
80
100
120
140
160
180
200
220
240
260
280
300
320

FIG. 6

FIG. 7

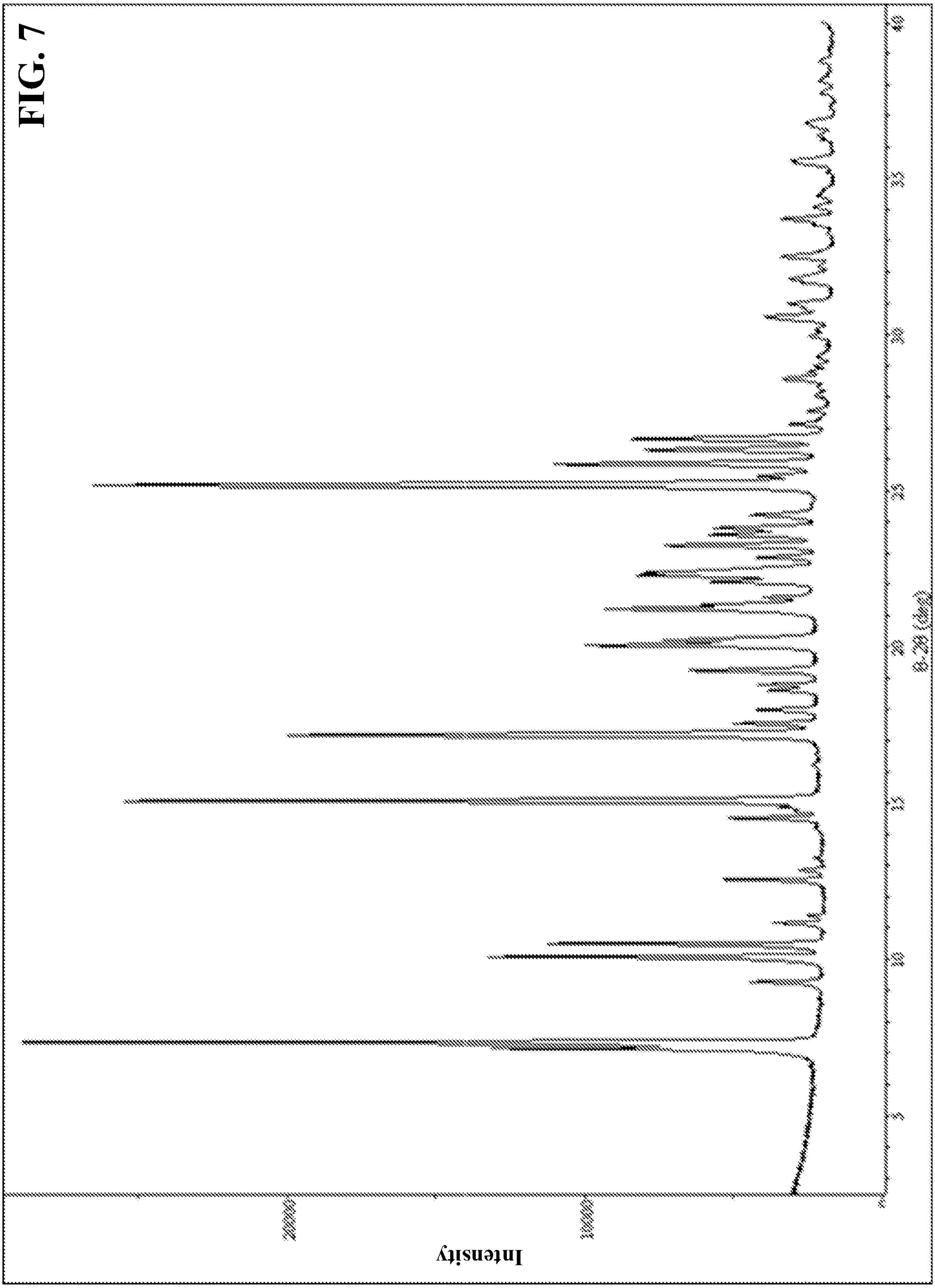

FIG. 8

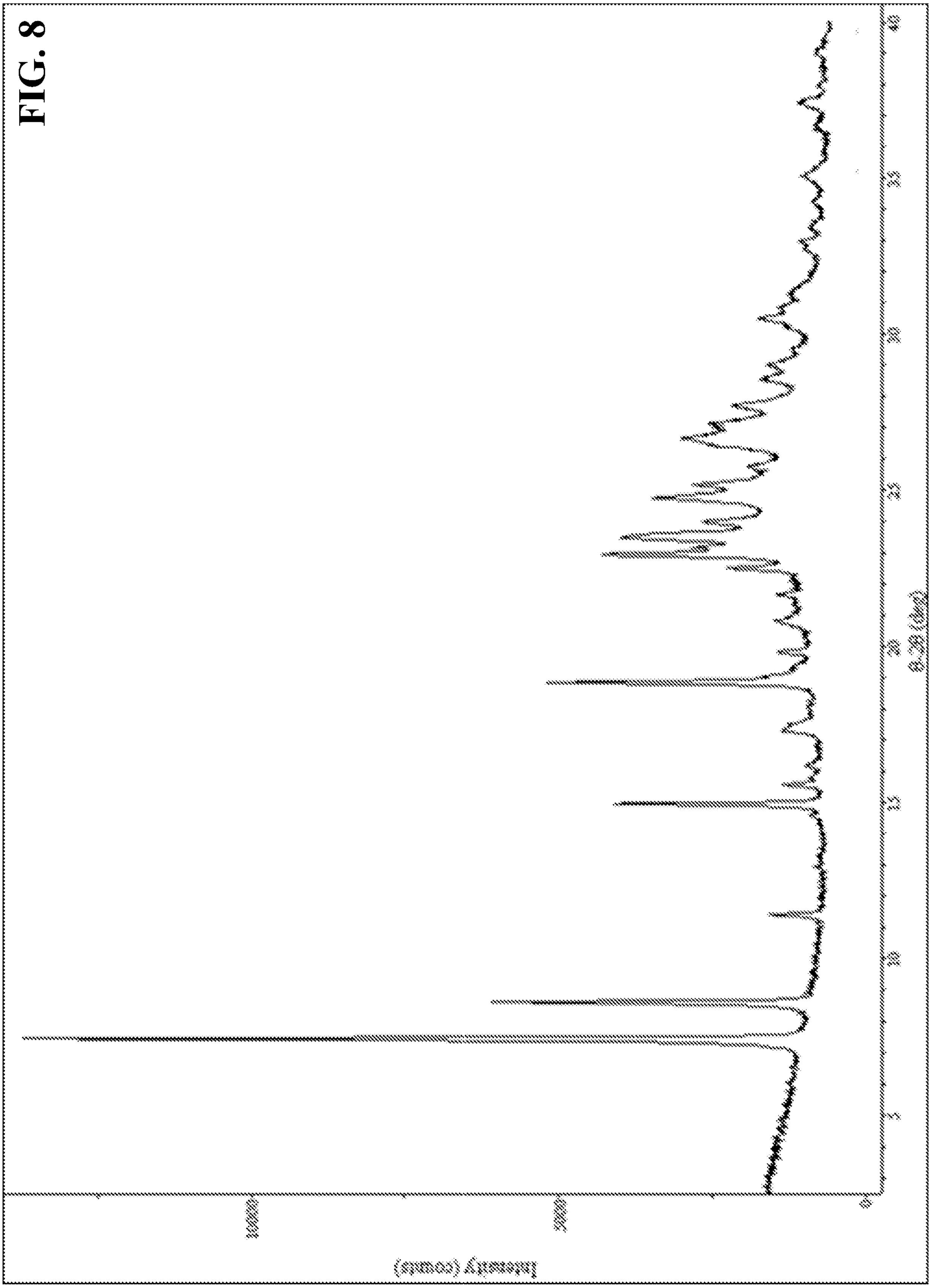

FIG. 9

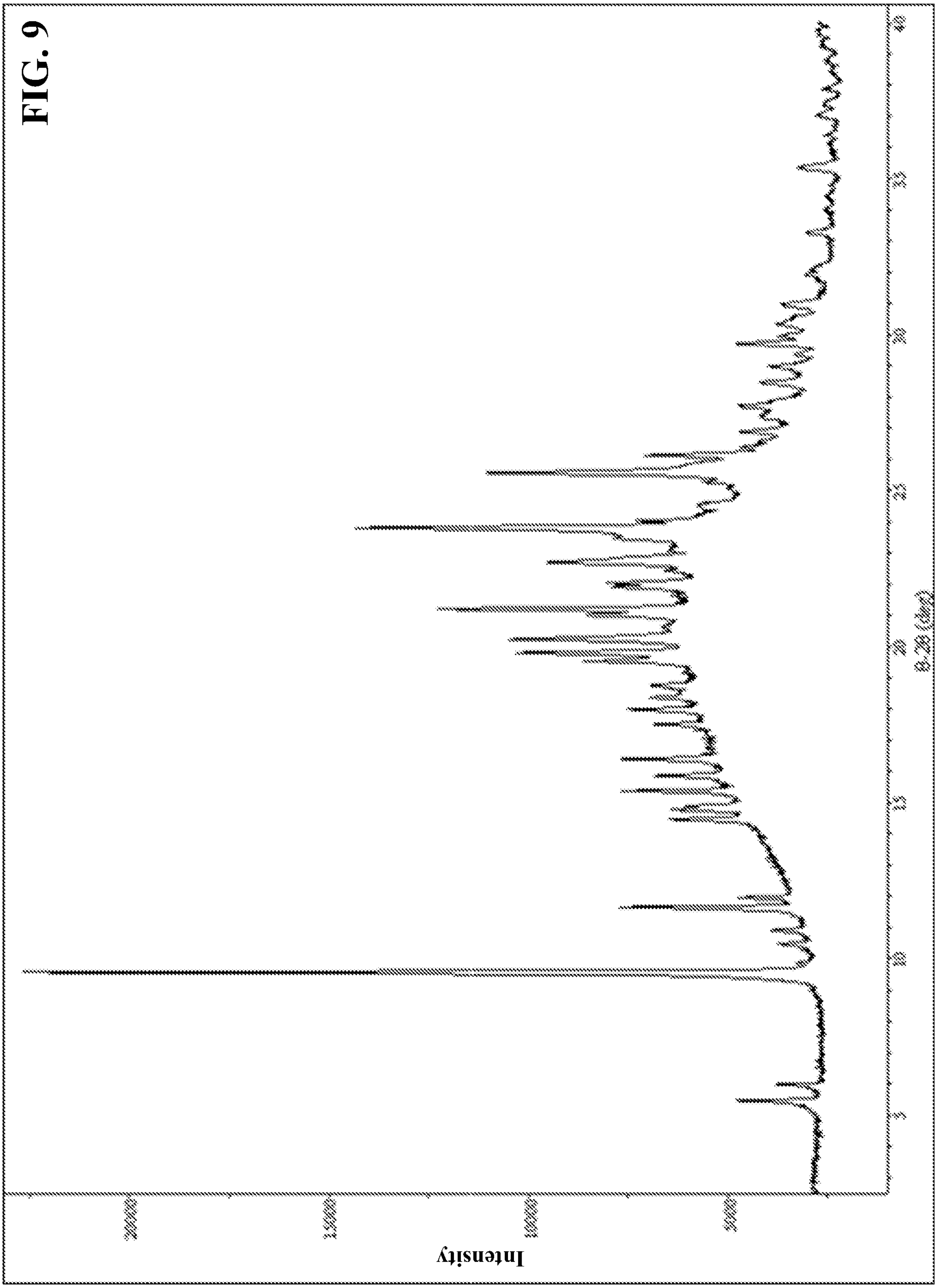

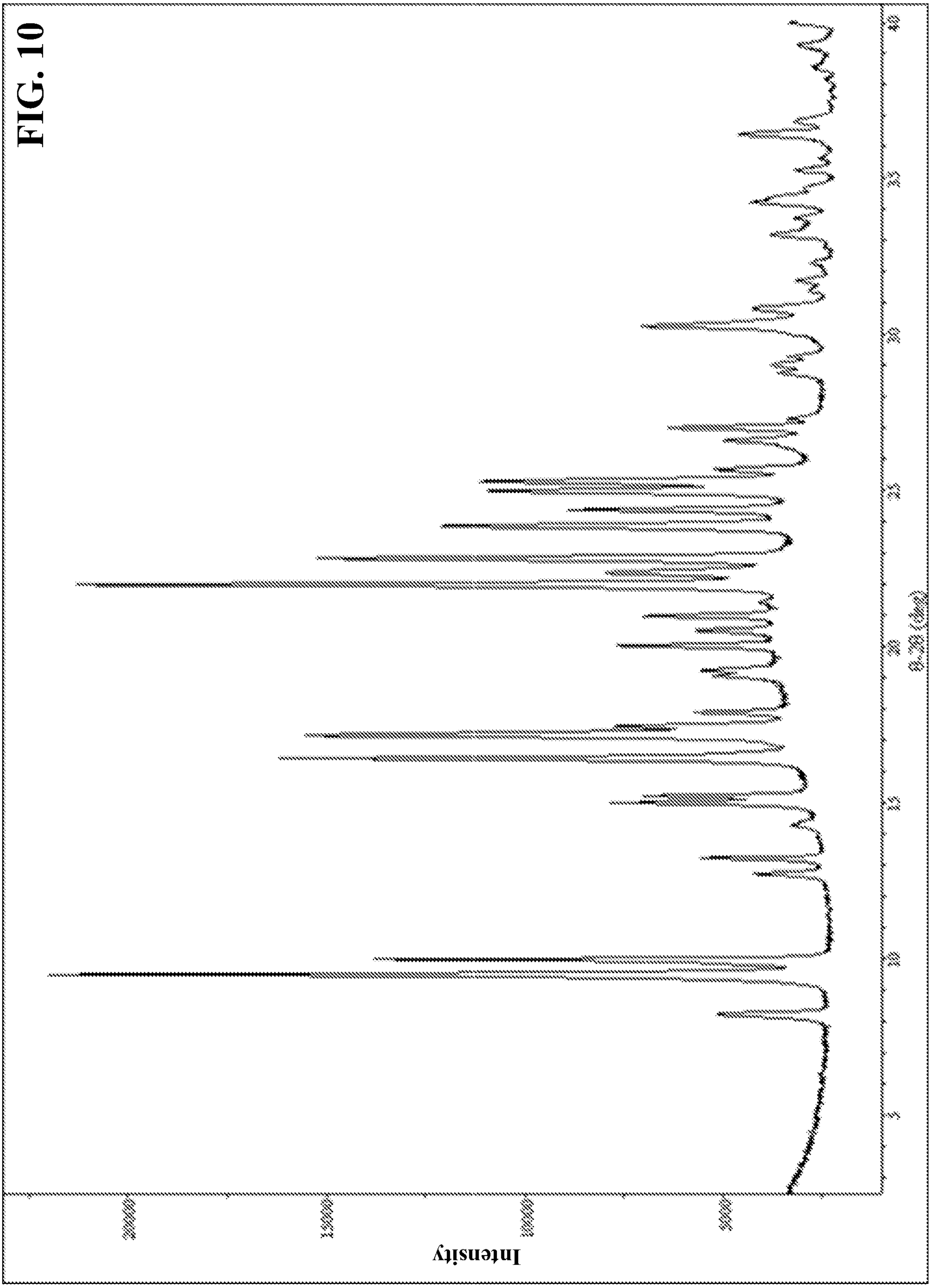


FIG. 10

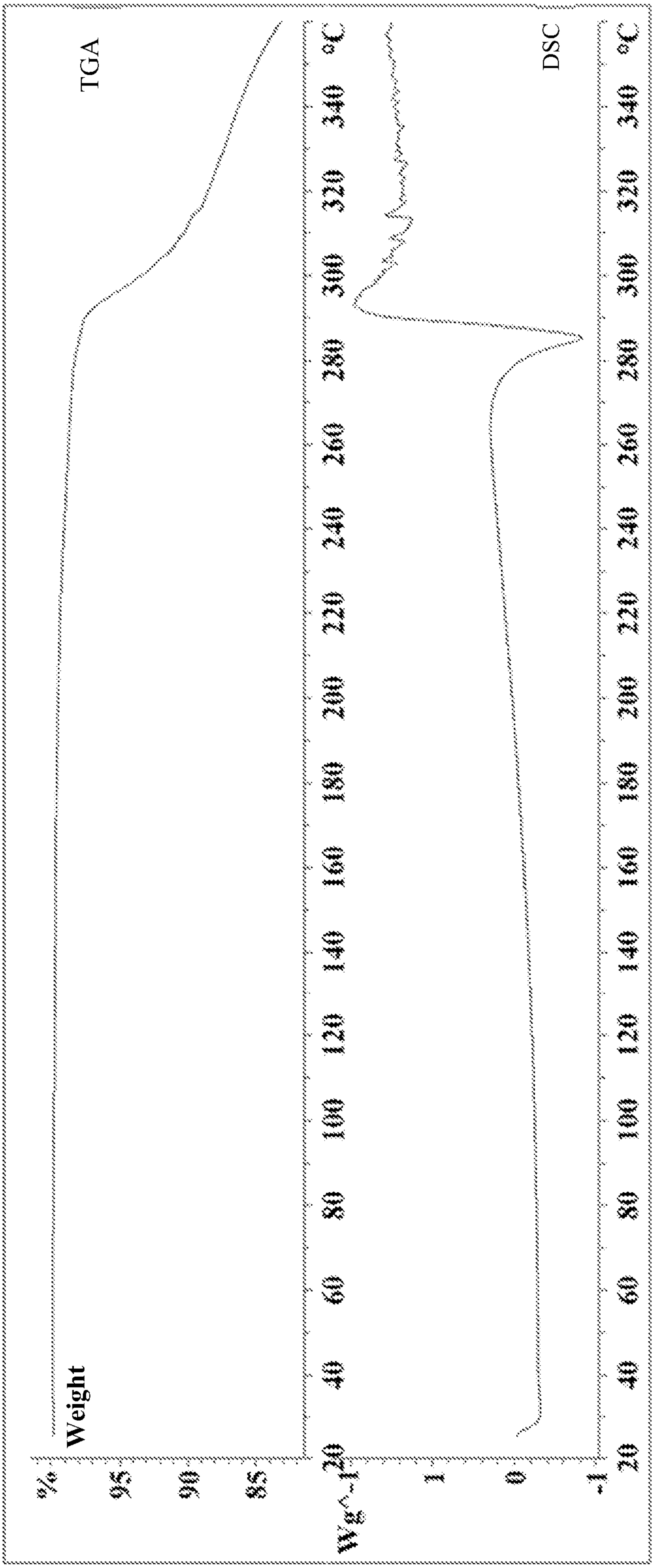
TGA
Weight
%
95
90
85
DSC
Wg^-1
1
0
-1
20
40
60
80
100
120
140
160
180
200
220
240
260
280
300
320
340
°C

FIG. 11

FIG. 12

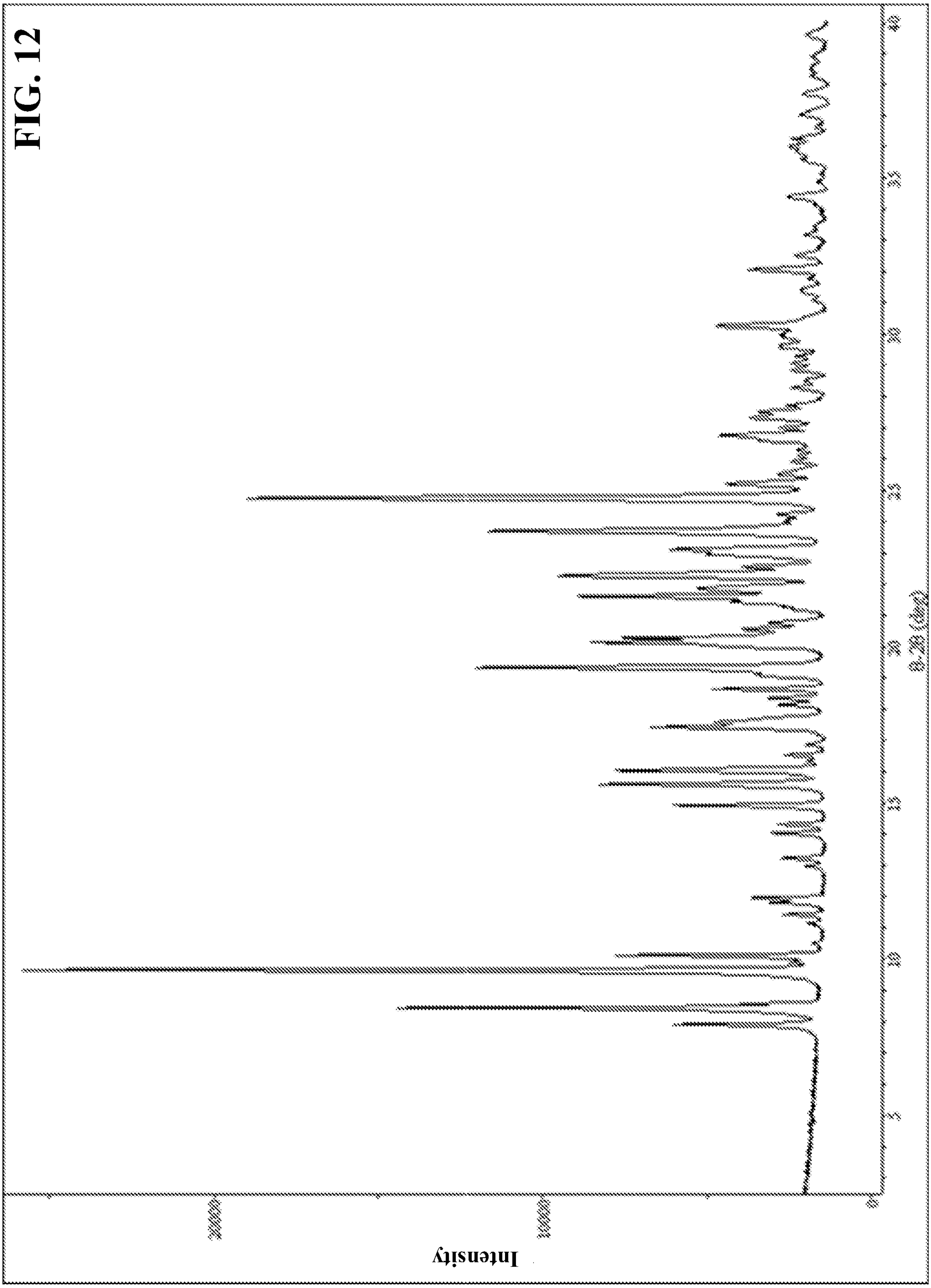

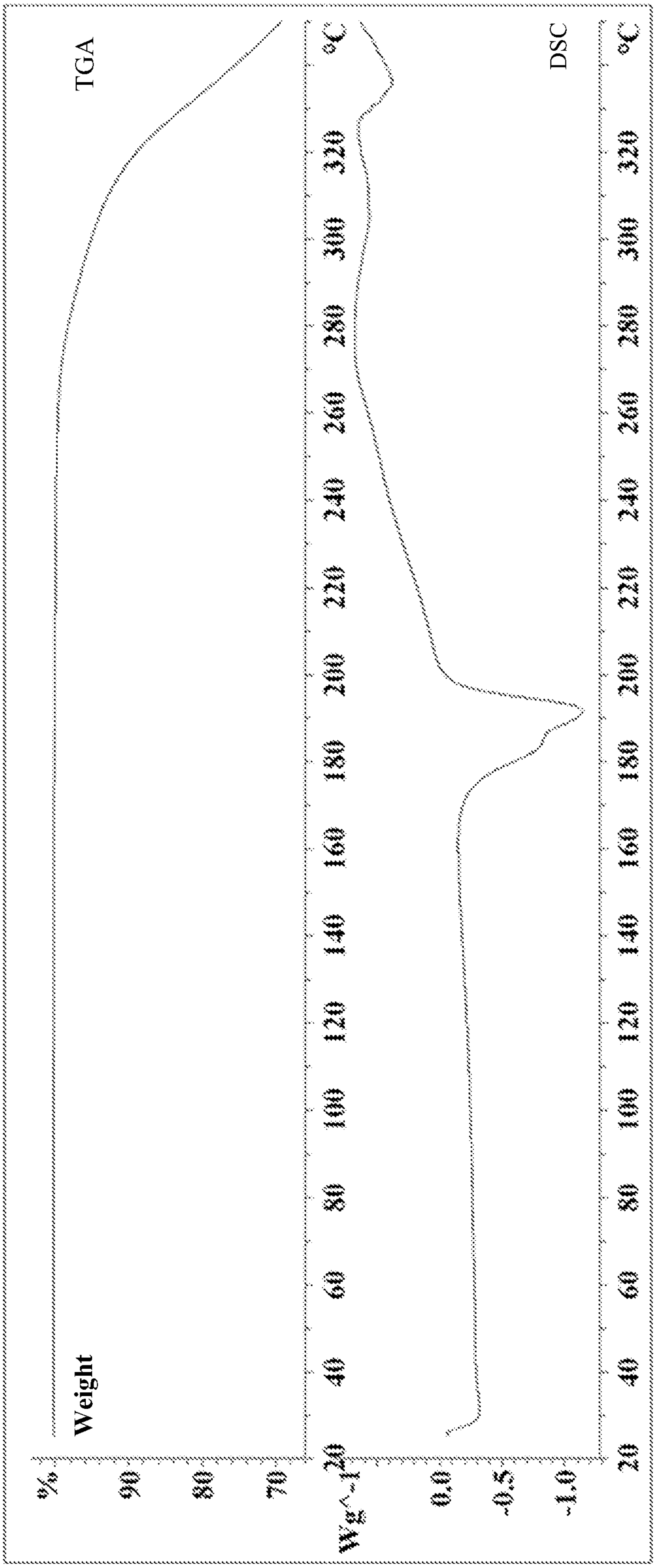
TGA
Weight
%
90
80
70
°C
20
40
60
80
100
120
140
160
180
200
220
240
260
280
300
320
DSC
Wg^-1
0.0
-0.5
-1.0
°C

FIG. 13

FIG. 14

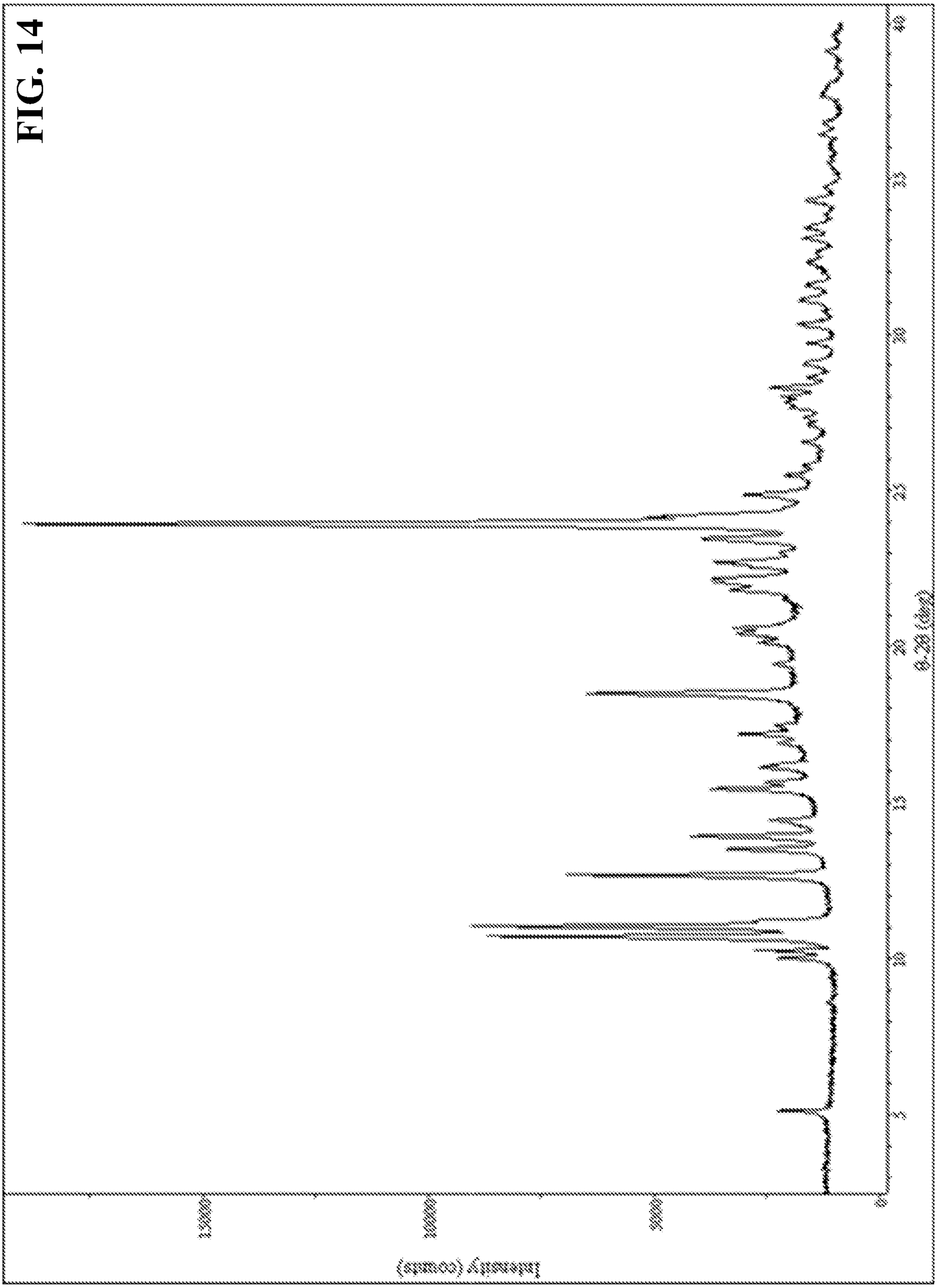

FIG. 15

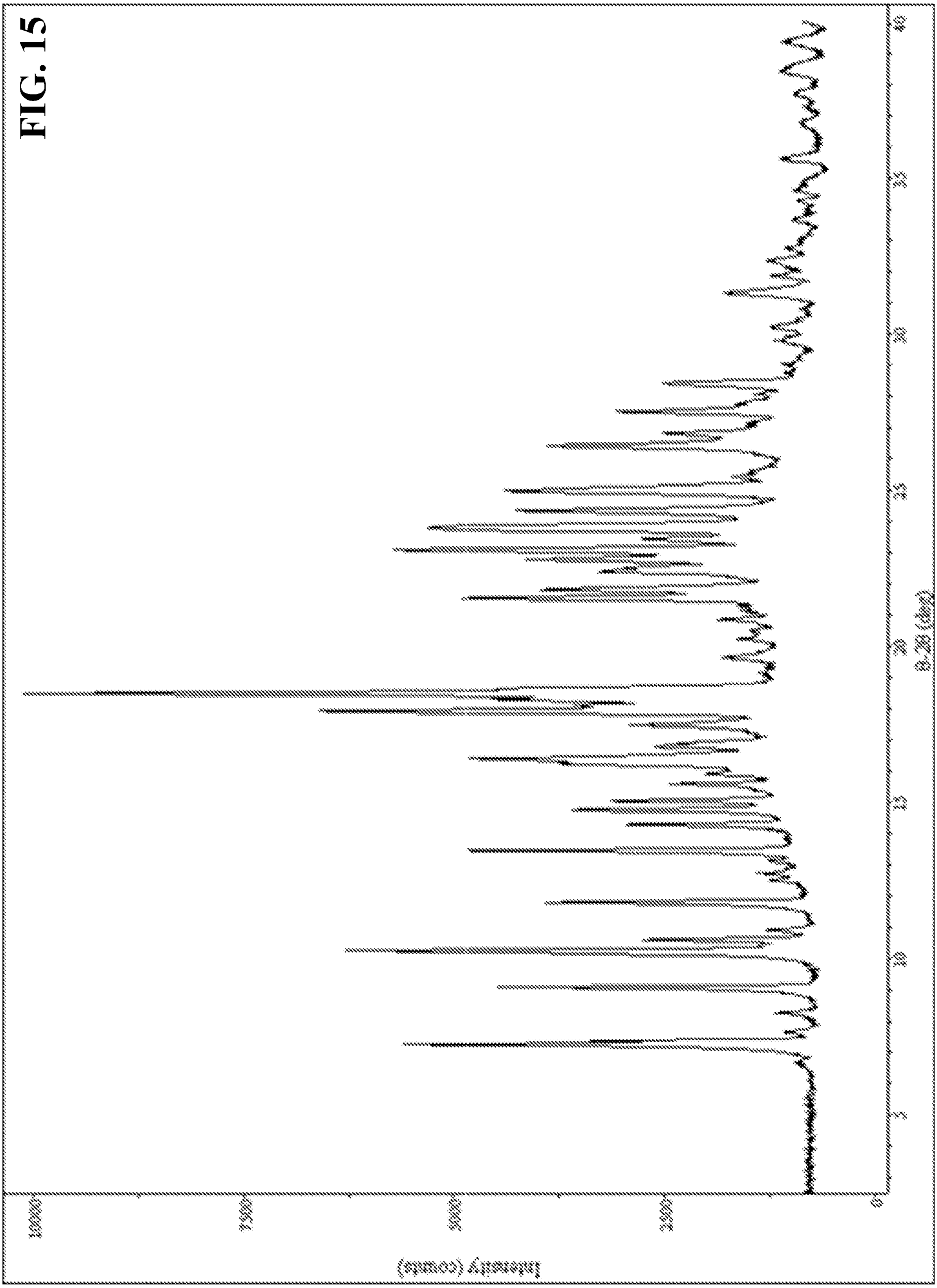
Intensity (counts)
10000
7500
5000
2500
0
5
10
15
20
25
30
35
40
θ-2θ (deg)

FIG. 16

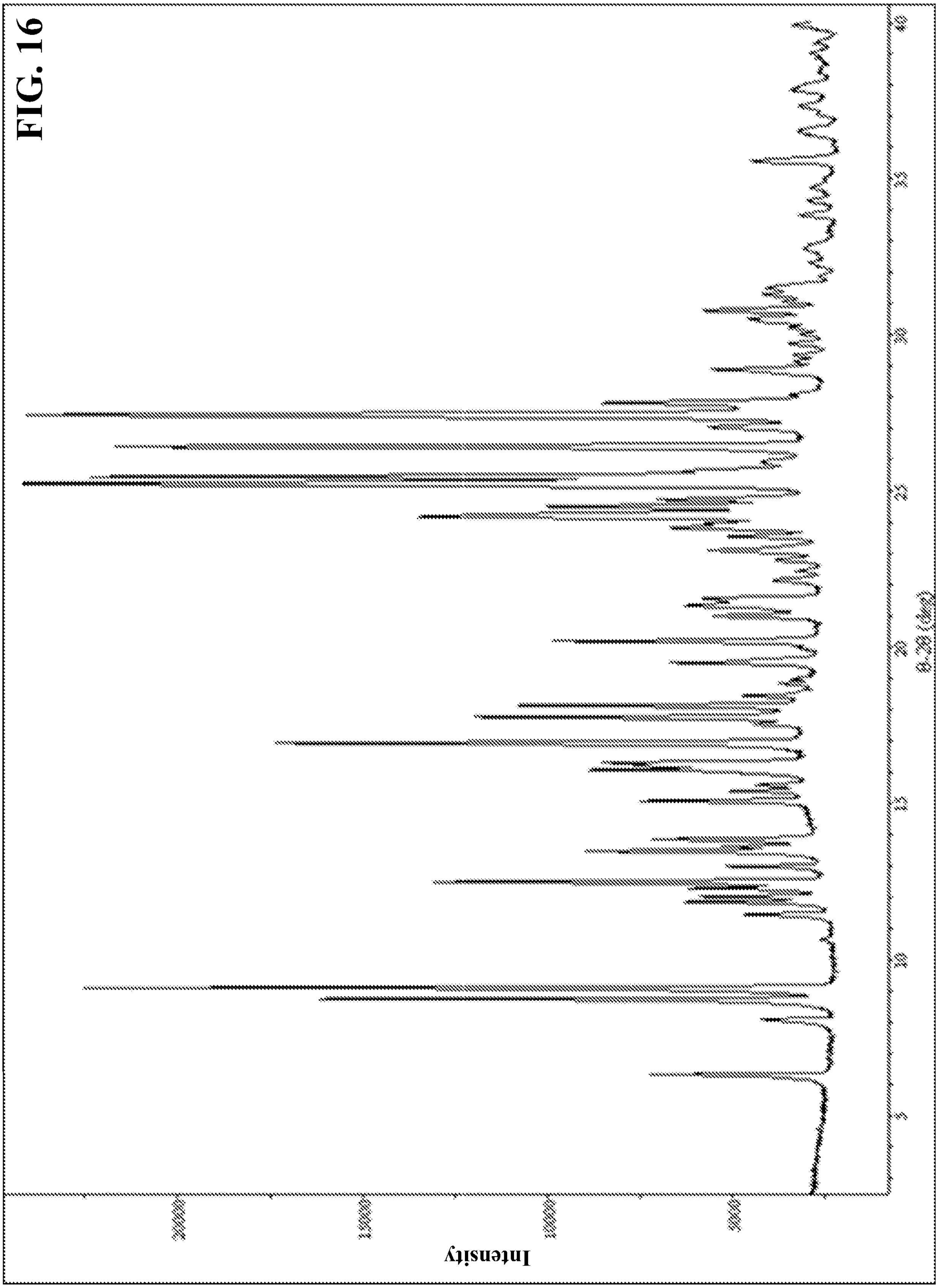

FIG. 17

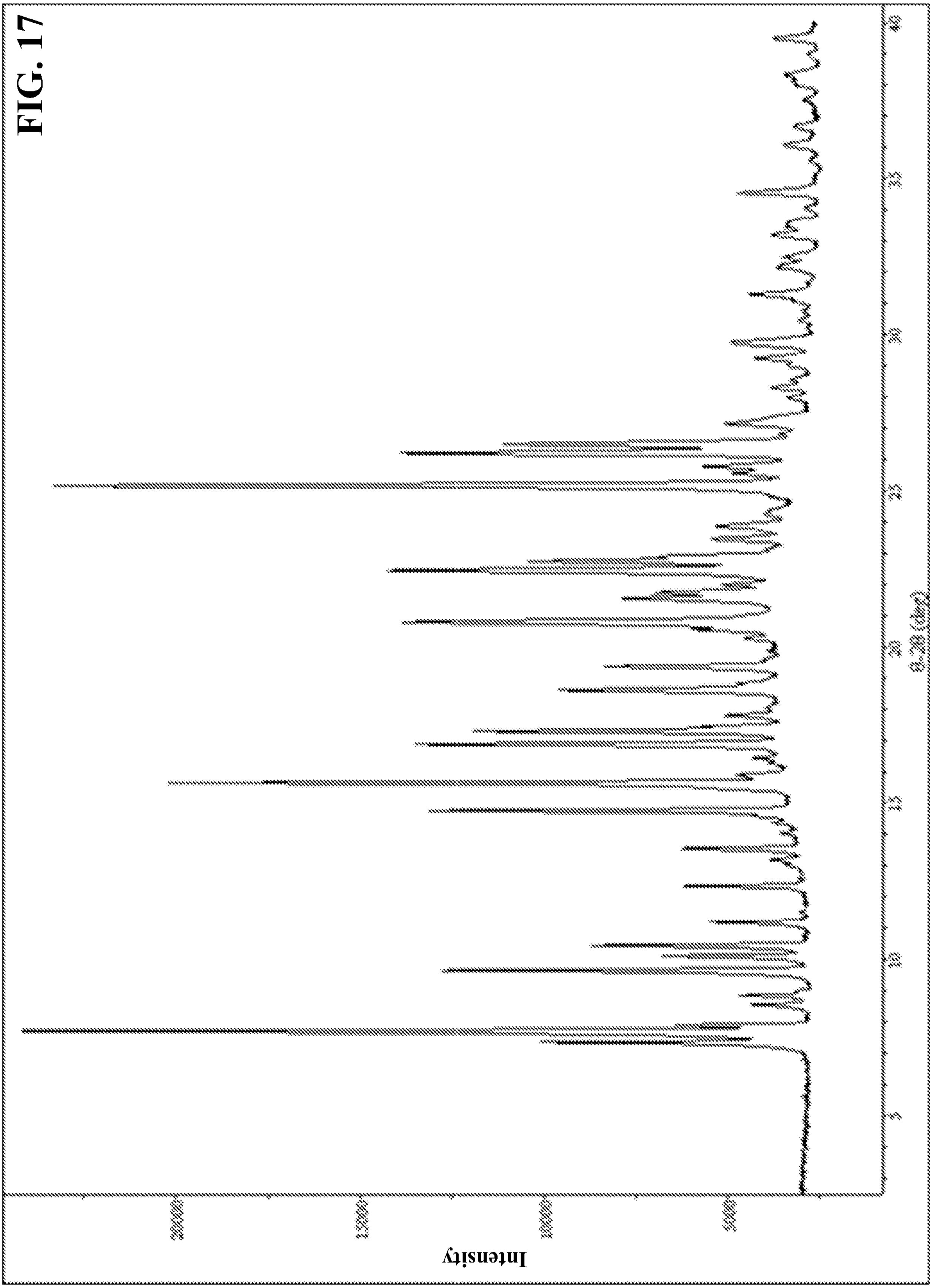

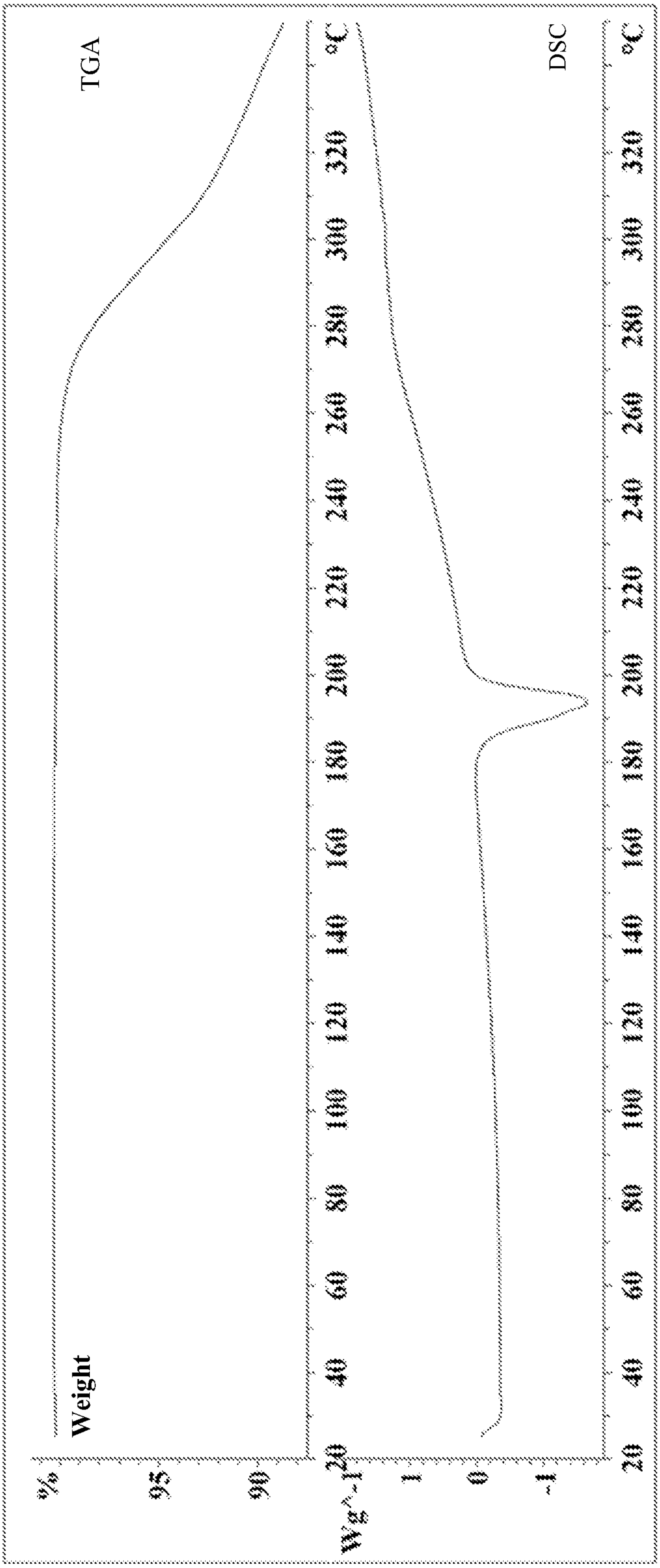
TGA
Weight
%
95
90
°C
DSC
Wg^-1
1
0
-1
20
40
60
80
100
120
140
160
180
200
220
240
260
280
300
320
°C

FIG. 18

FIG. 19

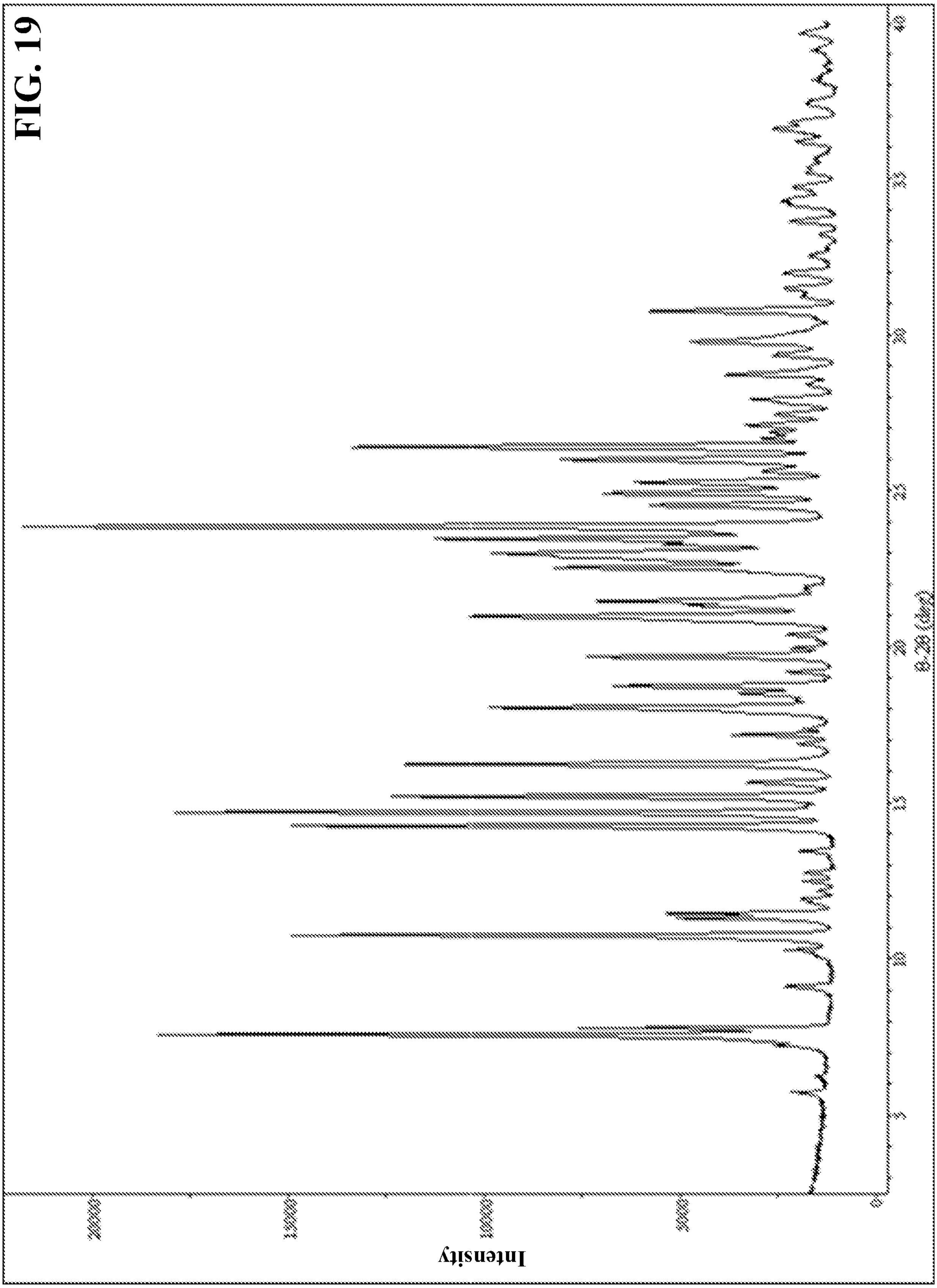

FIG. 20

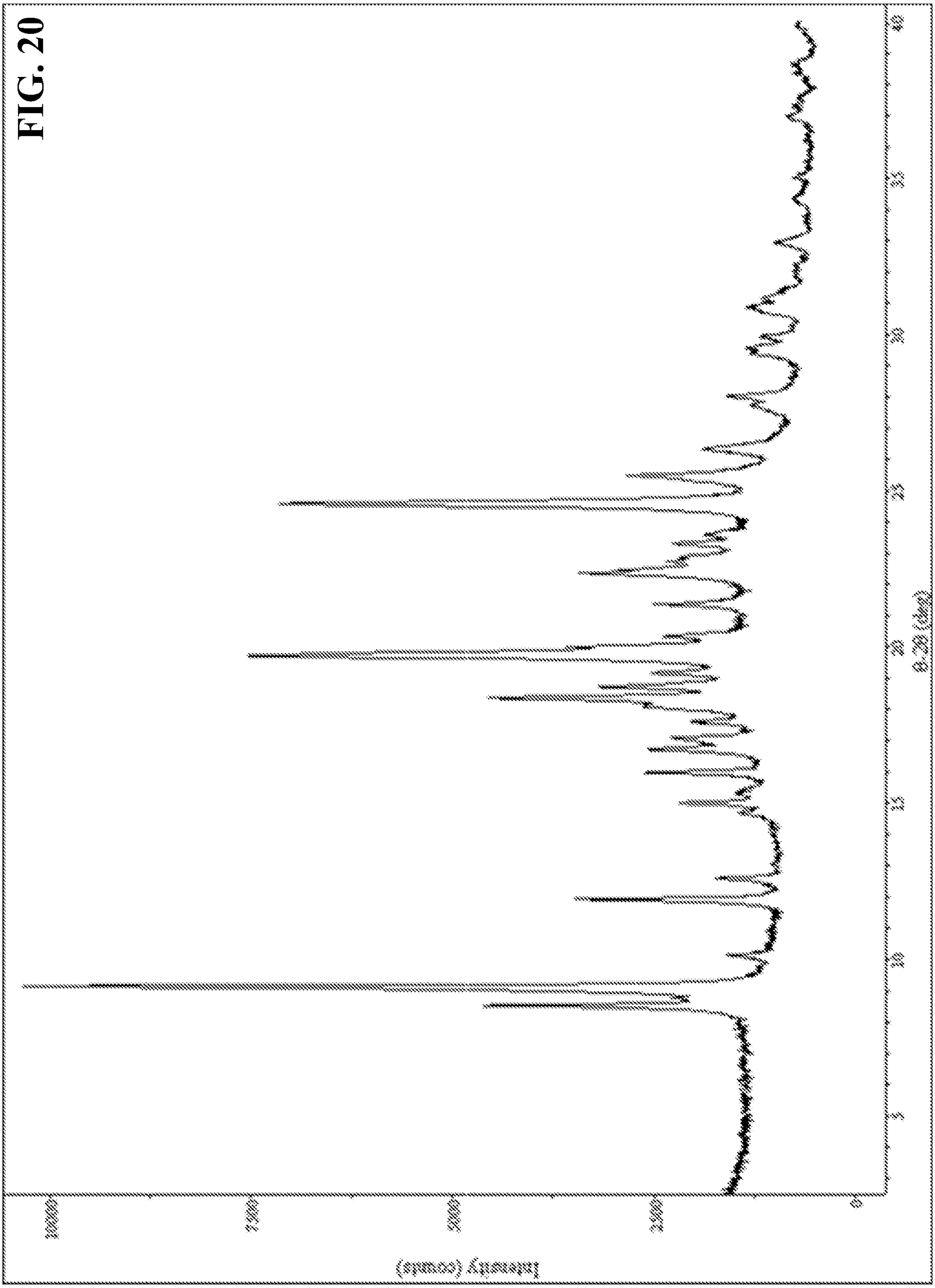

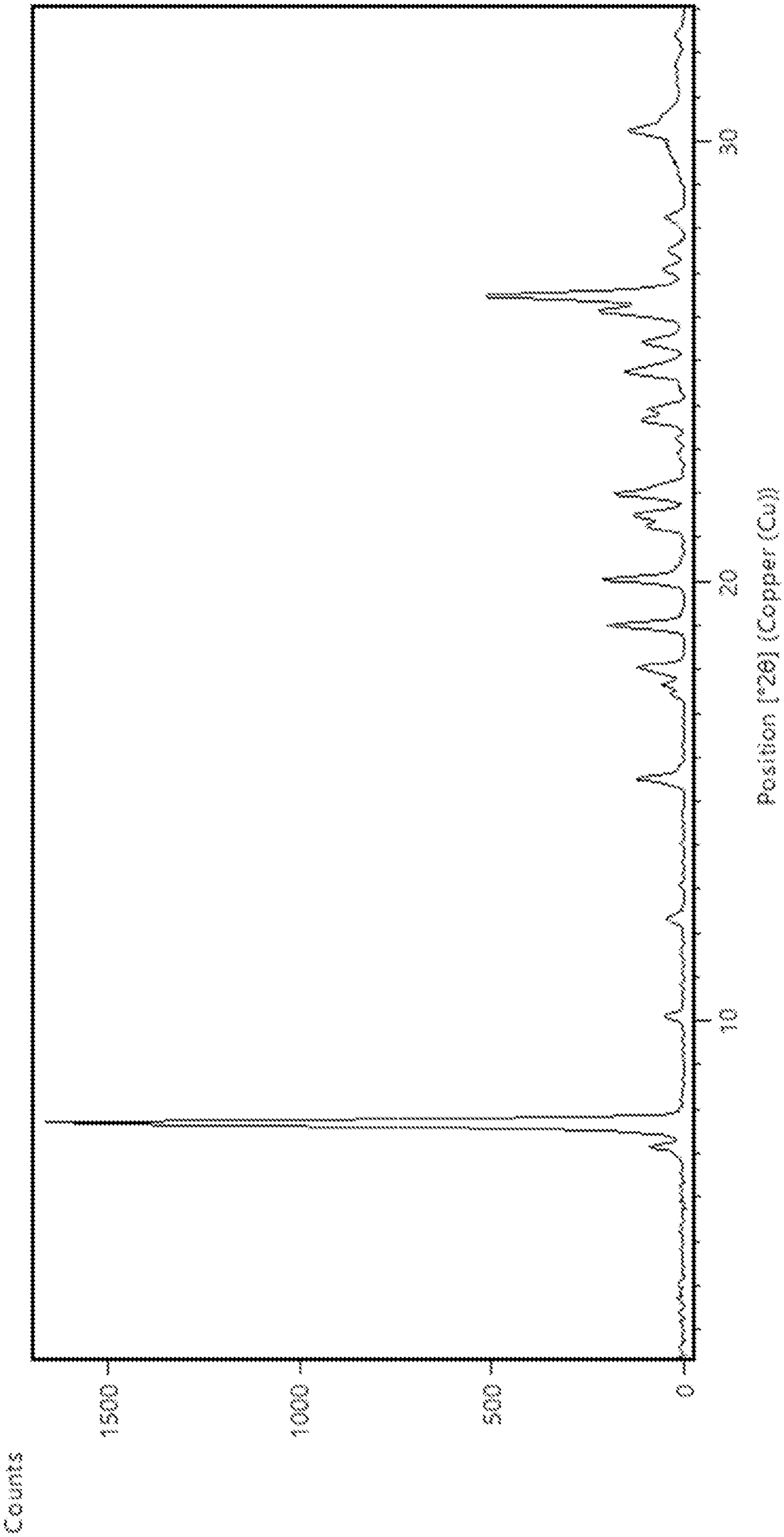


FIG. 21

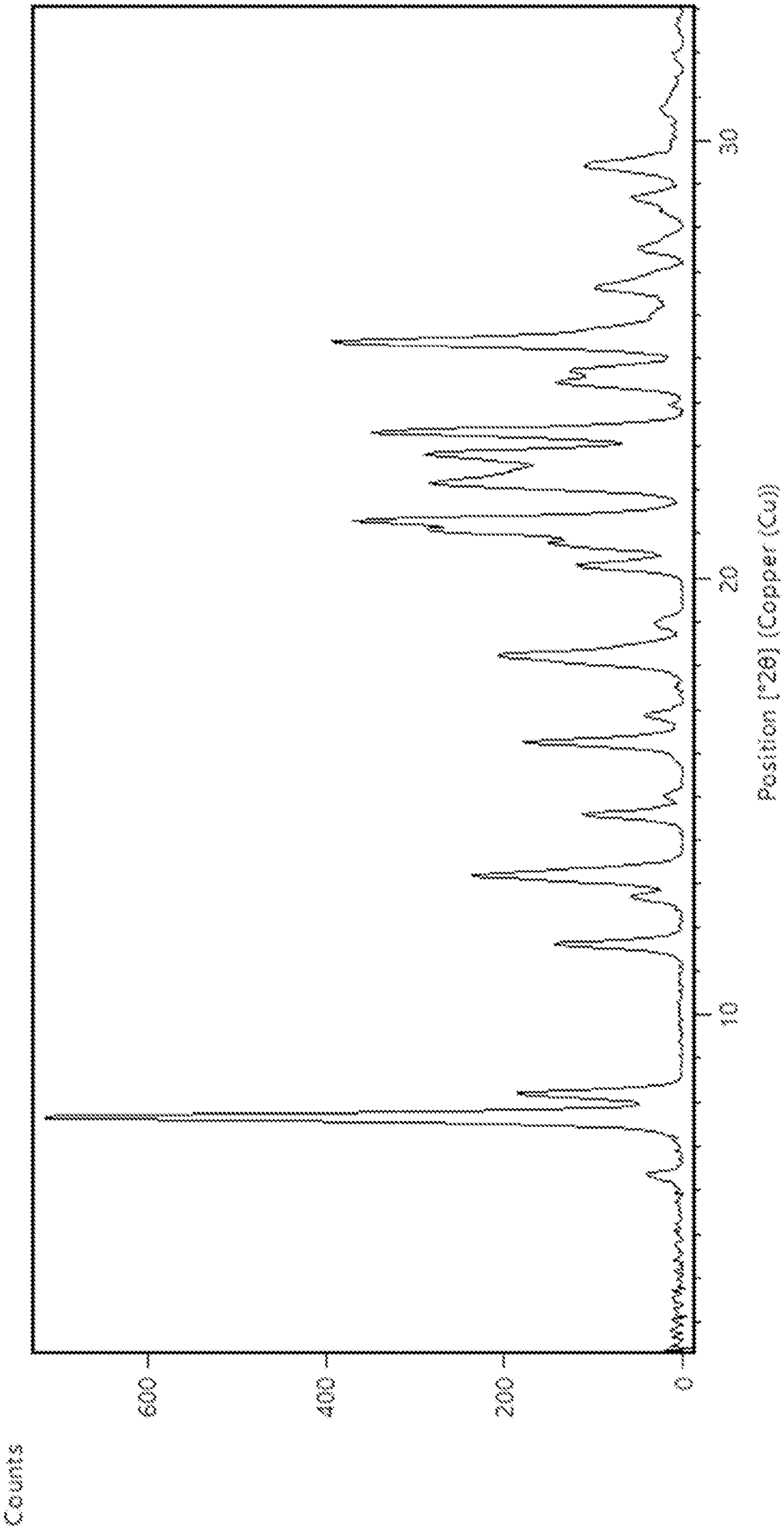


FIG. 22

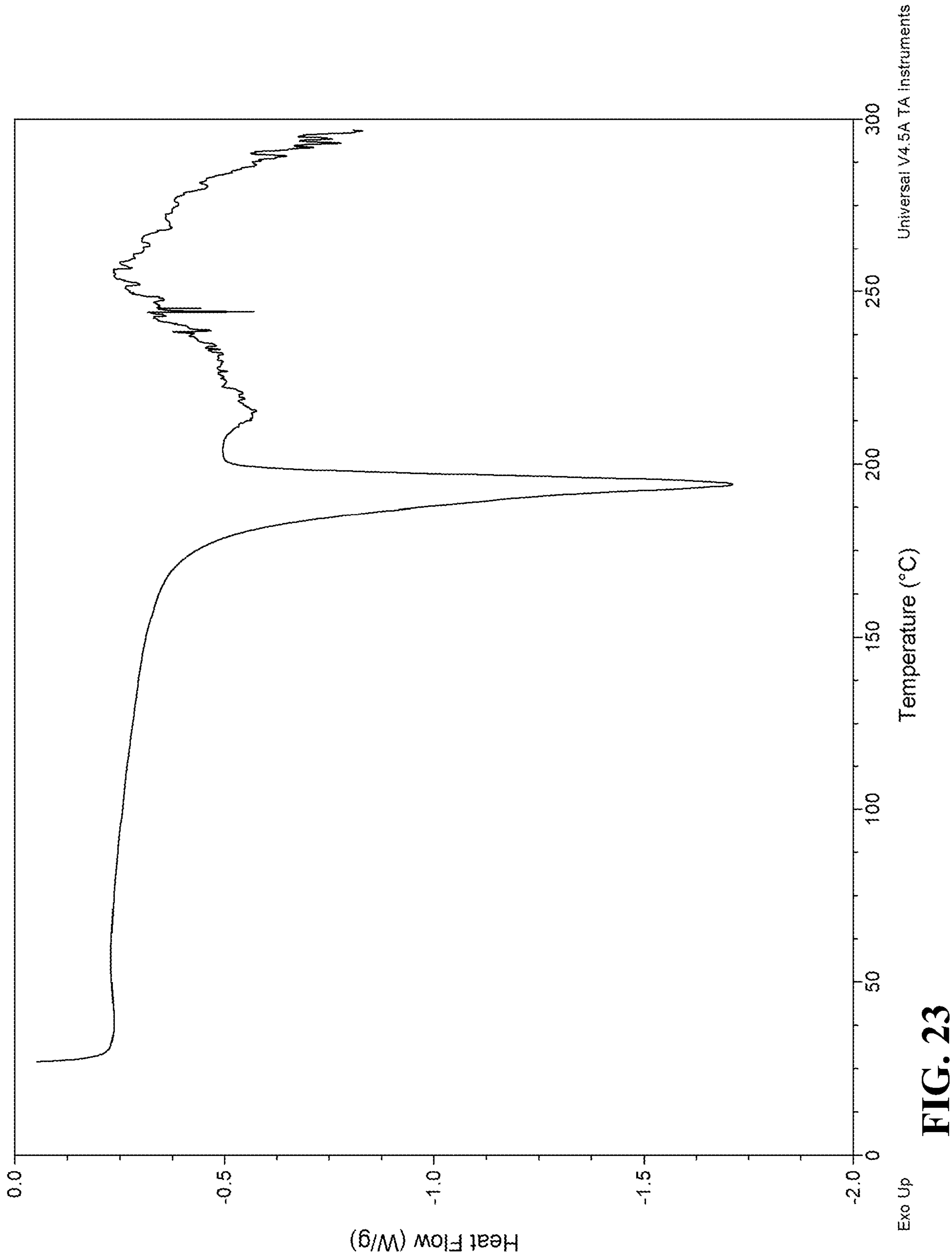
Heat Flow (W/g)
0.0
-0.5
-1.0
-1.5
-2.0
Exo Up
0
50
100
150
200
250
300
Temperature (°C)
Universal V4.5A TA Instruments

FIG. 23

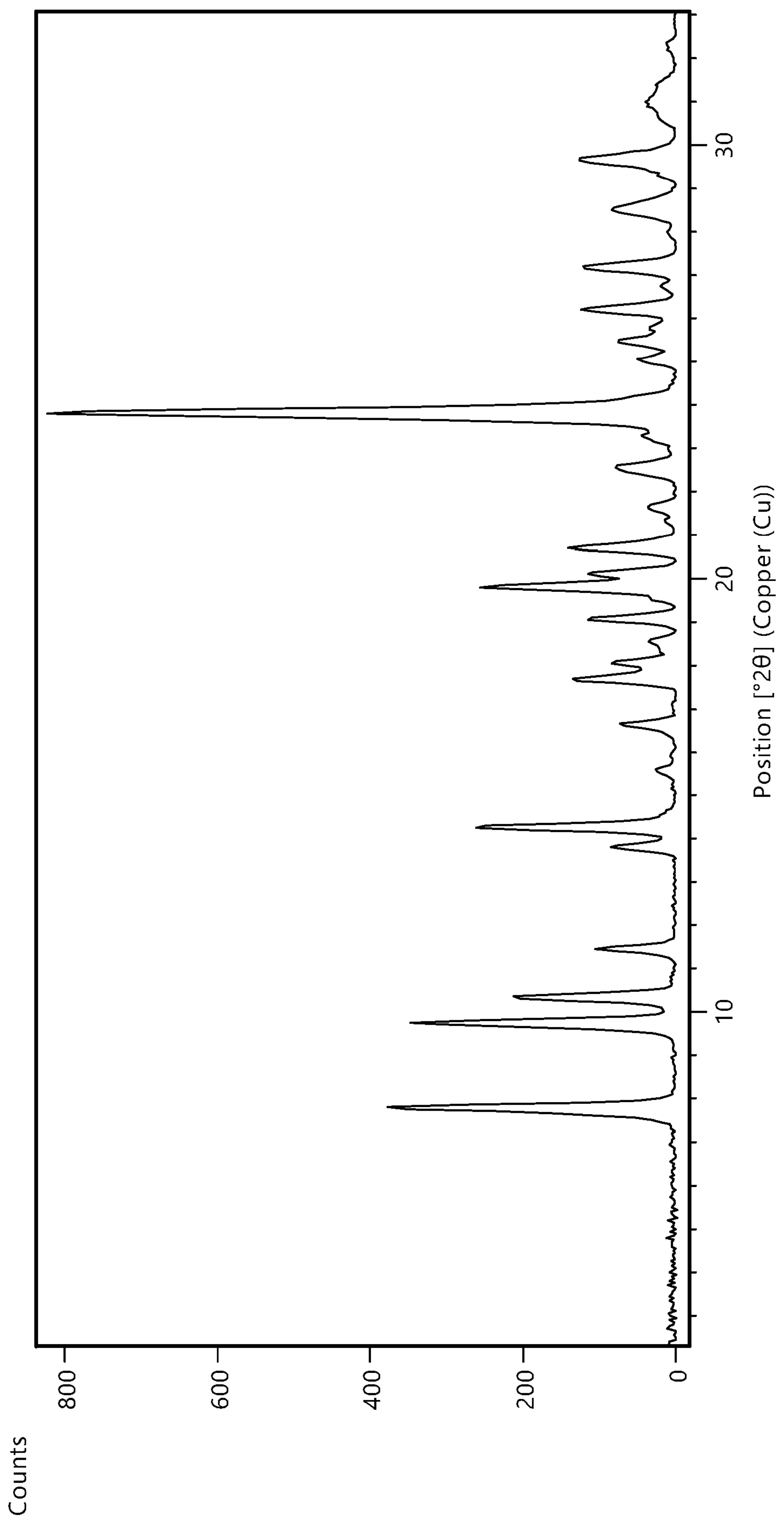


FIG. 24

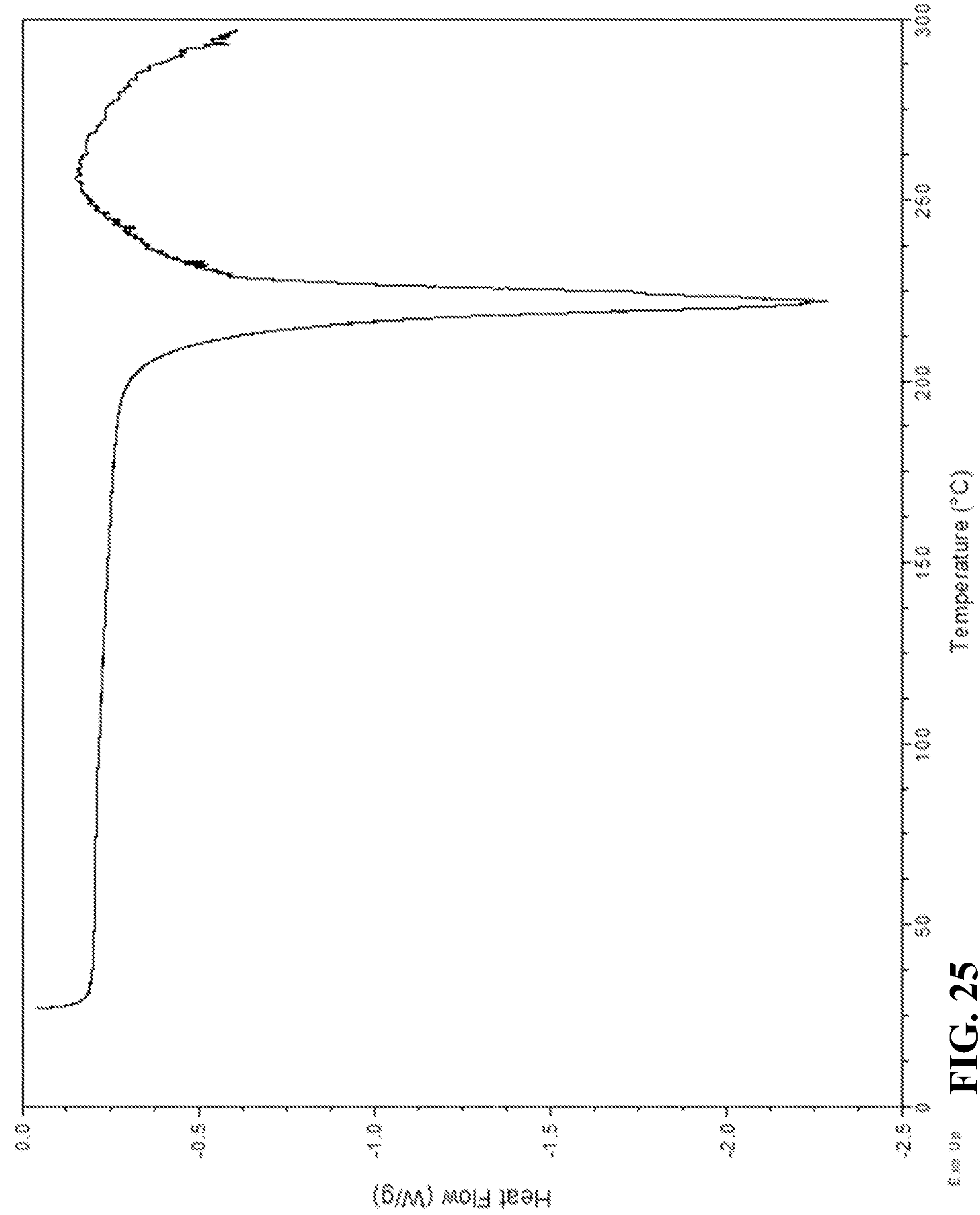
Heat Flow (W/g)
0.0
-0.5
-1.0
-1.5
-2.0
-2.5
Temperature (°C)
0
50
100
150
200
250
300

FIG. 25

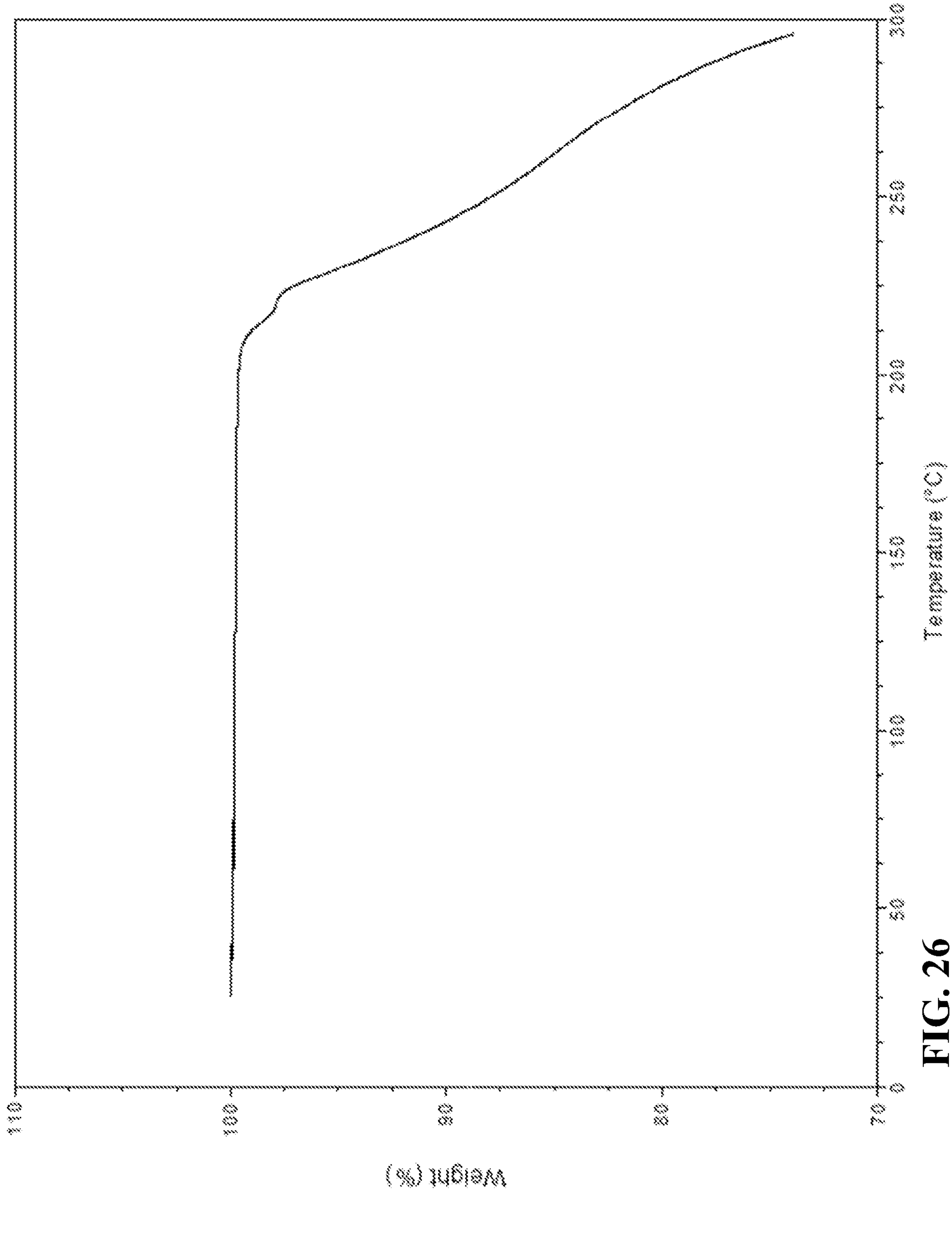


FIG. 26

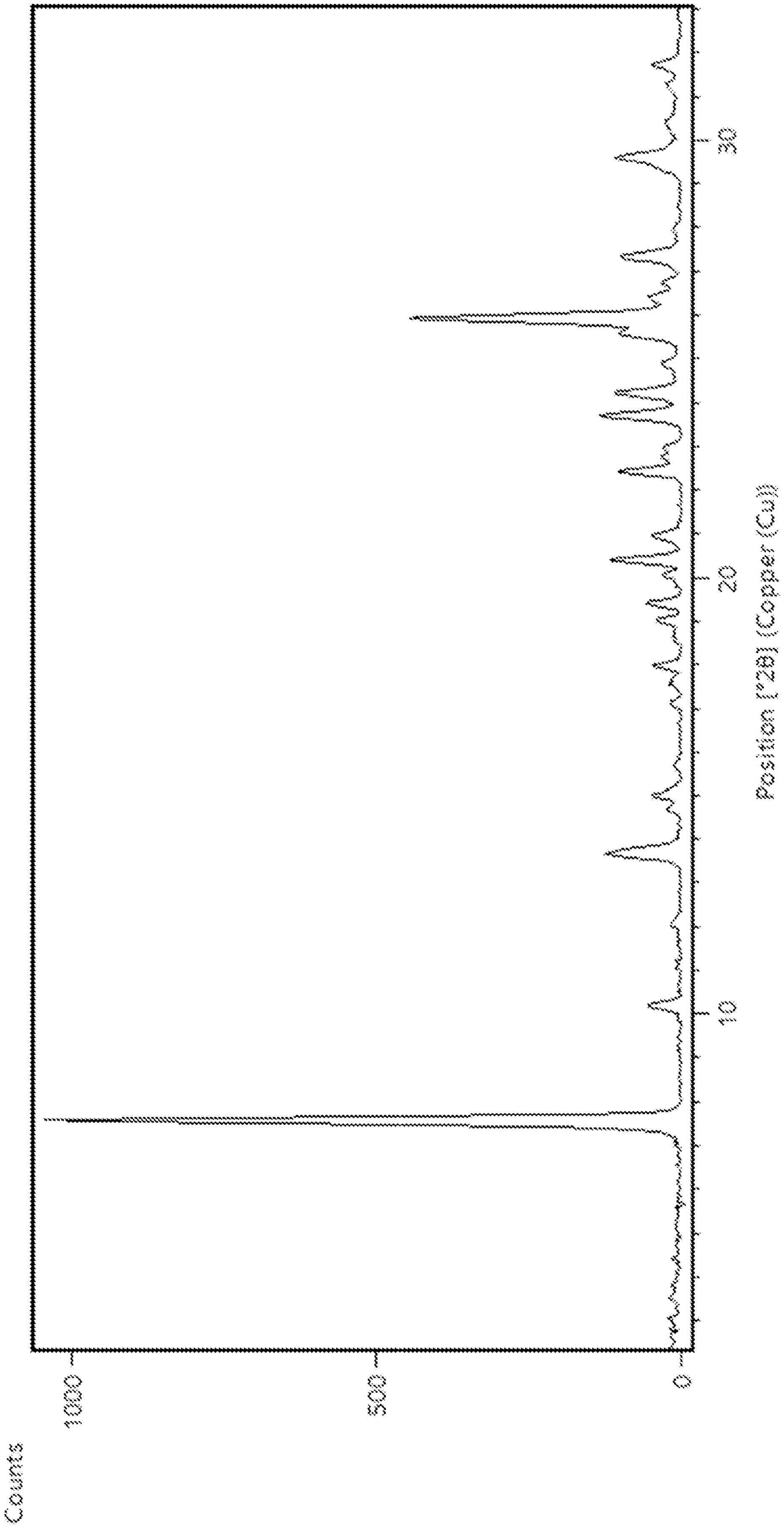


FIG. 27

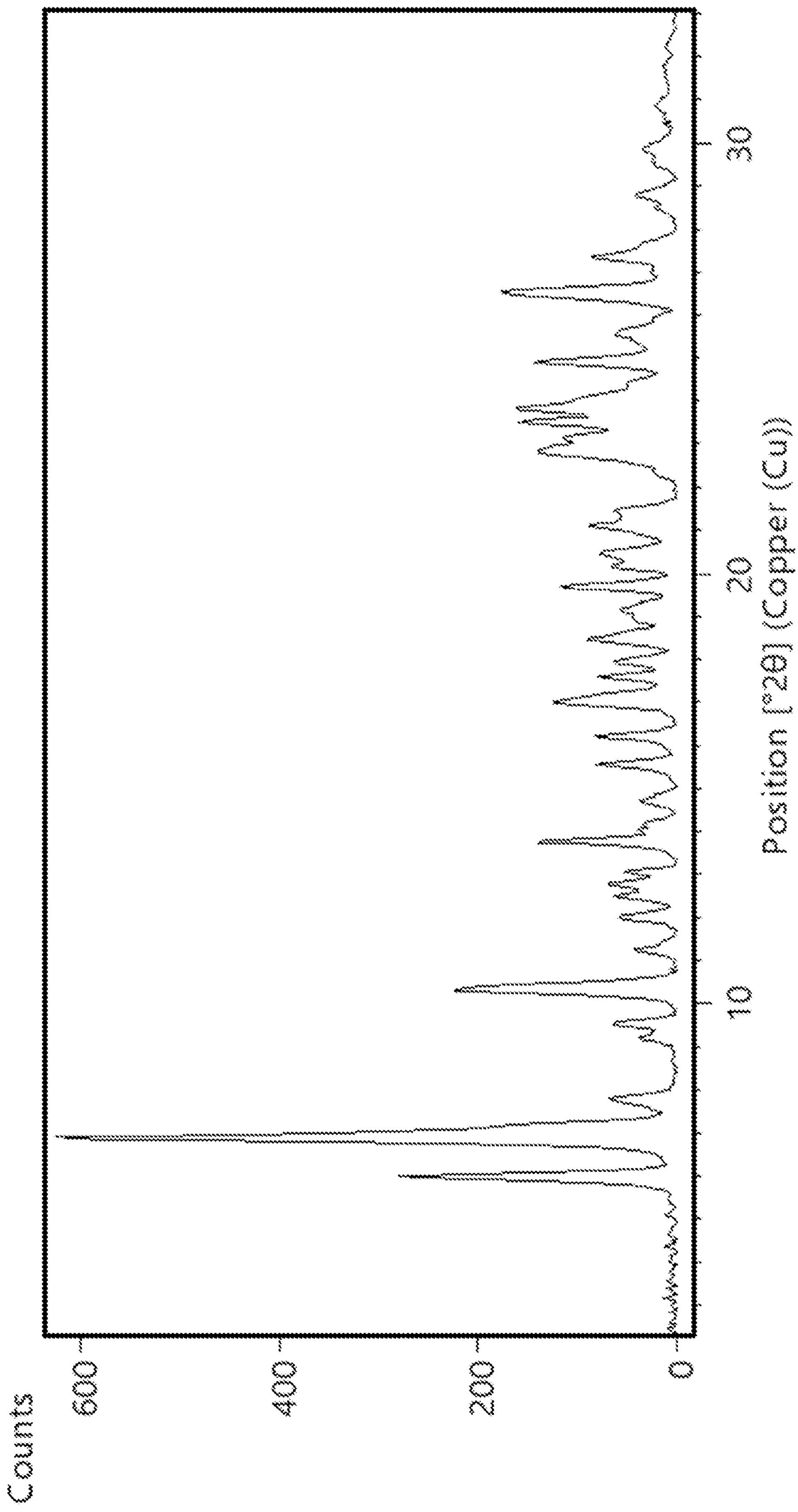


FIG. 28

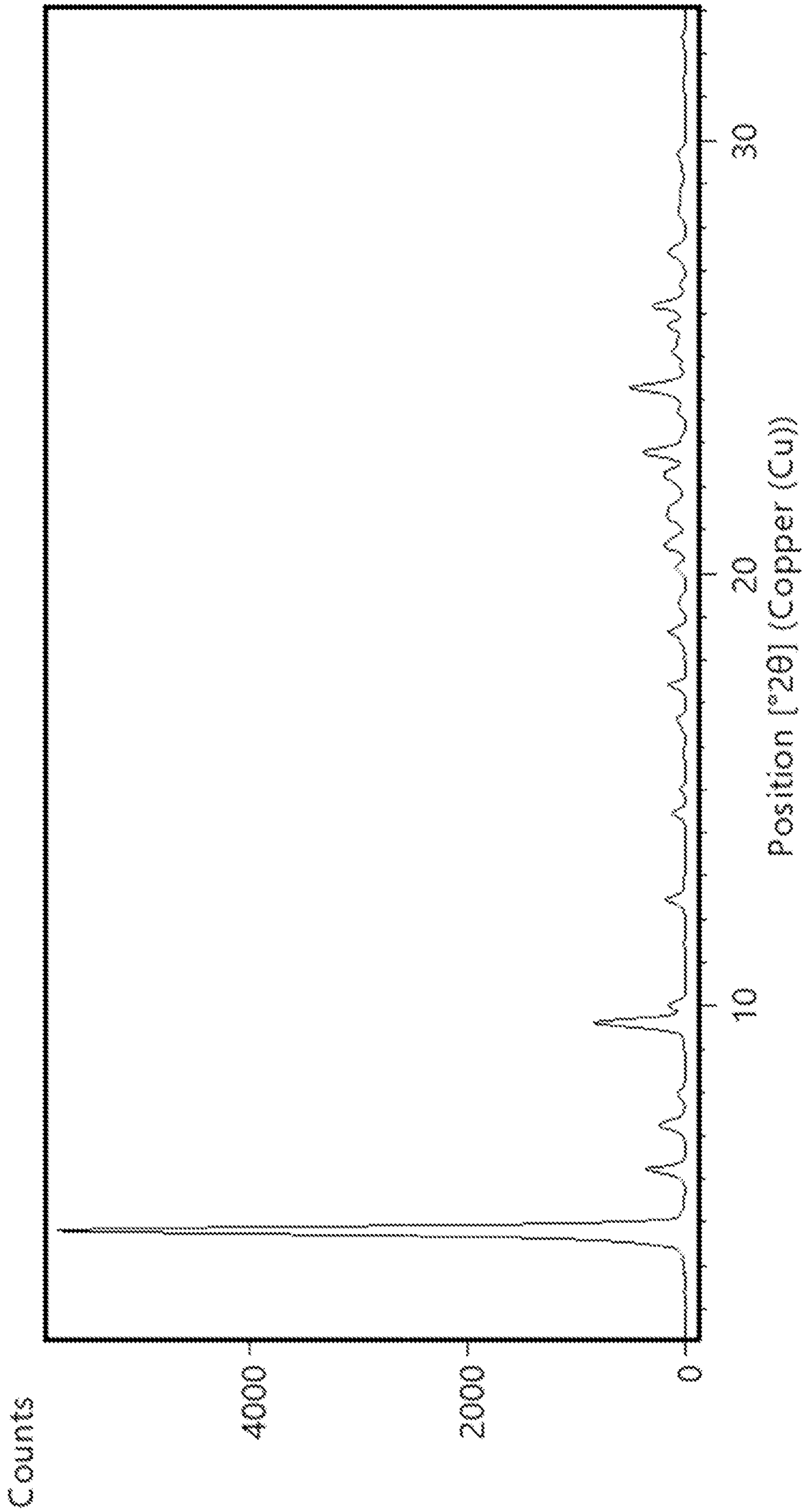


FIG. 29

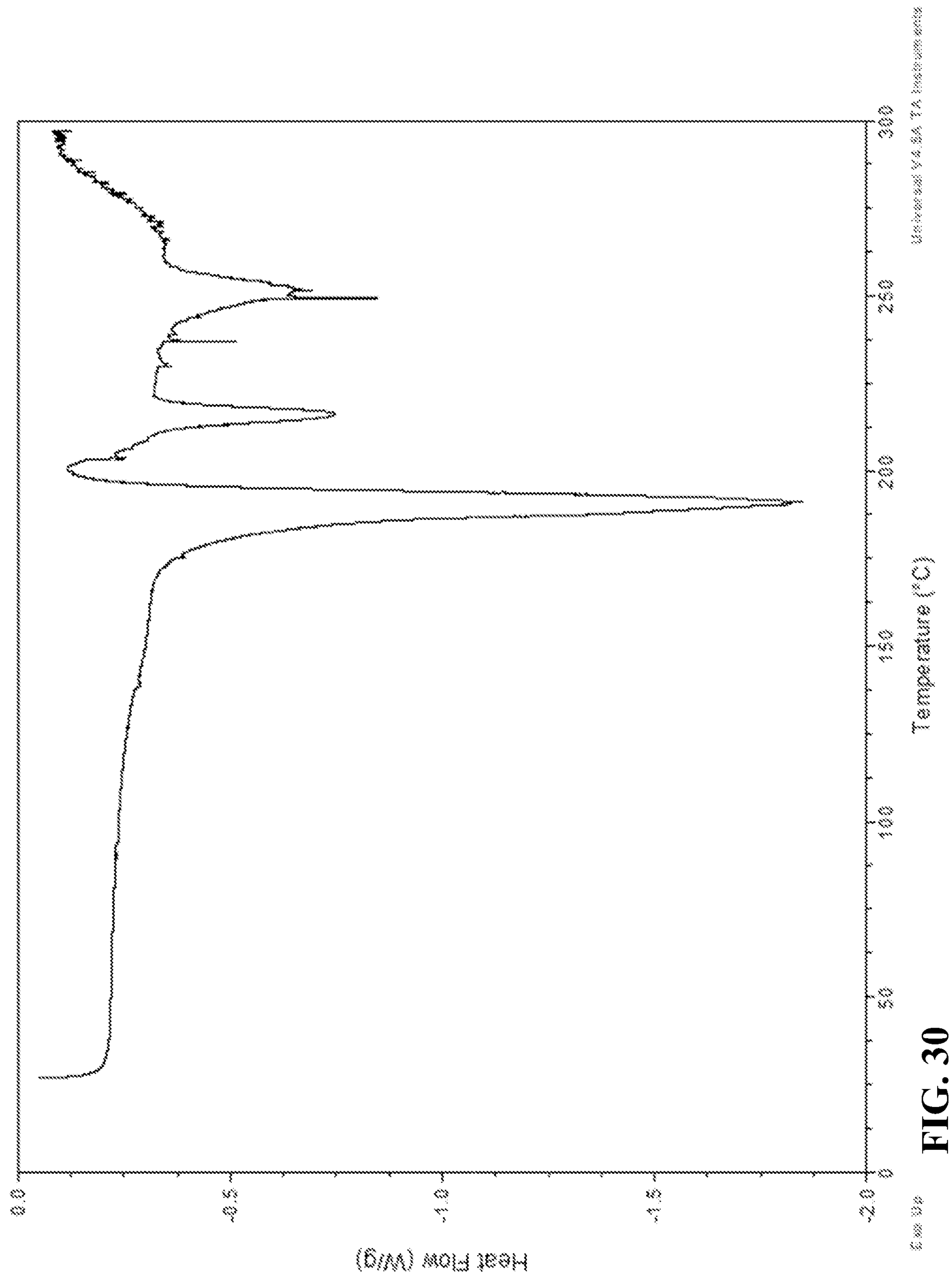
Heat Flow (W/g)
0.0
-0.5
-1.0
-1.5
-2.0
Temperature (°C)
0
50
100
150
200
250
300
Exo Up
Universal V4.5A TA Instruments

FIG. 30

FIG. 31

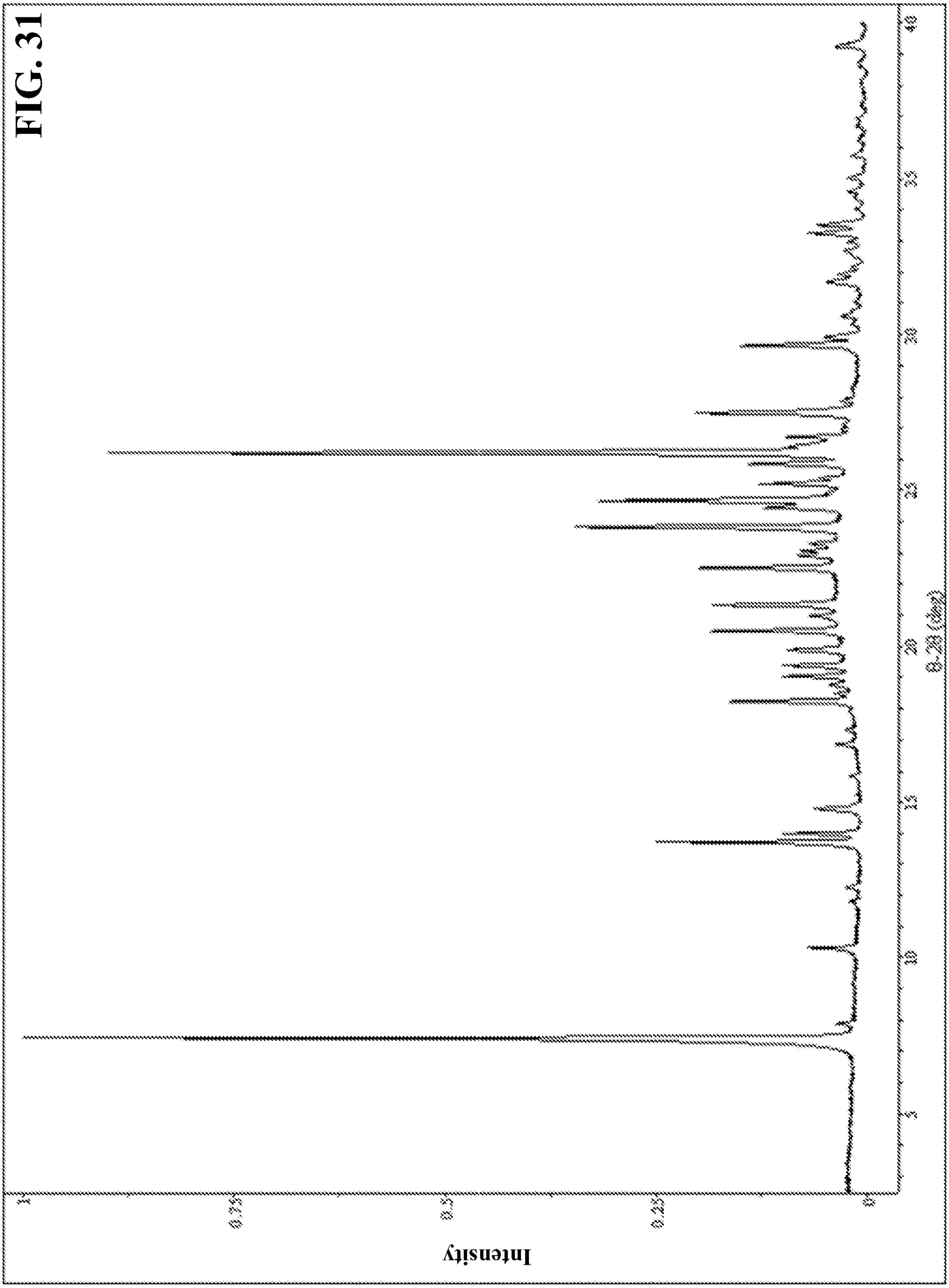

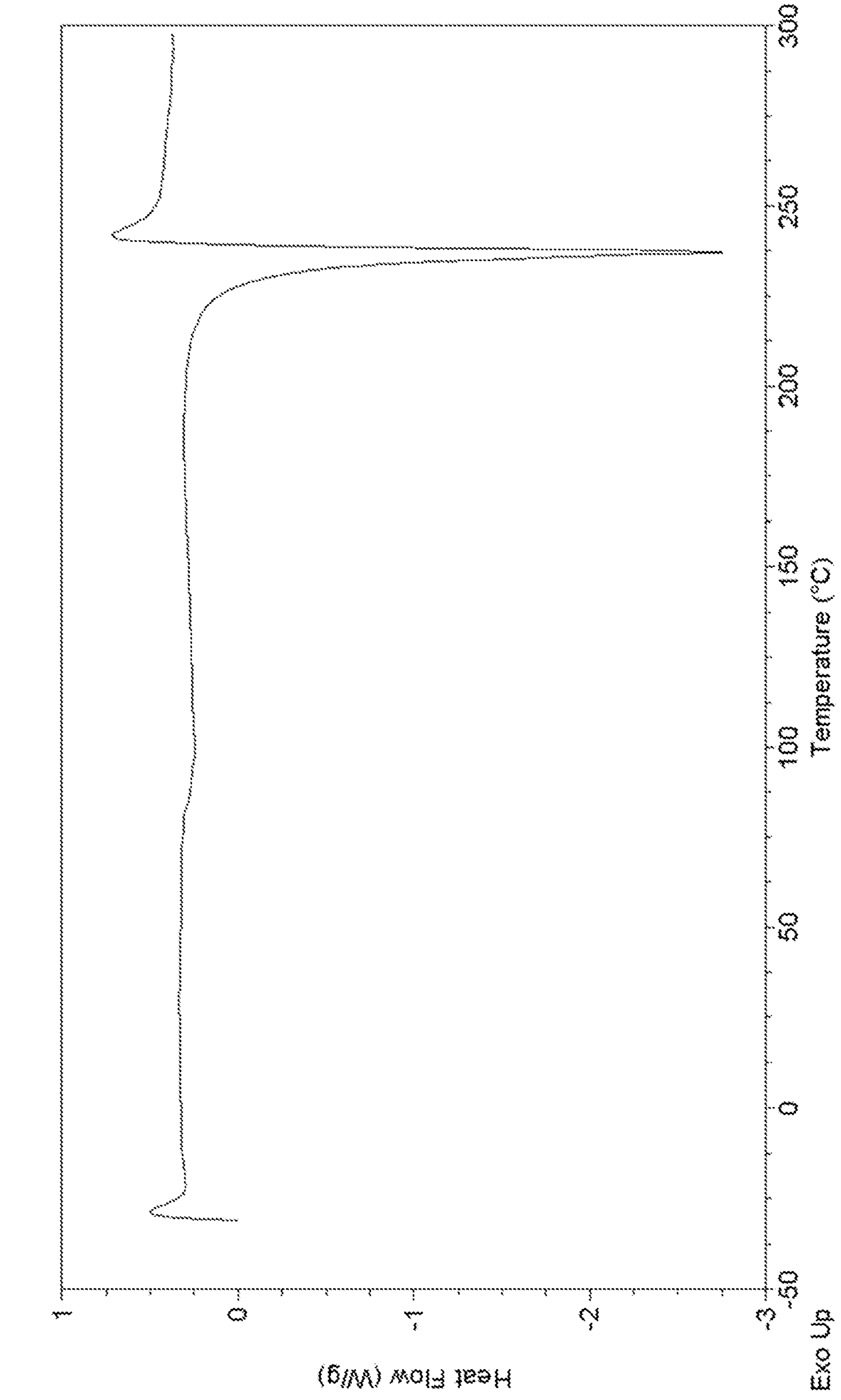
Heat Flow (W/g)
1
0
-1
-2
-3
Exo Up
-50
0
50
100
150
200
250
300
Temperature (°C)

FIG. 32

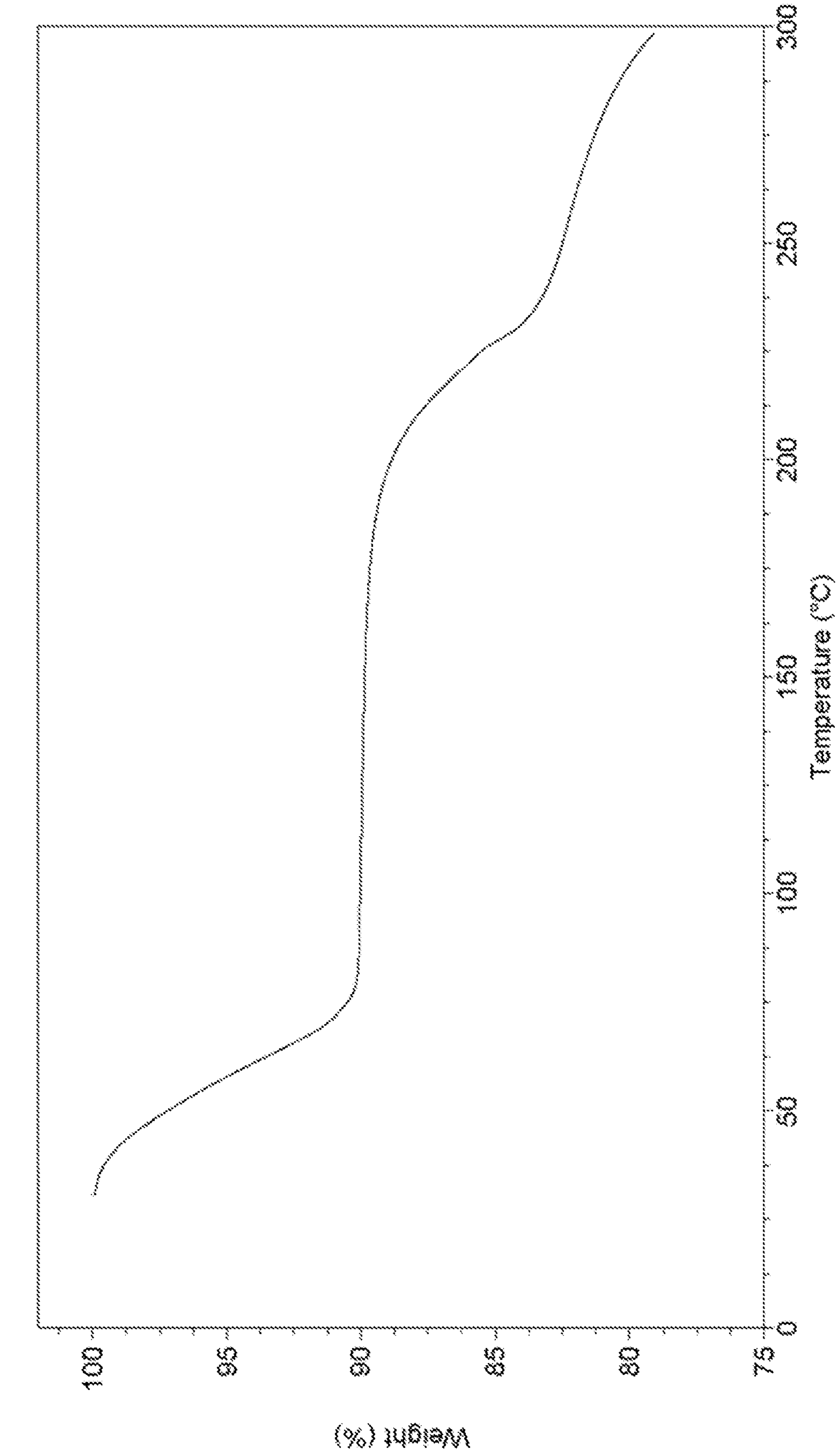


FIG. 33

FIG. 34

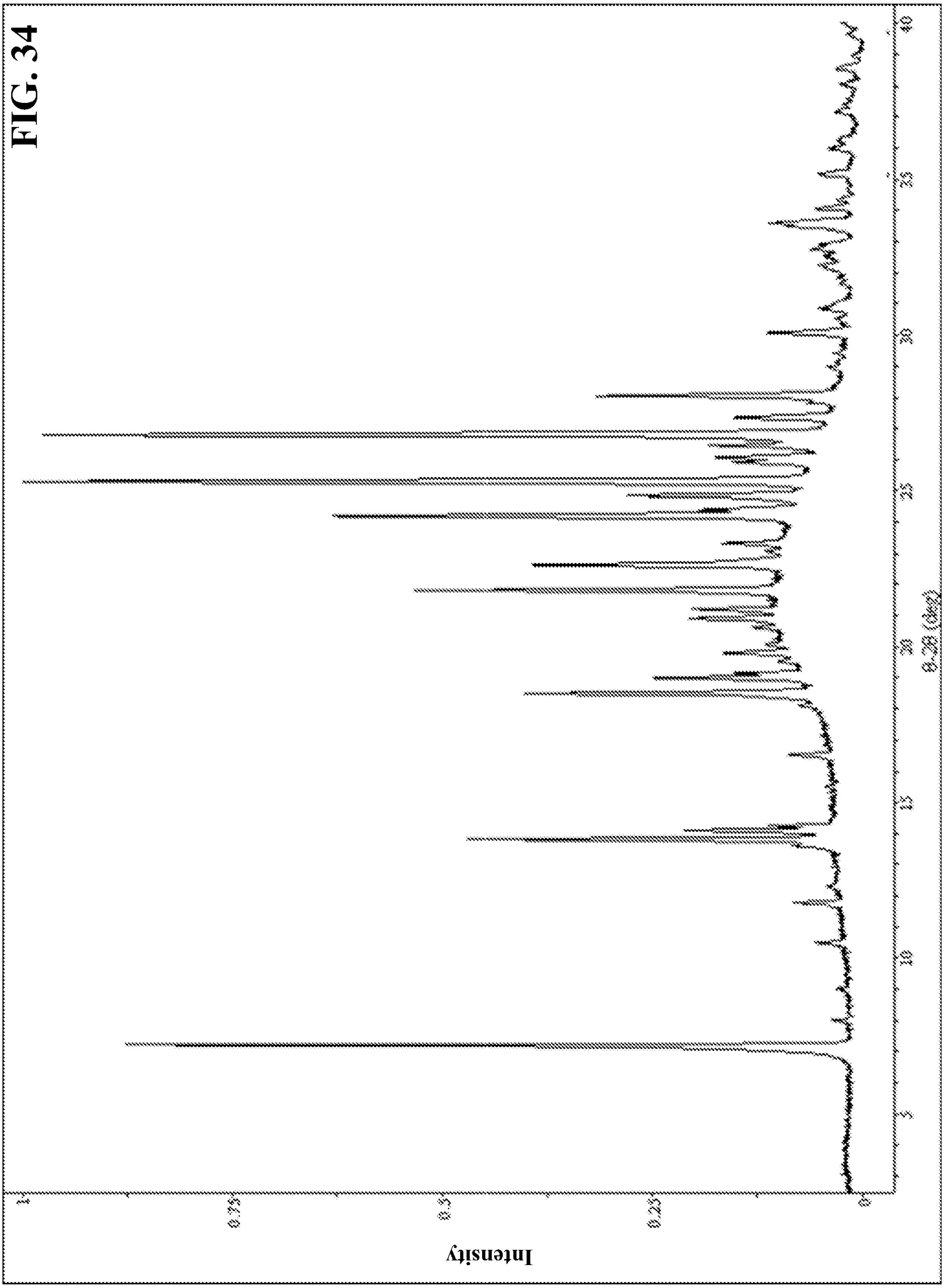

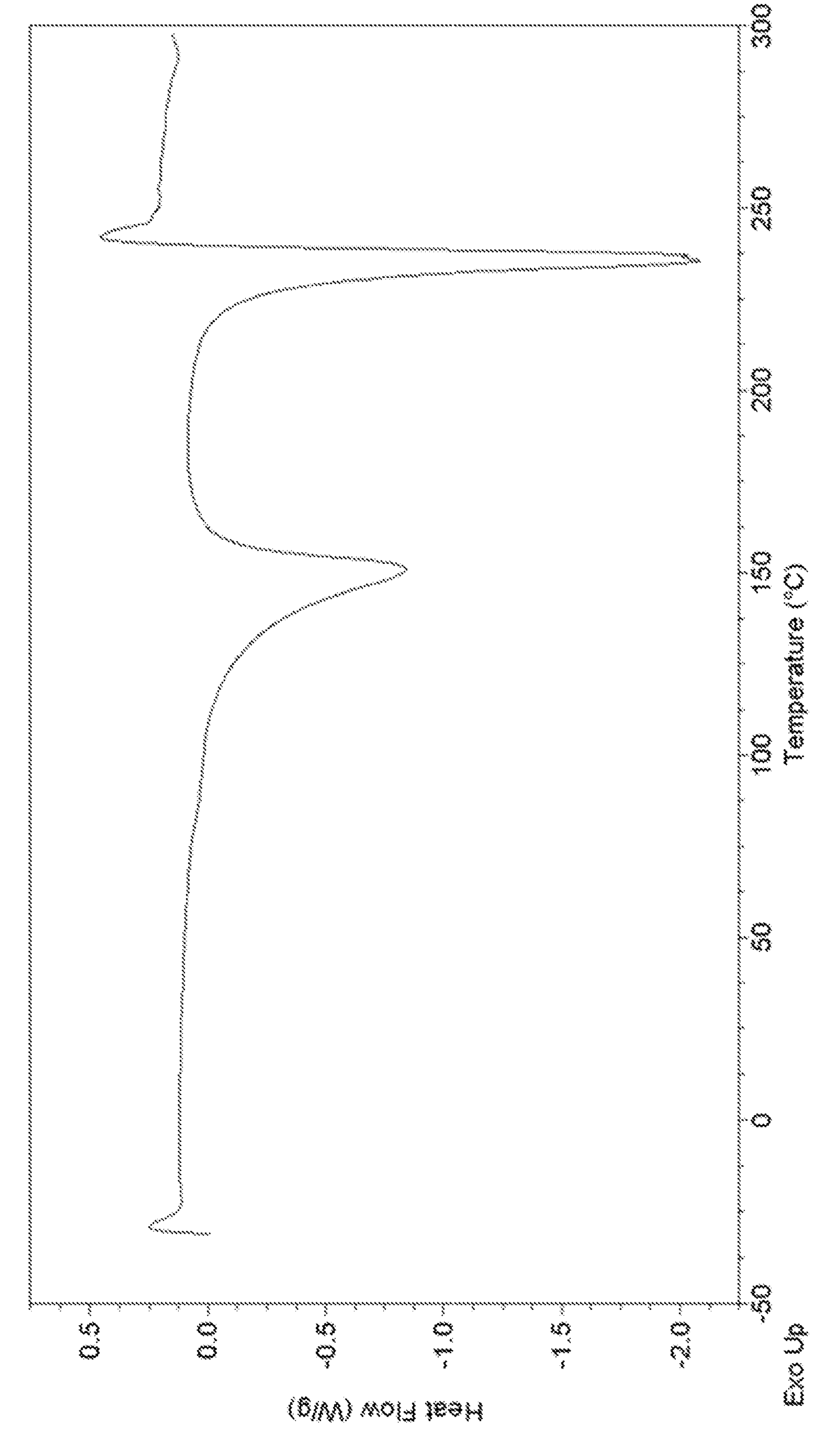
Heat Flow (W/g)
0.5
0.0
-0.5
-1.0
-1.5
-2.0
Exo Up
-50
0
50
100
150
200
250
300
Temperature (°C)

FIG. 35

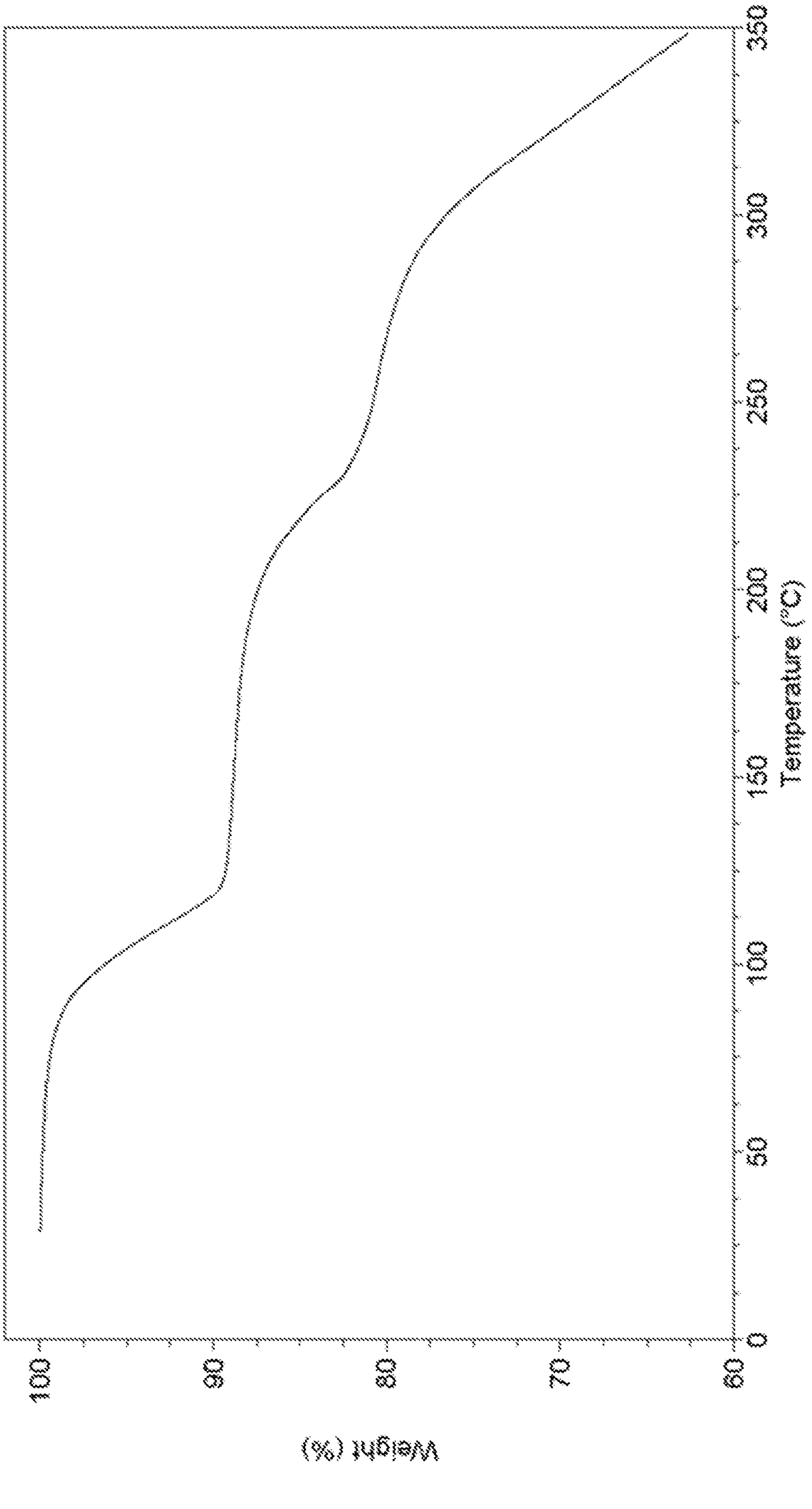
Weight (%)
100
90
80
70
60
0
50
100
150
200
250
300
350
Temperature (°C)

FIG. 36

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014074661 A
- WO 2018183656 A
- WO 2019232138 A
- WO 2020251911 A
- WO 2022165141 A
- WO 2022212181 A


### Non-patent literature cited in the description


- **S.R. BYRN** ; **R.R. PFEIFFER** ; **J.G. STOWELL**. Solid-State Chemistry of Drugs. 1999
- **J.W. MULLIN** ; **J. NYVLT**. Programmed Cooling of Batch Crystallizers. *Chemical Engineering Science*, 1971, vol. 26, 369-377
- **SMITH, D.K**. A FORTRAN Program for Calculating X-Ray Powder Diffraction Patterns,. *Lawrence Radiation Laboratory*, April 1963
- **STOUT** ; **JENSEN**. X-Ray Structure Determination: A Practical Guide. Macmillan Co., 1968